# EUROPEAN PATENT APPLICATION

(11) **EP 2 420 250 A1**
(43) Date of publication of application: **22.02.2012**
(21) Application number: 10172720.4
(22) Date of filing: 13.08.2010
(51) Int. Cl.: A61K 39/395, A61P 19/02, A61P 37/00, C07K 16/28, C12N 15/113

(54) **Anti-Syndecan-4 antibodies**

(71) Applicant: Universitätsklinikum Münster, 48148 Münster (DE)
(72) Inventor: Echtermeyer, Frank, 30900 Wedemark (DE); Pap, Thomas, 49549 Ladbergen (DE); Bertrand, Jessica, 49080 Osnabrück (DE); Dreier, Rita, 48653 Coesfeld (DE); Neugebauer, Katja, 13189 Berlin (DE)
(74) Representative: Schiweck, Weinzierl & Koch

(57) **Abstract**

The present invention relates to a recognition molecule against Syndecan-4, wherein said recognition molecule binds an epitope contained in an amino acid sequence corresponding to the amino acid sequence between amino acids 93 and 121 of the amino acid sequence shown in SEQ ID NO:1 (Figure 1) for use in the treatment and/or prevention of a pathological medical condition associated with turnover of the extracellular matrix and/or cartilage remodelling in which Syndecan-4 is involved. Said pathological condition is preferably osteoarthritis or rheumatoid arthritis.

## Description

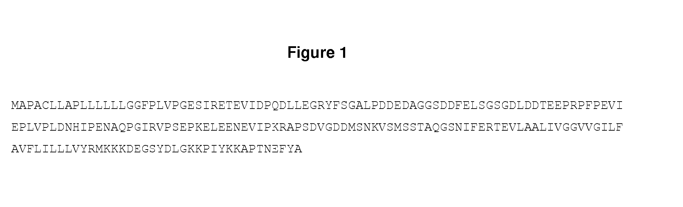

The present invention relates to a recognition molecule against Syndecan-4 (Sdc-4) wherein said recognition molecule binds an epitope contained in an amino acid sequence corresponding to the amino acid sequence between amino acids 93 and 121 of the amino acid sequence shown in SEQ ID NO:1 (Figure 1) for use in the treatment and/or prevention of a pathological medical condition associated with turnover of the extracellular matrix and/or cartilage remodelling in which Syndecan-4 is involved. Said pathological condition is preferably osteoarthritis or rheumatoid arthritis.

Syndecans are type 1 transmembrane proteoglycans and constitute a family of four transmembrane heparan sulfate proteoglycans (HSPG) They share a uniform structure: a divergent extracellular domain (ectodomain) containing glycosaminoglycan (GAG) and sometimes chondroitinsulfate attachment sites, a conserved one-span transmembrane domain (TM) and a conserved short (approximately 30 amino acids) cytoplasmic domain (CD). It is recognized that the Syndecan class is composed of four closely related family proteins (Syndecan-1,-2,-3 and -4, respectively) coded for by four different genes in vivo. Syndecans-1 and -4 are the most widely studied members of this class and show expression in a variety of different cell types including epithelial, endothelial, and vascular smooth muscle cells, although expression in quiescent tissues is at a fairly low level Bernfield et al., Annu. Rev. Cell Biol. 8: 365-393 (1992); Kim et al., Mol. Biol. Cell 5: 797-805 (1994). Syndecan-2 (also known as fibroglycan) is expressed at high levels in cultured lung and skin fibroblasts, although immunocytochemically this core protein is barely detectable in most adult tissues. However, Syndecan-3 (also known as N-Syndecan) demonstrates a much more limited pattern of expression, being largely restricted to peripheral nerves and central nervous system tissues (although high levels of expression are shown in the neonatal heart) (Carey et al., J. Cell Biol. 117: 191- 201 (1992)).

Syndecans bind a variety of soluble and insoluble extracellular effectors. Syndecan extracellular domains (ectodomains) can be shed intact by proteolytic cleavage near the transmembran domain, yielding soluble proteoglycans that retain the binding properties of their cell surface precursors. Syndecans are present as homodimers or multimers, and are often expressed in developmental and cell type-specific patterns. They have many biological roles including regulating cell growth, differentiation, and adhesion.

Among the family members only Syndecan-4 is expressed ubiquitously, involved directly in signal transduction and in the regulation of a variety of cellular processes. The Syndecan-4 core protein has a molecular weight of about 30 kDa. In the extracellular domain, there are three attachment sites of glycosaminoglycan chains of the heparan-sulfate type. Heparan sulfate chains of Syndecan-4 are considered to play various roles by binding to growth factors such as basic fibroblast growth factor (bFGF) and midkine, anticoagulation factors such as tissue factor pathway inhibitor, and cell adhesion molecules such as fibronectin. In many cases, the signal of ligand binding to heparan sulfate is transmitted to the intracellular signaling system or to the cytoskeleton via the cytoplasmic domain of the core protein.

Several studies have implicated Syndecan-4 in cell-matrix adhesion, cell migration, differentiation and proliferation (Bernfield et al, (1999), Annual Review of Biochemistry 68, 729-777. Together with integrins, Syndecan-4 is involved in signal transduction during the formation of focal adhesions and actin reorganization into stress fibers in fibroblasts (Echtermeyer, et al. (1999), J. Cell Sci. 112 (Pt 20), 3433-3441, Saoncella et al. (1999), PNAS USA 96, 2805-2810). In addition, Syndecan-4 can act as an alternative receptor for different growth factors such as fibroblast growth factor (FGF) -2 (Volk et al. (1999), J. Biol.Chem. 274, 24417-24424) and can initiate different signal cascades, e.g., through activation of mitogen-activated protein kinases (MAPKs). During skin wound healing, Syndecan-4 is upregulated in activated dermal fibroblasts, and Syndecan-4-/- mice show a distinct phenotype that is characterized by a delay in the healing of skin wounds and the absence of wound contraction (Echtermeyer et al. (2001), J. Clin. Investigation 107, R9-R14).

Antibodies against Syndecan-4 which activate Syndecan-4 are, for example, described in Echtermeyer et al. (2001), J. Clin. Investigation 107, R9-R14; Saoncella et al. (1999), Proc. Natl. Acad. Sci. USA 96, 2805-2810. While an antibody which is generally said to inhibit Syndecan-4 is mentioned in the prior art (Echtermeyer et al. (2009), Nat. Medicine 15, 1072-1077), it is neither mentioned which of the functions of Syndecan-4 should actually be inhibited nor is it mentioned against which specific portion of Syndecan-4 the antibody is directed (i.e., the location of the epitope which is bound by the antibody is not mentioned).

Meanwhile, Syndecan-4 has become the most extensively studied member of the Syndecan family. The proteoglycan has a number of activities, including modulation of FGF2 signaling, regulation of cell migration via cross-talk with β1 integrin, and control of adhesion via cytoskeletal modifications. All these various events are achieved via actions of a 33-aa cytoplasmic tail, one of the most overworked small proteins among the signaling giants. In some specific examples, Syndecan-4 regulates mesenchymal cell function during tissue repair (Cornelison, et al. (2001), Dev Biol 239, 79-94 (2001), Echtermeyer, et al (2001), J Clin Invest 107, R9-R14) and together with integrins modulates the activation of protein kinase C (Lim, et al. (2003), J Biol Chem 278, 13795-802, focal adhesion kinase; Wilcox-Adelman et al. (2002), J Biol Chem 277, 32970-7) and RhoA (Saoncella, et al., Proc. Natl. Acad. Sci. USA 96, 2805-10).

The antibody in the prior which is said to generally inhibit Syndecan-4 is shown to be useful in the treatment of osteoarthritis (Echtermeyer et al. (2009), Nat. Medicine 15, 1072-1077). However, as mentioned before, it is not described which of the functions of Syndecan-4 is to be inhibited in order to have a potential beneficial effect in the treatment of a cartilage-destructive disorder. It is not described either which epitope of the Syndecan-4 protein should be bound by said antibody in order to inhibit that function of Syndecan-4 that plays a role in the etiology of osteoarthritis.

Moreover, when aiming at the generation of an antibody, it is always highly desirable to generate a specific antibody. Yet, Syndecan-4 shares quite identical or at least highly similar amino acid stretches with Syndecan-1, 2 or 3. Thuis, despite the fact that Syndecan-4 would offer many potential immunogenic regions that are sufficiently different from similar regions of, for example, Syndecan-1, 2 or 3 so that a specific antibody against Syndecan-4 could be generated, it is, nonetheless unpredictable whether a hypothetically specific antibody will exert the desired function, i.e., to inhibit Syndecan-4 activity on the activation of ADAMTS-5. Indeed, it is known that Syndecan-4 has a number of activities, including modulation of FGF2 signaling, regulation of cell migration via cross-talk with β1 integrin, and control of adhesion via cytoskeletal modifications. Thus, Syndecan-4 appears to feed into multiple signalling pathways for which reason it is not predictable which region of the amino acid sequence of Syndecan-4 is to be targeted by an antibody to inhibit Syndecan-4 activity on the activation of ADAMTS-5 or on the pathway leading to the induction of ADAMTS-5 and other proteases which particpate in cartilage destruction.

Put it differently, though it would be highly desirable to have the prior art antibody available in a technically enabling manner, it would not have been possible in view of the teaching of the prior art, since - without knowing which function(s) of Syndecan-4 should be inhibited and which epitope should thus be bound - it would, spoken in technical terms, not have been possible to technically realize (materialize) the provision of said antibody in order to (physically) use that antibody in the treatment of, for example, osteoarthritis. Indeed, one would have required the present inventors' knowledge which, however was not available.

Accordingly, the technical problem underlying the present application is to comply with the needs set out above. Specifically, the technical problem is to provide means and methods for the prevention and/or treatment of pathological medical conditions in which Syndecan-4 is involved.

The present invention addresses these needs and thus provides as a solution to the technical problem a recognition molecule, preferably an antibody against Syndecan-4, wherein said recognition molecule binds an epitope within the extracellular domain of Syndecan-4. Said epitope is contained in an amino acid sequence corresponding to the amino acid sequence between amino acids 93 and 121 of the amino acid sequence shown in SEQ ID NO:1 (Figure 1). Preferably said recognition molecule inhibits phosphorylation of ERK 1/2 by Syndecan-4. Accordingly, said recognition molecule is, for example, preferably useful in the treatment and/or prevention of pathological medical conditions associated with turnover of the extracellular matrix and/or cartilage remodelling in which Syndecan-4 is involved. More specifically, said recognition molecule is useful for the treatment and/or prevention of disorders characterized by pathological breakdown and/or rebuilding (i.e., turnover) associated with Syndecan-4 expression, such as arthritis including osteoarthritis and rheumatoid arthritis.

The embodiments which characterize the present invention are described herein, shown in the Figures, illustrated in the Examples, and reflected in the claims.

It must be noted that as used herein, the singular forms "a", "an", and "the", include plural references unless the context clearly indicates otherwise. Thus, for example, reference to "a reagent" includes one or more of such different reagents and reference to "the method" includes reference to equivalent steps and methods known to those of ordinary skill in the art that could be modified or substituted for the methods described herein.

Unless otherwise indicated, the term "at least" preceding a series of elements is to be understood to refer to every element in the series. Those skilled in the art will recognize, or be able to ascertain using no more than routine experimentation, many equivalents to the specific embodiments of the invention described herein. Such equivalents are intended to be encompassed by the present invention.

The term "and/or" wherever used herein includes the meaning of "and", "or" and "all or any other combination of the elements connected by said term".

The term "about" or "approximately" as used herein means within 20%, preferably within 10%, and more preferably within 5% of a given value or range.

Throughout this specification and the claims which follow, unless the context requires otherwise, the word "comprise", and variations such as "comprises" and "comprising", will be understood to imply the inclusion of a stated integer or step or group of integers or steps but not the exclusion of any other integer or step or group of integer or step. When used herein the term "comprising" can be substituted with the term "containing" or "including" or sometimes when used herein with the term "having".

When used herein "consisting of" excludes any element, step, or ingredient not specified in the claim element. When used herein, "consisting essentially of" does not exclude materials or steps that do not materially affect the basic and novel characteristics of the claim.

In each instance herein any of the terms "comprising", "consisting essentially of" and "consisting of" may be replaced with either of the other two terms.

It should be understood that this invention is not limited to the particular methodology, protocols, and reagents, etc., described herein and as such can vary. The terminology used herein is for the purpose of describing particular embodiments only, and is not intended to limit the scope of the present invention, which is defined solely by the claims.

All publications and patents cited throughout the text of this specification (including all patents, patent applications, scientific publications, manufacturer's specifications, instructions, etc.), whether supra or infra, are hereby incorporated by reference in their entirety. Nothing herein is to be construed as an admission that the invention is not entitled to antedate such disclosure by virtue of prior invention. To the extent the material incorporated by reference contradicts or is inconsistent with this specification, the specification will supersede any such material.

The present inventors, with the aim of providing novel means and methods for the treatment and/or prevention of arthritis, in particular (destructive) inflammatory arthritis including osteoarthritis and rheumatoid arthritis investigated pathological processes during inflammation that result in damage and/or destruction and/or breakdown of the extracellular matrix and/or cartilage, in particular articular cartilage and found that Syndecan-4 plays an important role in mesenchymal cell (in particular chondrocyte) function, particularly in the remodelling of the extracellular matrix.

Specifically, the present inventors found that Syndecan-4 interacts directly with ADAMTS-5 (A disintegrin and metalloproteinase with a thrombospondin type 1 motif member 5) which is an aggrecan-degrading protease. The present inventors also found that Syndecan-4 is involved in the modulation of the activation of ADAMTS-5 by controlling the release of matrix metalloprotease-3 (MMP-3). MMP-3 can activate ADAMTS-5 and thus act in concert with ADAMTS-5 in particular on the degradation of aggrecan, a major protein of the extracellular matrix and of cartilage tissue. More specifically, Syndecan-4 controls the expression of MMP-3 via a mitogen activated protein kinase (MAPK) pathway through phosphorylation of ERK 1/2 which then transduces a signal leading finally to the expression of MMP-3.

In sum, on the basis of the findings of the present inventors, it is assumed that Syndecan-4 targets ADAMTS-5 to the cell surface through binding of ADAMTS-5 via its heparan sulfate chains on chondrocytes by direct interaction between Syndecan-4 and ADAMTS-5 and that Syndecan-4 regulates (preferably induces) MMP-3 expression by activating ERK 1/2. MMP-3 in turn is capable of activating ADAMTS-5, thereby causing degradation of aggrecan and thus causing destruction of cartilage damage because of the protease activity of ADAMTS-5 and/or MMP-3.

The extracellular matrix (ECM) usually provides structural support to animal cells in addition to performing various other important functions. ECM can serve many functions, such as providing support and anchorage for cells, segregating tissues from one another, and regulating intercellular communication. Components of the ECM are produced intracellularly by resident cells, and secreted into the ECM via exocytosis. Once secreted, they then aggregate with the existing matrix. The ECM is composed of an interlocking mesh of fibrous proteins and glycosaminoglycans (GAGs). GAGs are carbohydrate polymers and are usually attached to extracellular matrix proteins to form proteoglycans. Thus, proteoglycans are glycoproteins that are heavily glycosylated. They have a core protein with one or more covalently attached glycosaminoglycan (GAG) chain(s). The chains are long, linear carbohydrate polymers that are negatively charged under physiological conditions, due to the occurrence of sulfate and uronic acid groups. Proteoglycans can be categorised depending upon the nature of their glycosaminoglycan chains. These chains may be: chondroitin sulfate and dermatan sulfate, heparin and heparan sulfate, keratan sulfate. Proteoglycans occur, for example, in the connective tissue.

Cartilage is a stiff and inflexible connective tissue found in many areas in the bodies of humans and other animals, including, for example, the joints between bones and the intervertebral discs.

Cartilage is composed of specialized cells called chondrocytes that produce a large amount of extracellular matrix composed of Type II collagen (except fibrocartilage which also contains type I collagen) fibers, abundant ground substance rich in proteoglycan, and elastin fibers. Cartilage is classified in three types, elastic cartilage, hyaline cartilage and fibrocartilage, which differ in the relative amounts of these three main components.

Aggrecan, a large aggregating proteoglycan or chondroitin sulfate proteoglycan 1, is a proteoglycan, or a protein modified with carbohydrates; e.g., the human form of the protein is 2316 amino acids long and can be expressed in multiple isoforms due to alternative splicing. Along with Type-II collagen, aggrecan forms a major structural component of cartilage, particularly articular cartilage. Aggrecan consists of two globular structural domains at the N-terminal end and one globular domain at the C-terminal end, separated by a large domain heavily modified with glycosaminoglycans. The two main modifier moieties are themselves arranged into distinct regions, a chondroitin sulfate and a keratan sulfate region.

The linker domain between the N-terminal globular domains, called the interglobular domain, is highly sensitive to proteolysis. Such degradation has been associated with the development of arthritis. Proteases capable of degrading aggrecans are called aggrecanases, and they are members of the ADAMTS protein family.

A prominent feature, particularly in joint disease (such as rheumatoid arthritis and osteoarthritis) is a loss of aggrecan. Two major cleavage sites are present in the IGD-domain separating the G1 and G2 domains. Usually, cleavage occurs at DIPEN-FFG or NITEGE-ARG (aggrecanase). The enzymes (ADAMTS 3 and 4) also induce cleavage at other sites. By far the major portion of aggrecan released appears to be cleaved by aggrecanases, primarily ADAMTS-4 and 5, (which also acts during normal turnover).

In particular, it has also been found that aggrecan cleavage by ADAMTS-5 is critical for the breakdown of cartilage matrix during osteoarthritis (Glasson et al., Nature 434, 644-648 (2005); Stanton et al., Nature 434, 648-52 (2005)), a degenerative joint disease that leads to the progressive destruction of articular structures. The mechanisms of ADAMTS-5 activation and their links to the pathogenesis of osteoarthritis remain poorly understood, but Syndecan-1 has been described to be involved in the activation of ADAMTS-4 (Gao et al., J Biol Chem 279, 10042-51 (2004)).

ADAMTS are regulated not only at the transcriptional level, but also by post translational modifications starting with the processing of the pro-form by proprotein convertases or proteases such as furin or MMP9. At the cell surface further activation of ADAMTS-4 and -5 takes place, which is less well understood for ADAMTS-5 but in the case of ADAMTS-4 involve glycosylphosphatidyl inositol-anchored MMP-17 and Syndecan-1.

Expression of all four Syndecans has been detected in chondrocytes, but their function in these cells is incompletely understood. Since Syndecan-4 regulates mesenchymal cell function during tissue repair and together with integrins modulates the activation of protein kinase C, focal adhesion kinase and RhoA, the present inventors investigated whether Syndecan-4 may be functionally involved in particular, in cartilage remodelling during, e.g., osteoarthritis.

To this end, the present inventors found that Syndecan-4 controls the activation of ADAMTS-5 through direct interaction with the protease and through regulating ERK 1/2 mitogen activated protein kinase (MAPK) dependent synthesis of metalloproteinase 3 (MMP-3) and other metalloproteinases, thereby Syndecan-4 is likely to be involved in cartilage breakdown. By making use of these findings, the present inventors aimed at further elucidating the role of Syndecan-4 in these processes and found that loss of Syndecan-4 in genetically modified mice and similarly, intra-articular injections of anti-Syndecan-4 antibodies into wild-type mice, protects from proteoglycan loss and thereby prevents osteoarthritic cartilage damage in a surgically induced model of osteoarthritis. Reduced severity of osteoarthritis-like cartilage destruction in both Syndecan-4 deficient mice and in anti-Syndecan-4 antibody treated wild-type animals result from a significant decrease in ADAMTS-5 activity.

In addition, since the present inventors were aware of the fact that cytokines (in particular, IL-1 and TNFalpha) play a major role in the onset and duration of an inflammatory arthritis such as rheumatoid arthritis, they investigated as to whether Syndecan-4 has a specific function in this process. Surprisingly, the present inventors found that Syndecan-4 is a key modulator of cytokine response in mesenchymal cells. Specifically, it was shown that in human rheumatoid arthritis and a TNFalpha dependent mouse model of this disease, Syndecan-4 is involved critically in fibroblast mediated cartilage breakdown by binding directly interleukin (IL)-1 and providing essential co-signals for IL-1 induced MMP expression. In the course of destructive inflammatory arthritis, Syndecan-4 gets strongly induced in fibroblast-like synoviocytes with TNFalpha being an important regulator of Syndecan-4 expression. However, knockdown of Syndecan-4 by RNAi or the use of blocking antibodies inhibits the IL-1 induced expression of matrix metalloproteinases by impairing the ability of fibroblasts to activate ERK which is thus impaired in transducing an IL-1 induced signal via a MAPK cascade leading finally to the expression of matrix metalloproteinases which are involved in cartilage destruction. In IL-1 binding analyses as well as by reconstitution of Syndecan-4 deficient fibroblasts with different mutants of Syndecan-4 and the use of human IL-1 receptor antagonist in vitro, the present inventors demonstrated that Syndecan-4 directly binds IL-1 and that additional signals through Syndecan-4 are required for IL-1 dependent ERK phosphorylation. Absence of Syndecan-4 protects human TNF transgenic (hTNFtg) mice from inflammatory cartilage destruction by inhibition of MMP expression. Accordingly, hTNFtg animals that are treated over 4 weeks with blocking antibodies against Syndecan-4 showed a significant reduction in MMP-mediated cartilage destruction.

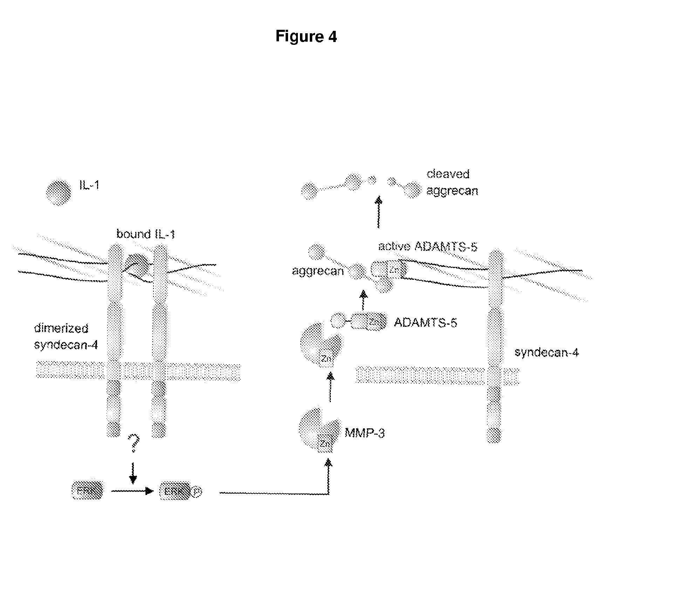

These latter findings that Syndecan-4 interacts directly with IL-1 and is involved in the provision of co-signals for IL-1 induced matrix metalloproteinase expression via ERK 1/2 phosphorylation came very much as a surprise for the present inventors. In fact, the present inventors found with Syndecan-4, so to say, a decisive player in the IL-1 induced signal transduction cascade, with IL-1 playing a prominent role in mediating (pathological) cartilage damage in rheumatoid arthritis because of its capability to induce matrix metalloproteinase expression via ERK/MAPKs. As is commonly known for pathological processes, it is highly desirable to act on the top level of a hierarchial cascade in order to prevent as early as possible and as effectively as possible the pathological process mediated by the cascade. So far, treatment of rheumatoid arthritis acts on TNFalpha (by way of a blocking antibody (Infliximab) or by way of a scavenger molecule, i.e., soluble TNF binding protein (Etanercept). In particular, it is known that blocking TNF effectively inhibits inflammation and structural damage in rheumatoid arthritis. Yet, so far it is unknown whether the effect of TNF is a direct one or indirect on up-regulation of other mediators. IL-1 may be one of these candidates because it has a central role in animal models of arthritis, and inhibition of IL-1 is used as a therapy of human rheumatoid arthritis (Zwerina et al., Proc. Natl. Acad. Sci. 104, 11742-11747 (2007)). However, with Syndecan-4 the present inventors provide a target that, as they have shown, can be specifically influenced by way of antibodies such that its activity on ADAMTS proteases and matrix metalloproteinsases can be inhibited. Thus, inhibiting Syndecan-4 induced activation of ERK 1/2 and subsequently downstream processes and/or blocking IL-1-Syndecan-4 interaction seems to provide a promising way of treating rheumatoid arthritis (see Figure 4).

That being so, as mentioned above, the present inventors also demonstrated that by way of an inhibiting antibody blocking of the direct interaction between Syndecan-4 and ADAMTS-5 as well as again the action of Syndecan-4 on ERK 1/2 phosphorylation seems to be beneficial for the treatment of osteoarthritis. These two independently found highly surprising findings seem to pave the way for a commonly applicable treatment of (inflammatory) arthritis, in particular osteoarthritis and rheumatoid arthritis, by way of a recognition molecule, preferably an antibody.

In sum, these data suggest that strategies aiming at the inhibition of Syndecan-4 will be of great value for the treatment of cartilage damage and/or matrix degeneration in the treatment and/or prevention of a pathological medical condition in which Syndecan-4 is involved, e.g., inflammatory conditions of joints, the matrix or connective tissue such as arthritis including, in particular, osteoarthritis and rheumatoid arthritis.

Accordingly, in a first aspect, the present invention relates to a recognition molecule, particularly preferable a recognition molecule against Syndecan-4, wherein said recognition molecule binds an epitope contained in an amino acid sequence corresponding to the amino acid sequence between amino acids 93 and 121 of the amino acid sequence shown in SEQ ID NO:1 (Figure 1) for use in the treatment and/or prevention of a pathological medical condition associated with turnover of the extracellular matrix and/or cartilage remodelling in which Syndecan-4 is involved. Said pathological condition is preferably Syndecan-4 mediated arthritis, including in particular osteoarthritis and rheumatoid arthritis. Preferably, said pathological condition is charcterized by cartilage destruction and/or damage.

Preferably said recognition molecule inhibits phosphorylation of ERK 1/2 through Syndecan-4, preferably IL-1 induced phosphorylation of ERK 1/2 through Syndecan-4. Specifically, without being bound by theory, it is assumed that the recognition molecule, preferably an antibody, inhibits or interferes with the dimerization of Syndecan-4 and, thus, Syndecan-4 is no longer capable of transducing a signal via phosphorylation of ERK 1/2 and subsequent downstream processes. Specifically, it is then further assumed that the MAPK ERK 1/2 cannot regulate downstream pathways, leading, inter alia, to the synthesis of matrix-metalloproteinase-3 (MMP-3). Thus, in the alternative and/or additionally it is preferred that the recognition molecule inhibits MMP-3 expression, preferably by inhibiting ERK 1/2 phosphorylation and thus the downstream action of ERK 1/2 via pathways which act on MMP-3 expression.

Given the assumption that the recognition molecule of the present invention preferably inhibits or interferes with the dimerization of Syndecan-4, it is in the alternative and/or additionally preferred that the recognition molecule inhibits dimerization of Syndecan-4. Without being bound by theory, it is believed that dimerization of Syndecan-4 effects phosphorylation of ERK 1/2.

This preferred property is believed to be advantageous, since Syndecan-4, though dimerization is inhibited or interfered with, may thus still be capable of fulfilling other functions in which it is involved, while its function as regards the phosphorylation of ERK 1/2 is interfered or inhibited. Hence, the recognition molecule of the present invention may preferably not influence Syndecan-4 in a manner so as to cause undesired effects such as interference with other functions that Syndecan-4 may have, for example, binding of IL-1 and/or ADAMTS-5.

"Inhibiting phosphorylation" or "inhibition of phosphorylation" means that the recognition molecule inhibits phosphorylation of ERK 1/2 by Syndecan-4, preferably IL-1 induced phosphorylation of ERK 1/2 by Syndecan-4, preferably to such an extent that ERK 1/2 can no longer induce the expression of, in particular, MMP-3. MMP-3, in turn, cannot activate ADAMTS-5 and/or other metalloproteinases that according to established data are activated by MMP-3. Accordingly, inhibition encompasses that the phosphorylation of ERK 1/2 by Syndecan-4 is at least 10, 20, 30, 40, 50%, more preferably at least 60% or even preferably 70% or more. The degree of phosphorylation of ERK 1/2 and thus its inhibition can be tested by means and methods known in the art (see, e.g., Example 1.8). The term "pERK" refers to "phosphorylated ERK protein", wherein "ERK" is ERK 1 and/or ERK 2 (e.g. the proteins encoded by the human ERK 1 and ERK 2 genes with NCBI GeneID numbers of 5595 and 5594), each phosphorylated at both the tyrosine and threonine amino acid residues required for ERK kinase activation (i.e. T202 and Y204 in human ERK1; and T 85 and Y 87 in human ERK2; the amino acid residues immediately surrounding these phosphorylation sites are identical in human ERK 1 and ERK 2). Double phosphorylation of ERK has been demonstrated to be required for activation of the kinase activity of the enzyme. (N.B. the numbering of the two phosphorylated residues may vary, depending on the species, but will be readily apparent to one of skill in the art by examination of the animal sequence homologous to the amino acid sequence around the human ERK 1 or ERK2 phosphorylation sites).

However, it is preferred that the inhibition of phosphorylation abolishes ERK 1/2 activity on the induction of MMP-3, thereby decreasing ADAMTS-5 activity which is believed to be activated by MMP-3. Accordingly, as a "read-out" for the inhibition of ERK 1/2 phosphorylation, it is preferred that MMP-3 expression and/or ADAMTS-5 activity is determined (i.e., "indirect" measurement of the inhibition of phosphorylation of ERK 1/2). Phosphorylation of ERK 1/2 can, for example, be measured as illustrated in the Examples.

ADAMTS-5 activity can be measured, for example, as done by Gendron et al., J. Biol. Chem. 282 (2007), 18294-18306 or in Example 7 of WO 2004/011637.

MMP-3 expression can be determined, for example, by RT-PCR. MMP-3 activity can be determined by a matrix metalloproteinase assay commonly known in the art.

Direct interaction between Syndecan-4 and ADAMTS-5 and/or Syndecan-4 and IL-1, respectively, can be determined by co-immunoprecipitation or a suitable two-hybrid system, for example, the yeast two-hybrid system.

A "recognition molecule" when used herein is a polypeptide which comprises one or more binding domains capable of binding to an epitope of a Syndecan-4 protein, wherein said epitope is preferably contained in an amino acid sequence corresponding to the amino acid sequence between amino acids 93 and 121 of the amino acid sequence shown in SEQ ID NO:1 (Figure 1). A recognition molecule, so to say, provides the scaffold for said one or more binding domains so that said binding domains can bind/interact with a given target structure/antigen/epitope. For example, such a scaffold could be provided by protein A, in particular, the Z-domain thereof (affibodies), ImmE7 (immunity proteins), BPTI/APPI (Kunitz domains), Ras-binding protein AF-6 (PDZ-domains), charybdotoxin (Scorpion toxin), CTLA-4, Min-23 (knottins), lipocalins (anticalins), neokarzinostatin, a fibronectin domain, an ankyrin consensus repeat domain or thioredoxin (Skerra, Curr. Opin. Biotechnol. 18, 295-304 (2005); Hosse et al., Protein Sci. 15, 14-27 (2006); Nicaise et al., Protein Sci. 13, 1882-1891 (2004) ; Nygren and Uhlen, Curr. Opin. Struc. Biol. 7, 463-469 (1997)).

The term "binding domain" characterizes in connection with the present invention a domain of a polypeptide which specifically binds/interacts with a given target epitope. An "epitope" is antigenic and thus the term epitope is sometimes also referred to herein as "antigenic structure" or "antigenic determinant". Thus, the binding domain is an "antigen-interaction-site". The term "antigen-interaction-site" defines, in accordance with the present invention, a motif of a polypeptide, which is able to specifically interact with a specific antigen or a specific group of antigens, e.g. the identical antigen in different species. Said binding/interaction is also understood to define a "specific recognition". The term "epitope" also refers to a site on an antigen (in the context of the present invention, the antigen is a Syndecan-4 protein) to which the recognition molecule binds. Preferably, an epitope is a site on a molecule (in the context of the present invention, the antigen is a Syndecan-4 protein) against which a recognition molecule, preferably an antibody will be produced and/or to which an antibody will bind.

For example, an epitope can be recognized by a recognition molecule, particularly preferably by an antibody defining the epitope. A "linear epitope" is an epitope where an amino acid primary sequence comprises the epitope recognized. A linear epitope typically includes at least 3, and more usually, at least 5, for example, about 8 to about 10 amino acids in a unique sequence.

A "conformational epitope", in contrast to a linear epitope, is an epitope wherein the primary sequence of the amino acids comprising the epitope is not the sole defining component of the epitope recognized (e.g., an epitope wherein the primary sequence of amino acids is not necessarily recognized by the antibody defining the epitope). Typically a conformational epitope comprises an increased number of amino acids relative to a linear epitope. With regard to recognition of conformational epitopes, the recognition molecule recognizes a 3-dimensional structure of the antigen, preferably a peptide or protein or fragment thereof (in the context of the present invention, the antigen is a Syndecan-4 protein). For example, when a protein molecule folds to form a three dimensional structure, certain amino acids and/or the polypeptide backbone forming the conformational epitope become juxtaposed enabling the antibody to recognize the epitope. Methods of determining conformation of epitopes include but are not limited to, for example, x-ray crystallography 2-dimensional nuclear magnetic resonance spectroscopy and site-directed spin labeling and electron paramagnetic resonance spectroscopy.

The term "specifically recognizing" (can be equally used with the term "directed to" or "reacting with") means in accordance with this invention that the recognition molecule is capable of specifically interacting with and/or binding to at least two, preferably at least three, more preferably at least four amino acids of an epitope as defined herein. Such binding may be exemplified by the specificity of a "lock-and-key-principle".

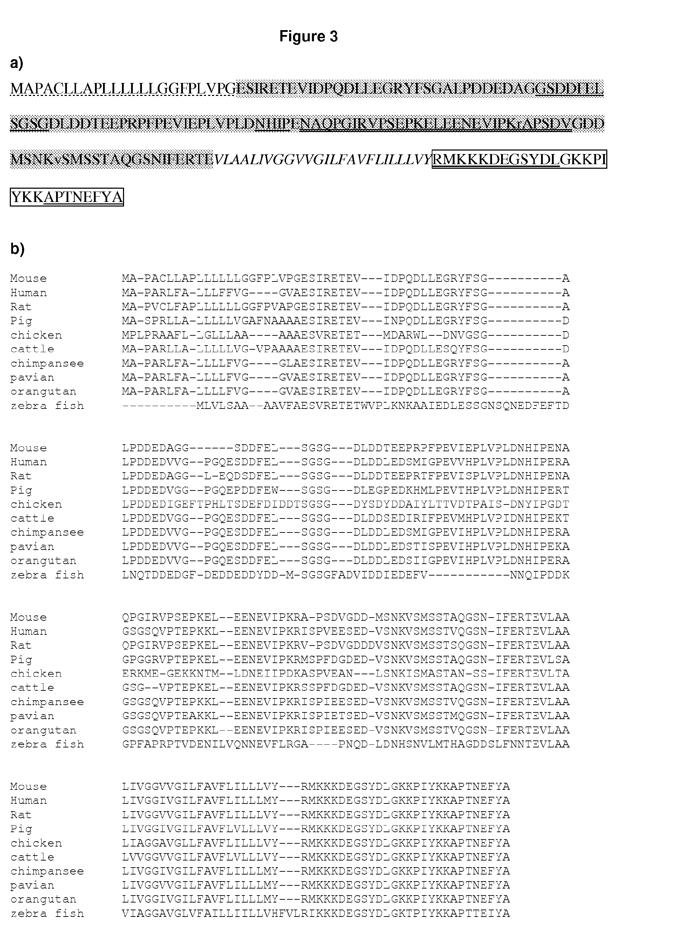

Thus, the term "specifically" in this context means that the recognition molecule binds to a Syndecan-4 protein but does not essentially bind to another protein. The term "another protein" includes any protein including proteins closely related to or being homologous to a Syndecan-4 protein against which the recognition molecule is directed to. However, the term "another protein" does not include that the recognition molecule cross-reacts with a Syndecan-4 protein from a species different from that against which the recognition molecule was generated. For example, it is envisaged that a recognition molecule directed against mouse Syndecan-4 would cross-react with human Syndecan-4, but not with, e.g., human Syndecan-1, -2, or -3. Thus, cross-species specific recognition molecules directed against Syndecan-4 are preferably contemplated by the present invention. For example, it is apparent that the mouse Syndecan-4 amino acid sequence is quite similar, i.e., at many positions it is identical, to the human Syndecan-4 amino acid sequence; see the alignment shown in Figure 3, in particular the region of the epitope between amino acids 93 and 121 of the mouse sequence corresponding to amino acids 92 and 122 of the human sequence. Thus, it is apparent that a recognition molecule against mouse Syndecan-4 highly likely binds human Syndecan-4. The polyclonal antibody (serum) of the present invention recognizes mouse Syndecan-4 and is preferably cross-reactive with human Synecan-4.

The term "cross-species recognition" or "interspecies specificity" as used herein means binding of a binding domain described herein to the same target molecule in humans and non-human species. Thus, "cross-species specificity" or "interspecies specificity" is to be understood as an interspecies reactivity to the same molecule X (in particular Syndecan-4) expressed in different species, but not to a molecule other than X (in particular, Syndecan-4). Preferably, a binding domain which binds to mouse Syndecan-4, in particular to the region between amino acids 93 and 121 of the amino acid sequence shown in SEQ ID NO:1 also binds to human Syndecan-4, in particular to a region in the amino acid sequence shown in SEQ ID NO:2 that corresponds to the region between amino acids 93 and 121 of the amino acid sequence shown in SEQ ID NO:1.

The term "does not essentially bind" means that the Syndecan-4 recognition molecule of the present invention does not bind another protein, i.e., shows a cross-reactivity of less than 30%, preferably 20%, more preferably 10%, particularly preferably less than 9, 8, 7, 6 or 5% with another protein.

Specific binding is believed to be effected by specific motifs in the amino acid sequence of the binding domain and the antigen bind to each other as a result of their primary, secondary or tertiary structure as well as the result of secondary modifications of said structure. The specific interaction of the antigen-interaction-site with its specific antigen may result as well in a simple binding of said site to the antigen. Moreover, the specific interaction of the antigen-interaction-site with its specific antigen may alternatively result in the initiation of a signal, e.g. due to the induction of a change of the conformation of the antigen, an oligomerization of the antigen, etc. A preferred example of a binding domain in line with the present invention is an antibody.

Typically, binding is considered specific when the binding affinity is higher than 10⁻⁶M. Preferably, binding is considered specific when binding affinity is about 10⁻¹¹ to 10⁻⁸ M (KD), preferably of about 10⁻¹¹ to 10⁻⁹ M.If necessary, nonspecific binding can be reduced without substantially affecting specific binding by varying the binding conditions. Whether the recognition molecule specifically reacts as defined herein above can easily be tested, inter alia, by comparing the reaction of said recognition molecule with a Syndecan-4 protein with the reaction of said recognition molecule with (an) other protein(s).

The term polypeptide" is equally used herein with the term "protein". Proteins (including fragments thereof, preferably biologically active fragments, and peptides, usually having les than 30 amino acids) comprise one or more amino acids coupled to each other via a covalent peptide bond (resuting in a chain of amino acids). The term "polypeptide" as used herein describes a group of molecules, which consist of more than 30 amino acids. Polypeptides may further form multimers such as dimers, trimers and higher oligomers, i.e. consisting of more than one polypeptide molecule. Polypeptide molecules forming such dimers, trimers etc. may be identical or non-identical. The corresponding higher order structures of such multimers are, consequently, termed homo- or heterodimers, homo- or heterotrimers etc. An example for a hereteromultimer is an antibody molecule, which, in its naturally occurring form, consists of two identical light polypeptide chains and two identical heavy polypeptide chains. The terms "polypeptide" and "protein" also refer to naturally modified polypeptides/proteins wherein the modification is effected e.g. by post-translational modifications like glycosylation, acetylation, phosphorylation and the like. Such modifications are well known in the art.

A preferred recognition molecule of the present invention is an antibody. An "antibody" when used herein is a protein comprising one or more polypeptides (comprising one or more binding domains, preferably antigen binding domains) substantially or partially encoded by immunoglobulin genes or fragments of immunoglobulin genes. The term "immunoglobulin" (Ig) is used interchangeably with "antibody" herein.

The recognized immunoglobulin genes include the kappa, lambda, alpha, gamma, delta, epsilon and mu constant region genes, as well as myriad immunoglobulin variable region genes.

In particular, an "antibody" when used herein, is typically tetrameric glycosylated proteins composed of two light (L) chains of approximately 25 kDa each and two heavy (H) chains of approximately 50 kDa each. Two types of light chain, termed lambda and kappa, may be found in antibodies. Depending on the amino acid sequence of the constant domain of heavy chains, immunoglobulins can be assigned to five major classes: A, D, E, G, and M, and several of these may be further divided into subclasses (isotypes), e.g., IgG1, IgG2, IgG3, IgG4, IgA1, and IgA2. An IgM antibody consists of 5 of the basic heterotetramer unit along with an additional polypeptide called a J chain, and contains 10 antigen binding sites, while IgA antibodies comprise from 2-5 of the basic 4-chain units which can polymerize to form polyvalent assemblages in combination with the J chain. In the case of IgGs, the 4-chain unit is generally about 150,000 daltons. Each light chain includes an N-terminal variable (V) domain (VL) and a constant (C) domain (CL). Each heavy chain includes an N-terminal V domain (VH), three or four C domains (CHs), and a hinge region.

The constant domains are not involved directly in binding an antibody to an antigen, but can exhibit various effector functions, such as participation of the antibody dependent cellular cytotoxicity (ADCC). If an antibody should exert ADCC, it is preferably of thelgG1 subtype, while the IgG4 subtype would not have the capability to exert ADCC:
The pairing of a VH and VL together forms a single antigen-binding site. The CH domain most proximal to VH is designated as CH1. Each L chain is linked to an H chain by one covalent disulfide bond, while the two H chains are linked to each other by one or more disulfide bonds depending on the H chain isotype. The VH and VL domains consist of four regions of relatively conserved sequences called framework regions (FR1, FR2, FR3, and FR4), which form a scaffold for three regions of hypervariable sequences (complementarity determining regions, CDRs). The CDRs contain most of the residues responsible for specific interactions of the antibody with the antigen. CDRs are referred to as CDR 1, CDR2, and CDR3. Accordingly, CDR constituents on the heavy chain are referred to as H1, H2, and H3, while CDR constituents on the light chain are referred to as L1, L2, and L3.

The term "variable" refers to the portions of the immunoglobulin domains that exhibit variability in their sequence and that are involved in determining the specificity and binding affinity of a particular antibody (i.e., the "variable domain(s)"). Variability is not evenly distributed throughout the variable domains of antibodies; it is concentrated in sub-domains of each of the heavy and light chain variable regions. These sub-domains are called "hypervariable" regions or "complementarity determining regions" (CDRs). The more conserved (i.e., non-hypervariable) portions of the variable domains are called the "framework" regions (FRM). The variable domains of naturally occurring heavy and light chains each comprise four FRM regions, largely adopting a β- sheet configuration, connected by three hypervariable regions, which form loops connecting, and in some cases forming part of, the β -sheet structure. The hypervariable regions in each chain are held together in close proximity by the FRM and, with the hypervariable regions from the other chain, contribute to the formation of the antigen- binding site (see Kabat et al., loc. cit.). The constant domains are not directly involved in antigen binding, but exhibit various effector functions, such as, for example, antibody- dependent, cell-mediated cytotoxicity and complement activation.

The terms "CDR", and its plural "CDRs", refer to a complementarity determining region (CDR) of which three make up the binding character of a light chain variable region (CDRL1, CDRL2 and CDRL3) and three make up the binding character of a heavy chain variable region (CDRH1, CDRH2 and CDRH3). CDRs contribute to the functional activity of an antibody molecule and are separated by amino acid sequences that comprise scaffolding or framework regions. The exact definitional CDR boundaries and lengths are subject to different classification and numbering systems. CDRs may therefore be referred to by Kabat, Chothia, contact or any other boundary definitions, including the numbering system described herein. Despite differing boundaries, each of these systems has some degree of overlap in what constitutes the so called "hypervariable regions" within the variable sequences. CDR definitions according to these systems may therefore differ in length and boundary areas with respect to the adjacent framework region. See for example Kabat, Chothia, and/or MacCallum et al., (Kabat et al., loc. cit.; Chothia et al., J. Mol. Biol, 1987, 196: 901; and MacCallum et al, J. Mol. Biol, 1996, 262: 732). However, the numbering in accordance with the so-called Kabat system is preferred.

The term "amino acid" or "amino acid residue" typically refers to an amino acid having its art recognized definition such as an amino acid selected from the group consisting of: alanine (Ala or A); arginine (Arg or R); asparagine (Asn or N); aspartic acid (Asp or D); cysteine (Cys or C); glutamine (Gln or Q); glutamic acid (Glu or E); glycine (Gly or G); histidine (His or H); isoleucine (He or I): leucine (Leu or L); lysine (Lys or K); methionine (Met or M); phenylalanine (Phe or F); pro line (Pro or P); serine (Ser or S); threonine (Thr or T); tryptophan (Trp or W); tyrosine (Tyr or Y); and valine (Val or V), although modified, synthetic, or rare amino acids may be used as desired. Generally, amino acids can be grouped as having a nonpolar side chain (e.g., Ala, Cys, He, Leu, Met, Phe, Pro, Val); a negatively charged side chain (e.g., Asp, Glu); a positively charged sidechain (e.g., Arg, His, Lys); or an uncharged polar side chain (e.g., Asn, Cys, Gln, Gly, His, Met, Phe, Ser, Thr, Trp, and Tyr).

The term "hypervariable region" (also known as "complementarity determining regions" or CDRs) when used herein refers to the amino acid residues of an antibody which are (usually three or four short regions of extreme sequence variability) within the V-region domain of an immunoglobulin which form the antigen-binding site and are the main determinants of antigen specificity. There are at least two methods for identifying the CDR residues: (1) An approach based on cross-species sequence variability (i. e., Kabat et al., loc. cit.); and (2) An approach based on crystallographic studies of antigen-antibody complexes (Chothia, C. et al., J. Mol. Biol. 196: 901-917 (1987)). However, to the extent that two residue identification techniques define regions of overlapping, but not identical regions, they can be combined to define a hybrid CDR. However, in general, the CDR residues are preferably identified in accordance with the so-called Kabat (numbering) system.

The term "framework region" refers to the art-recognized portions of an antibody variable region that exist between the more divergent (i.e., hypervariable) CDRs. Such framework regions are typically referred to as frameworks 1 through 4 (FR1, FR2, FR3, and FR4) and provide a scaffold for the presentation of the six CDRs (three from the heavy chain and three from the light chain) in three dimensional space, to form an antigen-binding surface.

The term "canonical structure" refers to the main chain conformation that is adopted by the antigen binding (CDR) loops. From comparative structural studies, it has been found that five of the six antigen binding loops have only a limited repertoire of available conformations. Each canonical structure can be characterized by the torsion angles of the polypeptide backbone. Correspondent loops between antibodies may, therefore, have very similar three dimensional structures, despite high amino acid sequence variability in most parts of the loops (Chothia and Lesk, J. Mol. Biol., 1987, 196: 901; Chothia et al, Nature, 1989, 342: 877; Martin and Thornton, J. Mol. Biol, 1996, 263: 800, each of which is incorporated by reference in its entirety). Furthermore, there is a relationship between the adopted loop structure and the amino acid sequences surrounding it. The conformation of a particular canonical class is determined by the length of the loop and the amino acid residues residing at key positions within the loop, as well as within the conserved framework (i.e., outside of the loop). Assignment to a particular canonical class can therefore be made based on the presence of these key amino acid residues. The term "canonical structure" may also include considerations as to the linear sequence of the antibody, for example, as catalogued by Kabat (Kabat et al, loc. cit.). The Kabat numbering scheme (system) is a widely adopted standard for numbering the amino acid residues of an antibody variable domain in a consistent manner and is the preferred scheme applied in the present invention as also mentioned elsewhere herein. Additional structural considerations can also be used to determine the canonical structure of an antibody. For example, those differences not fully reflected by Kabat numbering can be described by the numbering system of Chothia et al and/or revealed by other techniques, for example, crystallography and two or three-dimensional computational modeling. Accordingly, a given antibody sequence may be placed into a canonical class which allows for, among other things, identifying appropriate chassis sequences (e.g., based on a desire to include a variety of canonical structures in a library). Kabat numbering of antibody amino acid sequences and structural considerations as described by Chothia et al., loc. cit. and their implications for construing canonical aspects of antibody structure, are described in the literature.

CDR3 is typically the greatest source of molecular diversity within the antibody-binding site. H3, for example, can be as short as two amino acid residues or greater than 26 amino acids. The subunit structures and three-dimensional configurations of different classes of immunoglobulins are well known in the art. For a review of the antibody structure, see Antibodies: A Laboratory Manual, Cold Spring Harbor Laboratory, eds. Harlow et al., 1988. One of skill in the art will recognize that each subunit structure, e.g., a CH, VH, CL, VL, CDR, FR structure, comprises active fragments, e.g., the portion of the VH, VL, or CDR subunit the binds to the antigen, i.e., the antigen-binding fragment, or, e.g., the portion of the CH subunit that binds to and/or activates, e.g., an Fc receptor and/or complement. The CDRs typically refer to the Kabat CDRs, as described in Sequences of Proteins of immunological Interest, US Department of Health and Human Services (1991), eds. Kabat et al. Another standard for characterizing the antigen binding site is to refer to the hypervariable loops as described by Chothia. See, e.g., Chothia, et al. (1992; J. Mol. Biol. 227:799-817; and Tomlinson et al. (1995) EMBO J. 14:4628-4638. Still another standard is the AbM definition used by Oxford Molecular's AbM antibody modelling software. See, generally, e.g., Protein Sequence and Structure Analysis of Antibody Variable Domains. In: Antibody Engineering Lab Manual (Ed.: Duebel, S. and Kontermann, R., Springer-Verlag, Heidelberg). Embodiments described with respect to Kabat CDRs can alternatively be implemented using similar described relationships with respect to Chothia hypervariable loops or to the AbM-defined loops.

The sequence of antibody genes after assembly and somatic mutation is highly varied, and these varied genes are estimated to encode 10¹⁰ different antibody molecules (Immunoglobulin Genes, 2nd ed., eds. Jonio et al., Academic Press, San Diego, CA, 1995). Accordingly, the immune system provides a repertoire of immunoglobulins. The term "repertoire" refers to at least one nucleotide sequence derived wholly or partially from at least one sequence encoding at least one immunoglobulin. The sequence(s) may be generated by rearrangement in vivo of the V, D, and J segments of heavy chains, and the V and J segments of light chains. Alternatively, the sequence(s) can be generated from a cell in response to which rearrangement occurs, e.g., in vitro stimulation. Alternatively, part or all of the sequence(s) may be obtained by DNA splicing, nucleotide synthesis, mutagenesis, and other methods, see, e.g., U.S. Patent 5,565,332. A repertoire may include only one sequence or may include a plurality of sequences, including ones in a genetically diverse collection.

When used herein the term "antibody" does not only refer to an immunoglobulin (or intact antibody), but also to a fragment thereof, and encompasses any polypeptide comprising an antigen-binding fragment or an antigen-binding domain. Preferably, the fragment such as Fab, F(ab')₂, Fv, scFv, Fd, dAb, and other antibody fragments that retain antigen-binding function. Typically, such fragments would comprise an antigen-binding domain and have the same properties as the antibodies described herein. Accordingly, said fragment is preferably also capable to inhibit phosphorylation of ERK 1/2 by Syndecan-4.

The term "antibody" as used herein includes Syndeca-4 antibodies that compete for binding to the same epitope as the epitope bound by the antibodies of the present invention, preferably obtainable by the methods for the generation of an antibody as described herein elsewhere. As mentioned herein, the epitope is contained in an amino acid sequence corresponding to the amino acid sequence between amino acids 93 and 121 of the amino acid sequence shown in SEQ ID NO:1 (Figure 1).

To determine if a test antibody can compete for binding to the same epitope as the epitope bound by the Syndecan-4 antibodies of the present invention, a cross-blocking assay e.g., a competitive ELISA assay can be performed. In an exemplary competitive ELISA assay, Syndecan-4 coated wells of a microtiter plate, or Syndecan-4 coated sepharose beads, are pre-incubated with or without candidate competing antibody and then a biotin-labeled anti-Syndecan-4 antibody of the invention is added. The amount of labeled anti-Syndecan-4 antibody bound to the Syndecan-4 antigen in the wells or on the beads is measured using avidin-peroxidase conjugate and appropriate substrate. Alternatively, the anti-Syndecan-4 antibody can be labeled, e.g., with a radioactive or fluorescent label or some other detectable and measurable label. The amount of labeled anti-Syndecan-4 antibody that binds to the antigen will have an inverse correlation to the ability of the candidate competing antibody (test antibody) to compete for binding to the same epitope on the antigen, i.e., the greater the affinity of the test antibody for the same epitope, the less labeled anti-Syndecan antibody will be bound to the antigen-coated wells.

A candidate competing antibody is considered an antibody that binds substantially to the same epitope or that competes for binding to the same epitope as an anti-Syndecan-4 antibody of the invention if the candidate competing antibody can block binding of the anti-Syndecan-4 antibody by at least 20%, preferably by at least 20-50%, even more preferably, by at least 50% as compared to a control performed in parallel in the absence of the candidate competing antibody (but may be in the presence of a known noncompeting antibody). It will be understood that variations of this assay can be performed to arrive at the same quantitative value.

The term "antibody" also includes but is not limited to polyclonal, monoclonal, monospecific, polyspecific such as bispecific, non-specific, humanized, human, single-chain, chimeric, synthetic, recombinant, hybrid, mutated, grafted, and in vitro generated antibodies, with a polyclonal antibody being preferred.

Accordingly, the term "antibody" also relates to a purified serum, i.e., a purified polyclonal serum. Accordingly, said term preferably relates to a serum, more preferably a polyclonal serum and most preferably to a purified (polyclonal) serum. The antibody/serum is obtainable, and preferably obtained, for example, by the method or use described herein and illustrated in the appended Examples.

"Polyclonal antibodies" or "polyclonal antisera" refer to immune serum containing a mixture of antibodies specific for one (monovalent or specific antisera) or more (polyvalent antisera) antigens which may be prepared from the blood of animals immunized with the antigen or antigens.

Furthermore, the term "antibody" as employed in the invention also relates to derivatives or variants of the antibodies described herein which display the same specificity as the described antibodies. Examples of "antibody variants" include humanized variants of non- human antibodies, "affinity matured" antibodies (see, e.g. Hawkins et al. J. Mol. Biol. 254, 889-896 (1992) and Lowman et al., Biochemistry 30, 10832- 10837 (1991)) and antibody mutants with altered effector function (s) (see, e.g., US Patent 5, 648, 260).

The terms "antigen-binding domain", "antigen-binding fragment" and "antibody binding region" when used herein refer to a part of an antibody molecule that comprises amino acids responsible for the specific binding between antibody and antigen. The part of the antigen that is specifically recognized and bound by the antibody is referred to as the "epitope" as described herein above. As mentioned above, an antigen-binding domain may typically comprise an antibody light chain variable region (VL) and an antibody heavy chain variable region (VH); however, it does not have to comprise both. Fd fragments, for example, have two VH regions and often retain some antigen-binding function of the intact antigen-binding domain. Examples of antigen-binding fragments of an antibody include (1) a Fab fragment, a monovalent fragment having the VL, VH, CL and CH1 domains; (2) a F(ab')2 fragment, a bivalent fragment having two Fab fragments linked by a disulfide bridge at the hinge region; (3) a Fd fragment having the two VH and CH1 domains; (4) a Fv fragment having the VL and VH domains of a single arm of an antibody, (5) a dAb fragment (Ward et al., (1989) Nature 341 :544-546), which has a VH domain; (6) an isolated complementarity determining region (CDR), and (7) a single chain Fv (scFv). Although the two domains of the Fv fragment, VL and VH> are coded for by separate genes, they can be joined, using recombinant methods, by a synthetic linker that enables them to be made as a single protein chain in which the VL and VH regions pair to form monovalent molecules (known as single chain Fv (scFv); see e.g., Bird et al. (1988) Science 242:423-426; and Huston et al. (1988) Proc. Natl. Acad. Sci USA 85:5879-5883). These antibody fragments are obtained using conventional techniques known to those with skill in the art, and the fragments are evaluated for function in the same manner as are intact antibodies.

The term "monoclonal antibody" as used herein refers to an antibody obtained from a population of substantially homogeneous antibodies, i.e., the individual antibodies comprising the population are identical except for possible naturally occurring mutations and/or post- translation modifications (e.g., isomerizations, amidations) that may be present in minor amounts. Monoclonal antibodies are highly specific, being directed against a single antigenic site. Furthermore, in contrast to conventional (polyclonal) antibody preparations which typically include different antibodies directed against different determinants (epitopes), each monoclonal antibody is directed against a single determinant on the antigen. In addition to their specificity, the monoclonal antibodies are advantageous in that they are synthesized by the hybridoma culture, uncontaminated by other immunoglobulins. The modifier "monoclonal" indicates the character of the antibody as being obtained from a substantially homogeneous population of antibodies, and is not to be construed as requiring production of the antibody by any particular method. For example, the monoclonal antibodies to be used in accordance with the present invention may be made by the hybridoma method first described by Kohler et al., Nature, 256: 495 (1975), or may be made by recombinant DNA methods (see, e.g., U. S. Patent No. 4,816, 567). The"monoclonal antibodies"may also be isolated from phage antibody libraries using the techniques described in Clackson et al., Nature, 352: 624-628 (1991) and Marks et al., J. Mol. Biol., 222: 581-597 (1991), for example.

The monoclonal antibodies herein specifically include "chimeric" antibodies (immunoglobulins) in which a portion of the heavy and/or light chain is identical with or homologous to corresponding sequences in antibodies derived from a particular species or belonging to a particular antibody class or subclass, while the remainder of the chain (s) is (are) identical with or homologous to corresponding sequences in antibodies derived from another species or belonging to another antibody class or subclass, as well as fragments of such antibodies, so long as they exhibit the desired biological activity (U. S. Patent No. 4,816, 567; Morrison et al., Proc. Natl. Acad. Sci. USA, 81: 6851-6855 (1984)). Chimeric antibodies of interest herein include"primitized"antibodies comprising variable domain antigen-binding sequences derived from a non-human primate (e.g., Old World Monkey, Ape etc.) and human contant region sequences.

"Humanized" forms of non-human (e.g., murine) antibodies are chimeric immunoglobulins, immunoglobulin chains or fragments thereof (such as Fv, Fab, Fab', F (ab') 2 or other antigen-binding subsequences of antibodies) of mostly human sequences, which contain minimal sequence derived from non-human immunoglobulin. For the most part, humanized antibodies are human immunoglobulins (recipient antibody) in which residues from a hypervariable region (also CDR) of the recipient are replaced by residues from a hypervariable region of a non-human species (donor antibody) such as mouse, rat or rabbit having the desired specificity, affinity, and capacity. In some instances, Fv framework region (FR) residues of the human immunoglobulin are replaced by corresponding non-human residues. Furthermore, "humanized antibodies"as used herein may also comprise residues which are found neither in the recipient antibody nor the donor antibody. These modifications are made to further refine and optimize antibody performance. The humanized antibody optimally also will comprise at least a portion of an immunoglobulin constant region (Fc), typically that of a human immunoglobulin. For further details, see Jones et al., Nature, 321: 522-525 (1986); Reichmann et al., Nature, 332: 323-329 (1988); and Presta, Curr. Op. Struct. Biol., 2: 593-596 (1992).

The term "human antibody" includes antibodies having variable and constant regions corresponding substantially to human germline immunoglobulin sequences known in the art, including, for example, those described by Kabat et al. (See Kabat, et al. (1991) loc. cit.). The human antibodies of the invention may include amino acid residues not encoded by human germline immunoglobulin sequences (e.g., mutations introduced by random or site-specific mutagenesis in vitro or by somatic mutation in vivo), for example in the CDRs, and in particular, CDR3. The human antibody can have at least one, two, three, four, five, or more positions replaced with an amino acid residue that is not encoded by the human germline immunoglobulin sequence.

As used herein, "in vitro generated antibody" refers to an antibody where all or part of the variable region (e.g., at least one CDR) is generated in a non-immune cell selection (e.g., an in vitro phage display, protein chip or any other method in which candidate sequences can be tested for their ability to bind to an antigen). This term thus preferably excludes sequences generated by genomic rearrangement in an immune cell.

A "bispecific" or "bifunctional antibody" is an artificial hybrid antibody having two different heavy/light chain pairs and two different binding sites. Bispecific antibodies can be produced by a variety of methods including fusion of hybridomas or linking of Fab' fragments. See, e.g., Songsivilai & Lachmann, Clin. Exp. Immunol. 79:315-321 (1990); Kostelny et al., J. Immunol. 148, 1547-1553 (1992). In one embodiment, the bispecific antibody comprises a first binding domain polypeptide, such as a Fab' fragment, linked via an immunoglobulin constant region to a second binding domain polypeptide. Numerous methods known to those skilled in the art are available for obtaining antibodies or antigen-binding fragments thereof. For example, antibodies can be produced using recombinant DNA methods (U.S. Patent 4,816,567). Monoclonal antibodies may also be produced by generation of hybridomas (see e.g., Kohler and Milstein (1975) Nature, 256: 495-499) in accordance with known methods. Hybridomas formed in this manner are then screened using standard methods, such as enzyme-linked immunosorbent assay (ELISA) and surface plasmon resonance (BIACORE™) analysis, to identify one or more hybridomas that produce an antibody that specifically binds with a specified antigen. Any form of the specified antigen may be used as the immunogen, e.g., recombinant antigen, naturally occurring forms, any variants or fragments thereof, as well as antigenic peptide thereof.

One exemplary method of making antibodies includes screening protein expression libraries, e.g., phage or ribosome display libraries. Phage display is described, for example, in Ladner etal., U.S. Patent No. 5,223,409; Smith (1985) Science 228:1315-1317; Clackson et al. (1991) Nature, 352: 624-628; Marks et al. (1991) J. Mol. Bio!., 222: 581-597WO 92/18619; WO 91/17271; WO 92/20791 ; WO 92/15679; WO 93/01288; WO 92/01047; WO 92/09690; and WO 90/02809.

In addition to the use of display libraries, the specified antigen can be used to immunize a non-human animal, e.g., a rodent, e.g., a mouse, hamster, or rat. In one embodiment, the non-human animal includes at least a part of a human immunoglobulin gene. For example, it is possible to engineer mouse strains deficient in mouse antibody production with large fragments of the human Ig loci. Using the hybridoma technology, antigen-specific monoclonal antibodies derived from the genes with the desired specificity may be produced and selected. See, e.g., XENOMOUSE™, Green etal. (1994) Nature Genetics 7:13-21, US 2003- 0070185, WO 96/34096, and WO96/33735.

In another embodiment, a monoclonal antibody is obtained from the non-human animal, and then modified, e.g., humanized, deimmunized, chimeric, may be produced using recombinant DNA techniques known in the art. A variety of approaches for making chimeric antibodies have been described. See e.g., Morrison et al., Proc. Natl. Acad. ScL U.S.A. 81:6851 , 1985; Takeda et al., Nature 314:452, 1985, Cabilly et al., U.S. Patent No. 4,816,567; Boss et al., U.S. Patent No. 4,816,397; Tanaguchi et al., EP 171496; EP 173494, GB 2177096. Humanized antibodies may also be produced, for example, using transgenic mice that express human heavy and light chain genes, but are incapable of expressing the endogenous mouse immunoglobulin heavy and light chain genes. Winter describes an exemplary CDR-grafting method that may be used to prepare the humanized antibodies described herein (U.S. Patent No. 5,225,539). All of the CDRs of a particular human antibody may be replaced with at least a portion of a non-human CDR, or only some of the CDRs may be replaced with non-human CDRs. It is only necessary to replace the number of CDRs required for binding of the humanized antibody to a predetermined antigen.

Humanized antibodies or fragments thereof can be generated by replacing sequences of the Fv variable domain that are not directly involved in antigen binding with equivalent sequences from human Fv variable domains. Exemplary methods for generating humanized antibodies or fragments thereof are provided by Morrison (1985) Science 229:1202-1207; by Oi et al. (1986) BioTechniques 4:214; and by US 5,585,089; US 5,693,761; US 5,693,762; US 5,859,205; and US 6,407,213. Those methods include isolating, manipulating, and expressing the nucleic acid sequences that encode all or part of immunoglobulin Fv variable domains from at least one of a heavy or light chain. Such nucleic acids may be obtained from a hybridoma producing an antibody against a predetermined target, as described above, as well as from other sources. The recombinant DNA encoding the humanized antibody molecule can then be cloned into an appropriate expression vector.

In certain embodiments, a humanized antibody is optimized by the introduction of conservative substitutions, consensus sequence substitutions, germline substitutions and/or backmutations. Such altered immunoglobulin molecules can be made by any of several techniques known in the art, (e.g., Teng et al., Proc. Natl. Acad. Sci. U.S.A., 80: 7308-7312, 1983; Kozbor etal, Immunology Today, 4: 7279, 1983; Olsson et al., Meth. Enzymol., 92: 3-16, 1982), and may be made according to the teachings of WO 92/06193 or EP 239400).

An antibody or fragment thereof may also be modified by specific deletion of human T cell epitopes or "deimmunization" by the methods disclosed in WO 98/52976 and WO 00/34317. Briefly, the heavy and light chain variable domains of an antibody can be analyzed for peptides that bind to MHC Class II; these peptides represent potential T-cell epitopes (as defined in WO 98/52976 and WO 00/34317). For detection of potential T-cell epitopes, a computer modeling approach termed "peptide threading" can be applied, and in addition a database of human MHC class II binding peptides can be searched for motifs present in the VH and VL sequences, as described in WO 98/52976 and WO 00/34317. These motifs bind to any of the 18 major MHC class II DR allotypes, and thus constitute potential T cell epitopes. Potential T-cell epitopes detected can be eliminated by substituting small numbers of amino acid residues in the variable domains, or preferably, by single amino acid substitutions. Typically, conservative substitutions are made. Often, but not exclusively, an amino acid common to a position in human germline antibody sequences may be used. Human germline sequences, e.g., are disclosed in Tomlinson, et at. (1992) J. Mol. Biol. 227:776-798; Cook, G. P. etai. (1995) Immunol. Today Vol. 16 (5): 237-242; Chothia, et al. (1992) J. Mol. Biol. 227:799-817; and Tomlinson et al. (1995) EMBO J. 14:4628-4638. The V BASE directory provides a comprehensive directory of human immunoglobulin variable region sequences (compiled by Tomlinson, LA. etal. MRC Centre for Protein Engineering, Cambridge, UK). These sequences can be used as a source of human sequence, e.g., for framework regions and CDRs. Consensus human framework regions can also be used, e.g., as described in U.S. Patent No. 6,300,064.

It is known that an antibody may exert effector functions. Accordingly, it is envisaged that an antibody of the invention can exert one or more effector functions due to its immunoglobulin constant or Fc region. Alternatively, in certain embodiments it is envisaged that an antibody can contain an altered immunoglobulin constant or Fc region. For example, an antibody produced in accordance with the teachings herein may bind more strongly or with more specificity to effector molecules such as complement and/or Fc receptors, which can control several immune functions of the antibody such as effector cell activity, lysis, complement-mediated activity, antibody clearance, and antibody half-life. Typical Fc receptors that bind to an Fc region of an antibody (e.g., an IgG antibody) include, but are not limited to, receptors of the FcγRI, FcγRII, and FcγRIII and FcRn subclasses, including allelic variants and alternatively spliced forms of these receptors. Fc receptors are reviewed in Ravetch and Kinet, Annu. Rev. Immunol 9:457-92, 1991; Capel et al., Immunomethods 4:25-34, 1994; and de Haas et al., J. Lab. Clin. Med. 126:330-41 , 1995).

Antibody "effector functions" refer to those biological activities attributable to the Fc region (a native sequence Fc region or amino acid sequence variant Fc region) of an antibody, and vary with the antibody isotype. Examples of antibody effector functions include: Clq binding and complement dependent cytotoxicity (CDC); Fc receptor binding; antibody-dependent cell-mediated cytotoxicity (ADCC); phagocytosis; down regulation of cell surface receptors (e.g., B cell receptors); and B cell activation. In order to exert effector functions an antibody, so to say, recruits effector cells.

"Complement dependent cytotoxicity" or "CDC" refers to the lysis of a target cell in the presence of complement. Activation of the classical complement pathway is initiated by the binding of the first component of the complement system (Clq) to antibodies (of the appropriate subclass) which are bound to their cognate antigen. To assess complement activation, a CDC assay, e.g., as described in Gazzano-Santoro et al., J. Immunol. Methods 202: 163 (1996) may be performed.

"Antibody-dependent cell-mediated cytotoxicity" or ADCC refers to a form of cytotoxicity in which secreted Ig bound onto Fc receptors (FcRs) present on certain cytotoxic cells (e.g., natural killer (NK) cells, neutrophils and macrophages) enable these cytotoxic effector cells to bind specifically to an antigen-bearing target cell and subsequently kill the target cell with cytotoxins. The antibodies"arm"the cytotoxic cells and are required for killing of the target cell by this mechanism. The primary cells for mediating ADCC, NK cells, express FcγRE only, whereas monocytes express FcγRI, FcγRII and FcγRIII. Fc expression on hematopoietic cells is summarized in Table 3 on page 464 of Ravetch and Kinet, Annu. Rev. Immunol. 9: 457-92 (1991). To assess ADCC activity of a molecule of interest, an in vitro ACDD assay, such as that described in U. S. Patent No. 5,500, 362

or 5,821, 337 may be performed. Useful effector cells for such assays include peripheral blood mononuclear cells (PBMC) and natural killer (NK) cells. Alternatively, or additionally, ADCC activity of the molecule of interest may be assessed in vivo, e.g. , in an animal model such as that disclosed in Clynes et al., PNAS USA 95: 652-656 (1998).

"Effector cells", preferably human effector cells are leukocytes which express one or more FcRs and perform effector functions. Preferably, the cells express at least FcyRm and perform ADCC effector function. Examples of human leukocytes which mediate ADCC include peripheral blood mononuclear cells (PBMC), natural killer (NK) cells, monocytes, cytotoxic T cells and neutrophils, with and MNK cells being preferred. The effector cells may be isolated from a native source, e.g., blood.

Techniques for the production of antibodies, including polyclonal, monoclonal, humanized, bispecific and heteroconjugate antibodies follow.

### 1) Polyclonal antibodies.

Polyclonal antibodies are generally raised in animals by multiple subcutaneous (sc) or intraperitoneal (ip) injections of the relevant antigen and an adjuvant. It may be useful to conjugate the relevant antigen to a protein that is immunogenic in the species to be immunized, e.g., keyhole limpet hemocyanin (KLH), serum albumin, bovine thyroglobulin, or soybean trypsin inhibitor, using a bifunctional or derivatizing agent, e.g., maleimidobenzoyl sulfosuccinimide ester (conjugation through cysteine residues), N-hydroxysuccinimide (through lysien residues), glutaraldehyde, succinic anhydride. Examples of adjuvants which may be employed include Freund's complete adjuvant and MPL-TDM adjuvant (monophosphoryl Lipid A, synthetic trehalose dicorynomycolate). The immunization protocol may be selected by one skilled in the art without undue experimentation.

For example, the animals are immunized against the antigen, immunogenic conjugates, or derivatives by combining the protein or conjugate (for rabbits or mice, respectively) with 3 volumes of Freund's complete adjuvant and injecting the solution intradermally at multiple sites. One month later, the animals are boosted with 1/5 to 1/10 the original amount of peptide or conjugate in Freund's complete adjuvant by subcutaneous injection at multiple sites. Seven to fourteen days later, the animals are bled and the serum is assayed for antibody titer. Animals are boosted until the titer plateaus. Conjugates also can be made in recombinant cell culture as protein fusions. Also, aggregating agents such as alum are suitable used to enhance the immune response.

However, in a preferred aspect, the present invention relates to a method for the generation of an antibody against Syndecan-4, preferably human Syndecan-4 which inhibits phosphorylation of ERK 1/2 by Syndecan-4, comprising
(a) immunizing a non-human mammal with a polypeptide comprising amino acids 93 to 121 of the amino acid sequence shown in SEQ ID NO:1 or with a polypeptide comprising amino acids 92 to 122 of the amino acid sequence shown in SEQ ID NO:2;
(b) isolating an antibody obtained in step(a); and
(c) screening (including determing) whether the antibody obtained in step (b) inhibits phosphorylation of ERK 1/2 by Syndecan-4.

Likewise, the present invention relates to the use of a polypeptide comprising amino acids 93 to 121 of the amino acid sequence shown in SEQ ID NO:1 or a polypeptide comprising amino acids 92 to 122 of the amino acid sequence shown in SEQ ID NO:2 for immunizing a non-human animal. Said use is preferably for generating a monoclonal or polyclonal antibody against said polypeptide.

The term "immunizing" refers to the step or steps of administering one or more antigens (in case of the present invention a Syndecan-4 protein or one or more immunogenic fragments thereof) to a non-human animal so that antibodies can be raised in the animal.

The terms "antigen "and "immunogen" are used interchangeably herein to refer to a molecule or substance which induces an immune response (preferably an antibody response) in an animal, preferably a non-human animal immunized therewith (i.e. the antigen is "immunogenic" in the animal). In case of the present invention when an antibody is generated against Syndecan-4 the antigen is preferably a Syndecan-4 protein, immunogenic peptide or fragment thereof or a nucleic acid encoding and expressing Syndecan-4. The Syndecan-4 antigen may be naturally-occurring or recombinantly produced.

Preferably, the antigen used for immunizing a non-human animal is a purified antigen. A "purified" antigen is one which has been subjected to one or more purification procedures. The purified antigen may be "homogeneous", which is used herein to refer to a composition comprising at least about 70% to about 100% by weight of the antigen of interest, based on total weight of the composition, preferably at least about 80% to about 100% by weight of the antigen of interest.

Generally, immunizing comprises injecting the antigen or antigens into the non-human animal. Immunization may involve one or more administrations of the antigen or antigens.

Specifically, the non-human animal is preferably immunized at least two, more preferably three times with said polypeptide (antigen), optionally in admixture with an adjuvant. An "adjuvant" is a nonspecific stimulant of the immune response.

The adjuvant may be in the form of a composition comprising either or both of the following components: (a) a substance designed to form a deposit protecting the antigen (s) from rapid catabolism (e.g. mineral oil, alum, aluminium hydroxide, liposome or surfactant (e.g. pluronic polyol) and (b) a substance that nonspecifically stimulates the immune response of the immunized host animal (e.g. by increasing lymphokine levels therein).

Exemplary molecules for increasing lymphokine levels include lipopolysaccaride (LPS) or a Lipid A portion thereof; Bordetalla pertussis; pertussis toxin; Mycobacterium tuberculosis; and muramyl dipeptide (MDP). Examples of adjuvants include Freund's adjuvant (optionally comprising killed M. tuberculosis; complete Freund's adjuvant); aluminium hydroxide adjuvant; and monophosphoryl Lipid A-synthetic trehalose dicorynomylcolate (MPL-TDM).

The "non-human animal" to be immunized herein is preferably a rodent. A "rodent" is an animal belonging to the rodentia order of placental mammals. Exemplary rodents include mice, rats, guinea pigs, squirrels, hamsters, ferrets etc, with mice being the preferred rodent for immunizing according to the method herein.

Other non-human animals which can be immunized herein include non-human primates such as Old World monkey (e.g. baboon or macaque, including Rhesus monkey and cynomolgus monkey ; see US Patent 5, 658, 570) ; birds (e.g. chickens); rabbits; goats; sheep; cows; horses; pigs; donkeys; dogs etc.

The antibody that can be obtained by the preferred method is a polyclonal antibody or polyclonal serum (e.g., obtainable from a rodent, more preferably from a rabbit, goat or sheep) or, if antibody-producing cells are isolated from the non-human animal, a monoclonal antibody (e.g., obtainable from a rodent, more preferably from a mouse, rat or sheep) can be produced as is commonly known in the art and described herein.

Accordingly, the invention provides a method for making monoclonal antibodies comprising the following steps: (a) immunizing an animal with two or more different antigens (i.e., fragments from one and the same polypeptide, in case of the present invention Syndecan-4) so as to generate polyclonal antibodies against each antigen in the animal; (b) preparing monoclonal antibodies using immune cells of the immunized animal which produce said polyclonal antibodies; and (c) screening said monoclonal antibodies to identify one or more monoclonal antibodies that bind to each antigen. In the screening step, one finds at least one monoclonal antibody against at least two different antigens. Preferably, at least one monoclonal antibody is found for each antigen with which the animal was immunized.

Preferably, the animal is immunized with a composition comprising a mixture of the two or more different antigens; and step (b) comprises fusing immune cells from the immunized animal with myeloma cells in order to generate hybridoma cell lines producing the monoclonal antibodies.

The term "immune cells" refers to cells which are capable of producing antibodies. The immune cells of particular interest herein are lymphoid cells derived, e.g. from spleen, peripheral blood lymphoctes (PBLs), lymph node, inguinal node, Peyers patch, tonsil, bone marrow, cord blood, pleural effusions and tumor-infiltrating lymphocytes (TIL).

By "screening" is meant subjecting one or more monoclonal antibodies (e.g., purified antibody and/or hybridoma culture supernatant comprising the antibody) to one or more assays which determine qualitatively and/or quantitatively the ability of an antibody to bind to an antigen of interest.

By "immuno-assay" is meant an assay that determines binding of an antibody to an antigen, wherein either the antibody or antigen, or both, are optionally adsorbed on a solid phase (i. e., an "immunoadsorbent" assay) at some stage of the assay. Exemplary such assays include ELISAs, radioimmunoassays (RIAs), and FACS assays.

Given the above, the present invention provides thus a monoclonal or polyclonal antibody obtainable by the aforedescribed methods for the generation of an antibody, i.e., by immunizing a non-human animal as described before. Hence, the term "antibody" when used herein also encompasses an antibody (monoclonal or polyclonal) obtainable by the methods for the generation of an antibody against Syndecan-4, preferably human Syndecan-4.

### 2) Monoclonal antibodies.

Monoclonal antibodies are obtained from a population of substantially homogeneous antibodies, i.e., the individual antibodies comprising the population are identical except for possible naturally occurring mutations and/or post-translational modifications (e.g., isomerizations, amidations) that may be present in minor amounts. Thus, the modifier "monoclonal"indicates the character of the antibody as not being a mixture of discrete antibodies.

For example, the monoclonal antibodies may be made using the hybridoma method first described by Kohler et al., Nature, 256: 495 (1975), or may be made by recombinant DNA methods (U. S. Patent No. 4,816, 567).

In the hybridoma method, a mouse or other appropriate host animal, such as a hamster, is immunized as hereinabove described to elicit lymphocytes that produce or are capable of producing antibodies that will specifically bind to the protein used for immunization. Alternatively, lymphocytes may be immunized in vitro. Lymphocytes then are fused with myeloma cells using a suitable fusing agent, such as polyethylene glycol, to form a hybridoma cell (Goding, Monoclonal Antibodies: Principles and Practice, pp. 59-103 (Academic Press, 1986).

The immunizing agent will typically include the antigenic protein or a fusion variant thereof. Generally either peripheral blood lymphocytes ("PBLs") are used if cells of human origin are desired, or spleen cells or lymph node cells are used if non-human mammalian sources are desired. The lymphoctyes are then fused with an immortalized cell line using a suitable fusing agent, such as polyethylene glycol, to form a hybridoma cell. Goding, Monoclonal Antibodies: Principles and Practice, Academic Press (1986), pp. 59-103.

Immortalized cell lines are usually transformed mammalian cell, particularly myeloma cells of rodent, bovine and human origin. Usually, rat or mouse myeloma cell lines are employed. The hybridoma cells thus prepared are seeded and grown in a suitable culture medium that preferably contains one or more substances that inhibit the growth or survival of the unfused, parental myeloma cells. For example, if the parental myeloma cells lack the enzyme hypoxanthine guanine phosphoribosyl transferase (HGPRT or HPRT), the culture medium for the hybridomas typically will include hypoxanthine, aminopterin, and thymidine (HAT medium), which substances prevent the growth of HGPRT-deficient cells.

Preferred immortalized myeloma cells are those that fuse efficiently, support stable high-level production of antibody by the selected antibody-producing cells, and are sensitive to a medium such as HAT medium. Among these, preferred are murine myeloma lines, such as those derived from MOPC-21 and MPC-11 mouse tumors available from the Salk Institute Cell Distribution Center, San Diego, California USA, and SP-2 cells (and derivatives thereof, e.g. , X63-Ag8-653) available from the American Type Culture Collection, Manassus, Virginia USA. Human myeloma and mouse-human heteromyeloma cell lines also have been described for the production of human monoclonal antibodies (Kozbor, J. Immunol., 133: 3001 (1984); Brodeur et al., Monoclonal Antibody Production Techniques and Applications, pp. 51-63 (Marcel Dekker, Inc., New York, 1987)).

Culture medium in which hybridoma cells are growing is assayed for production of monoclonal antibodies directed against the antigen. Preferably, the binding specificity of monoclonal antibodies produced by hybridoma cells is determined by immunoprecipitation or by an in vitro binding assay, such as radioimmunoassay (RIA) or enzyme-linked immunoabsorbent assay (ELISA).

The culture medium in which the hybridoma cells are cultured can be assayed for the presence of monoclonal antibodies directed again desired antigen. Preferably, the binding affinity and specificity of the monoclonal antibody can be determined by immunoprecipitation or by an in vitro binding assay, such as radioimmunoassay (RIA) or enzyme-linked assay (ELISA). Such techniques and assays are known in the in art. For example, binding affinity may be determined by the Scatchard analysis of Munson et al., Anal. Biochem., 107: 220 (1980).

After hybridoma cells are identified that produce antibodies of the desired specificity, affinity, and/or activity, the clones may be subcloned by limiting dilution procedures and grown by standard methods (Goding, supra). Suitable culture media for this purpose include, for example, D-MEM or RPMI-1640 medium. In addition, the hybridoma cells may be grown in vivo as ascites tumors in a mammal.

The monoclonal antibodies secreted by the subclones are suitably separated from the culture medium, ascites fluid, or serum by conventional immunoglobulin purification procedures such as, for example, protein A-Sepharose, hydroxylapatite chromatography, gel electrophoresis, dialysis, or affinity chromatography.

Monoclonal antibodies may also be made by recombinant DNA methods, such as those described in U. S. Patent No. 4,816, 567, and as described above. DNA encoding the monoclonal antibodies is readily isolated and sequenced using conventional procedures (e.g., by using oligonucleotide probes that are capable of binding specifically to genes encoding the heavy and light chains of murine antibodies). The hybridoma cells serve as a preferred source of such DNA. Once isolated, the DNA may be placed into expression vectors, which are then transfected into host cells such as E. coli cells, simian COS cells, Chinese hamster ovary (CHO) cells, or myeloma cells that do not otherwise produce immunoglobulin protein, in order to synthesize monoclonal antibodies in such recombinant host cells. Review articles on recombinant expression in bacteria of DNA encoding the antibody include Skerra et al., Curr. Opinion in Immunol., 5: 256-262 (1993) and Pluckthun, Immunol. Revs. 130: 151-188 (1992).

In a further embodiment, antibodies can be isolated from antibody phage libraries generated using the techniques described in McCafferty et al., Nature, 348: 552-554 (1990).

Clackson et al., Nature, 352: 624-628 (1991) and Marks et al., J. Mol. Biol., 222: 581-597 (1991) describe the isolation of murine and human antibodies, respectively, using phage libraries. Subsequent publications describe the production of high affinity (nM range) human antibodies by chain shuffling (Marks et al., Bioll'eclanology, 10: 779-783 (1992)), as well as combinatorial infection and in vivo recombination as a strategy for constructing very large phage libraries (Waterhouse et al., Nucl. Acids Res., 21: 2265-2266 (1993)). Thus, these techniques are viable alternatives to traditional monoclonal antibody hybridoma techniques for isolation of monoclonal antibodies.
The DNA also may be modified, for example, by substituting the coding sequence for human heavy-and light-chain constant domains in place of the homologous murine sequences (U. S. Patent No. 4,816, 567; Morrison, et al., Proc. Natl Acad. Sci. USA, 81: 6851 (1984)), or by covalently joining to the immunoglobulin coding sequence all or part of the coding sequence for a non-immunoglobulin polypeptide. Typically such non-immunoglobulin polypeptides are substituted for the constant domains of an antibody, or they are substituted for the variable domains of one antigen-combining site of an antibody to create a chimeric bivalent antibody comprising one antigen-combining site having specificity for an antigen and another antigen-combining site having specificity for a different antigen.
The monoclonal antibodies described herein may by monovalent, the preparation of which is well known in the art. For example, one method involves recombinant expression of immunoglobulin light chain and a modified heavy chain. The heavy chain is truncated generally at any point in the Fc region so as to prevent heavy chain crosslinking. Alternatively, the relevant cysteine residues may be substituted with another amino acid residue or are deleted so as to prevent crosslinking. In vitro methods are also suitable for preparing monovalent antibodies. Digestion of antibodies to produce fragments thereof, particularly Fab fragments, can be accomplished using routine techniques known in the art.
Chimeric or hybrid antibodies also may be prepared in vitro using known methods in synthetic protein chemistry, including those involving crosslinking agents. For example, immunotoxins may be constructed using a disulfide-exchange reaction or by forming a thioether bond. Examples of suitable reagents for this purpose include iminothiolate and methyl-4-mercaptobutyrimidate.

### 3) Humanized antibodies

The antibodies of the invention may further comprise humanized or human antibodies. Humanized forms of non-human (e.g., murine) antibodies are chimeric immunoglobulins, immunoglobulin chains or fragments thereof (such as Fv, Fab, Fab', F (ab') 2 or other antigen- binding subsequences of antibodies) which contain minimal sequence derived from non-human immunoglobulin. Humanized antibodies include human immunoglobulins (recipient antibody) in which residues from a complementarity determining region (CDR) of the recipient are replaced by residues from a CDR of a non-human species (donor antibody) such as mouse, rat or rabbit having the desired specificity, affinity and capacity. In some instances, Fv framework residues of the human immunoglobulin are replaced by corresponding non-human residues.

Humanized antibodies may also comprise residues which are found neither in the recipient antibody nor in the imported CDR or framework sequences. In general, the humanized antibody will comprise substantially all of at least one, and typically two, variable domain, in which all or substantially all of the CDR regions correspond to those of a non-human immunoglobulin and all or substantially all of the FR regions are those of a human immunoglobulin consensus sequence. The humanized antibody optimally also will comprise at least a portion of an immunoglobulin constant region (Fc), typically that of a human immunoglobulin. Jones et al., Nature 321: 522-525 (1986); Riechmann et al., Nature 332: 323-329 (1988) and Presta, Curr. Opin. Struct. Biol. 2 : 593-596 (1992). Methods for humanizing non-human antibodies are well known in the art. Generally, a humanized antibody has one or more amino acid residues introduced into it from a source which is non-human. These non-human amino acid residues are often referred to as"import" residues, which are typically taken from an"import"variable domain. Humanization can be essentially performed following the method of Winter and coworkers, Jones et al., Nature 321: 522-525 (1986); Riechmann et al., Nature 332: 323-327 (1988); Verhoeyen et al., Science 239: 1534-1536 (1988), or through substituting rodent CDRs or CDR sequences for the corresponding sequences of a human antibody. Accordingly, such"humanized"antibodies are chimeric antibodies (U. S. Patent No. 4, 816, 567), wherein substantially less than an intact human variable domain has been substituted by the corresponding sequence from a non-human species. In practice, humanized antibodies are typically human antibodies in which some CDR residues and possibly some FR residues are substituted by residues from analogous sites in rodent antibodies.

The choice of human variable domains, both light and heavy, to be used in making the humanized antibodies is very important to reduce antigenicity. According to the so-called "best-fit"method, the sequence of the variable domain of a rodent antibody is screened against the entire library of known human variable-domain sequences. The human sequence which is closest to that of the rodent is then accepted as the human framework (FR) for the humanized antibody. Sims et al., J. Immunol.) 151: 2296 (1993); Chothia et al., J. Mol. Biol., 196: 901 (1987). Another method uses a particular framework derived from the consensus sequence of all human antibodies of a particular subgroup of light or heavy chains. The same framework may be used for several different humanized antibodies. Carter et al., Proc. Natl. Acad. Sci. USA, 89 : 4285 (1992); Presta et al., J. Immunol., 151: 2623 (1993).

It is further important that antibodies be humanized with retention of high affinity for the antigen and other favorable biological properties. To achieve this goal, according to a preferred method, humanized antibodies are prepared by a process of analysis of the parental sequences and various conceptual humanized products using three-dimensional models of the parental and humanized sequences. Three-dimensional immunoglobulin models are commonly available and are familiar to those skilled in the art. Computer programs are available which illustrate and display probable three-dimensional conformational structures of selected candidate immunoglobulin sequences. Inspection of these displays permits analysis of the likely role of the residues in the functioning of the candidate immunoglobulin sequence, i.e., the analysis of residues that influence the ability of the candidate immunoglobulin to bind its antigen. In this way, FR residues can be selected and combined from the recipient and import sequences so that the desired antibody characteristic, such as increased affinity for the target antigen (s), is achieved. In general, the CDR residues are directly and most substantially involved in influencing antigen binding.

Various forms of the humanized antibody are contemplated. For example, the humanized antibody may be an antibody fragment, such as an Fab, which is optionally conjugated with one or more cytotoxic agent (s) in order to generate an immunoconjugate.

Alternatively, the humanized antibody may be an intact antibody, such as an intact IgGI antibody.

### 4) Human antibodies

As an alternative to humanization, human antibodies can be generated. For example, it is now possible to produce transgenic animals (e.g., mice) that are capable, upon immunization, of producing a full repertoire of human antibodies in the absence of endogenous immunoglobulin production. For example, it has been described that the homozygous deletion of the antibody heavy-chain joining region (JH) gene in chimeric and germ-line mutant mice results in complete inhibition of endogenous antibody production. Transfer of the human germ- line immunoglobulin gene array in such germ-line mutant mice will result in the production of human antibodies upon antigen challenge. See, e.g., Jakobovits et al., Proc. Natl. Acad. Sci. USA, 90: 2551 (1993); Jakobovits et al., Nature, 362: 255-258 (1993); Bruggermann et al., Year in Immun., 7: 33 (1993); U. S. Patent Nos. 5,591, 669 and WO 97/17852.

Alternatively, phage display technology can be used to produce human antiobdies and antibody fragments in vitro, from immunoglublin variable (V) domain gene repertoires from unimmunized donors. McCafferty et al., Nature 348: 552-553 (1990); Hoogenboom and Winter, J. Mol. Biol. 227: 381 (1991). According to this technique, antibody V domain genes are cloned in-frame into either a major or minor coat protein gene of a filamentous bacteriophage, such as M13, and displayed as functional antibody fragments on the surface of the phage particle. Because the filamentous particle contains a single-stranded DNA copy of the phage genome, selections based on the functional properties of the antibody also result in seletion of the gene encoding the antibody exhibiting those properties. Thus, the phage mimics some of the properties of the B-cell. Phage display can be performed in a variety of formats, reviewed in, e.g., Johnson, Kevin S. and Chiswell, David J., Curr. Opin Struct. Biol. 3: 564-571 (1993). Several sources of V-gene segments can be used for phage display. Clackson et al., Nature 352: 624-628 (1991) isolated a diverse array of anti-oxazolone antibodies from a small random combinatorial library of V genes derived from the spleens of immunized mice. A repertoire of V genes from unimmunized hman donors can be constructed and antibodies to a diverse array of antigens (including self-antigens) can be isoalted essentially following the technqieus described by Marks et al. , J. Mol. Biol. 222: 581-597 (1991), or Griffith et al. , EMBO J. 12 : 725-734 (1993). See also, U. S. Patent. Nos. 5,565, 332 and 5,573, 905.

The techniques of Cole et al., and Boerner et al., are also available for the preparation of human monoclonal antibodies (Cole et al., Monoclonal Antibodies and Cancer Therapy, Alan R. Liss, p. 77 (1985) and Boerner et al., J. Immunol. 147 (1) : 86-95 (1991). Similarly, human antibodies can be made by introducing human immunoglobulin loci into transgenic animals, e.g., mice in which the endogenous immunoglobulin genes have been partially or completely inactivated. Upon challenge, human antibody production is observed, which closely resemble that seen in human in all respects, including gene rearrangement, assembly and antibody repertoire. This approach is described, for example, in U. S. Patent Nos. 5,545, 807; 5,545, 806,5, 569,825, 5,625, 126,5, 633,425, 5,661, 016 and in the following scientific publications: Marks et al., Bio/Technology 10: 779-783 (1992); Lonberg et al., Nature 368 : 856-859 (1994); Morrison, Nature 368: 812-13 (1994), Fishwild et al., Nature Biotechnology 14 : 845-51 (1996), Neuberger, Nature Biotechnology 14: 826 (1996) and Lonberg and Huszar, Intern. Rev. Immunol. 13: 65-93 (1995).

Finally, human antibodies may also be generated in vitro by activated B cells (see U. S. Patent Nos 5,567, 610 and 5,229, 275).

### 5) Antibody Fragments

In certain circumstances there are advantages to using antibody fragments, rather than whole antibodies. Smaller fragment size allows for rapid clearance, and may lead to improved access to solid tumors.

Various techniques have been developed for the production of antibody fragments.

Traditionally, these fragments were derived via proteolytic digestion of intact antibodies (see, e.g., Morimoto et al., J Biochem Biophys. Method. 24: 107-117 (1992); and Brennan et al., Science 229: 81 (1985)). However, these fragments can now be produced direclty by recombinant host cells. Fab, Fv and scFv antibody fragments can all be expressed in and secreted from E. coli, thus allowing the facile production of large amounts of these fragments.

Antibody fragments can be isolated from the antibody phage libraries discussed above. Alternatively, Fab'-SH fragments can be directly recovered from E. coli and chemically coupled to form F (ab') 2 fragments (Carter et aL, BiolTechnology 10 : 163-167 (1992)). According to another approach, F (ab') 2 fragments can be isolated directly from recombinant host cell culture. Fab and F (ab') 2 with increase in vivo half-life is described in U. S. Patent No. 5,869, 046. In other embodiments, the antibody of choice is a single chain Fv fragment (scFv).

See WO 93/16185; U. S. Patent No. 5,571, 894 and U. S. Patent No. 5,587, 458. The antibody fragment may also be a"linear antibody", e.g., as described in U. S. Patent 5,641, 870. Such linear antibody fragments may be monospecific or bispecific.

### 6) Bispecific and polyspecific antibodies

Bispecific antibodies (BsAbs) are antibodies that have binding specificities for at least two different epitopes, including those on the same or another protein. Alternatively, one arm can be armed to bind to the target antigen, and another arm can be combined with an arm that binds to a triggering molecule on a leukocyte such as a T-cell receptor molecule (e.g., CD3), or Fc receptors for IgG (FcyR) such as FcyRI (CD64), FcyRII (CD32) and FcyRin (CD16), so as to focus and localize cellular defense mechanisms to the target antigen-expressing cell. Such antibodies can be derived from full length antibodies or antibody fragments (e.g. F(ab')₂ bispecific antibodies).

Bispecific antibodies may also be used to localize cytotoxic agents to cells which express the target antigen. Such antibodies possess one arm that binds the desired antigen and another arm that binds the cytotoxic agent (e.g., methotrexate).

Methods for making bispecific antibodies are known in the art. Traditional production of full length bispecific antibodies is based on the coexpression of two immunoglobulin heavy chain-light chain pairs, where the two chains have different specificities. Millstein et al., Nature, 305: 537-539 (1983). Because of the random assortment of immunoglobulin heavy and light chains, these hybridomas (quadromas) produce a potential mixture of 10 different antibody molecules, of which only one has the correct bispecific structure. Purification of the correct molecule, which is usually done by affinity chromatography steps, is rather cumbersome, and the product yields are low. Similar procedures are disclosed in WO 93/08829 and in Traunecker et al., EMBO J. , 10: 3655-3659 (1991).

According to a different approach, antibody variable domains with the desired binding specificities (antibody-antigen combining sites) are fused to immunoglobulin constant domain sequences. The fusion preferably is with an immunoglobulin heavy chain constant domain, comprising at least part of the hinge, CH2, and CH3 regions. It is preferred to have the first heavy-chain constant region (CH1) containing the site necessary for light chain binding, present in at least one of the fusions. DNAs encoding the immunoglobulin heavy chain fusions and, if desired, the immunoglobulin light chain, are inserted into separate expression vectors, and are co-transfected into a suitable host organism. This provides for great flexibility in adjusting the mutual proportions of the three polypeptide fragments in embodiments when unequal ratios of the three polypeptide chains used in the construction provide the optimum yields. It is, however, possible to insert the coding sequences for two or all three polypeptide chains in one expression vector when the expression of at least two polypeptide chains in equal ratios results in high yields or when the ratios are of no particular significance.

In a preferred embodiment of this approach, the bispecific antibodies are composed of a hybrid immunoglobulin heavy chain with a first binding specificity in one arm, and a hybrid immunoglobulin heavy chain-light chain pair (providing a second binding specificity) in the other arm. It was found that this asymmetric structure facilitates the separation of the desired bispecific compound from unwanted immunoglobulin chain combinations, as the presence of an immunoglobulin light chain in only one half of the bispecific molecules provides for an easy way of separation. This approach is disclosed in WO 94/04690. For further details of generating bispecific antibodies, see, for example, Suresh et al., Methods in Enzymology 121: 210 (1986).

According to another approach described in WO 96/27011 or US 5,731,168, the interface between a pair of antibody molecules can be engineered to maximize the percentage of heterodimers which are recovered from recombinant cell culture. The preferred interface comprises at least a part of the CH3 region of an antibody constant domain. In this method, one or more small amino acid side chains from the interface of the first antibody molecule are replaced with larger side chains (e.g., tyrosine or tryptophan). Compensatory "cavities" of identical or similar size to the large side chains (s) are created on the interface of the second antibody molecule by replacing large amino acid side chains with smaller ones (e.g., alanine or threonine). This provides a mechanism for increasing the yield of the heterodimer over other unwanted end-products such as homodimers.

Techniques for generating bispecific antibodies from antibody fragments have been described in the literature. For example, bispecific antibodies can be prepared using chemical linkage. Brennan et al., Science 229: 81 (1985) describe a procedure wherein intact antibodies are proteolytically cleaved to generate F(ab')₂ fragments. These fragments are reduced in the presence of the dithiol complexing agent sodium arsenite to stabilize vicinal dithiols and prevent intermolecular disulfide formation. The Fab'fragments generated are then converted to thionitrobenzoate (TNB) derivatives. One of the Fab'-TNB derivatives is then reconverted to the Fab'-TNB derivative to form the bispecific antibody. The bispecific antibodies produced can be used as agents for the selective immobilization of enzymes.

Fab'fragments may be directly recovered from E. coli and chemically coupled to form bispecific antibodies. Shalaby et al., J. Exp. Med. 175: 217-225 (1992) describes the production of fully humanized bispecific antibody F (ab') 2 molecules. Each Fab'fragment was separately secreted from E. coli and subjected to directed chemical coupling in vitro to form the bispecific antibody. The bispecific antibody thus formed was able to bind to cells overexpressing the ErbB2 receptor and normal human T cells, as well as trigger the lytic activity of human cytotoxic lymphocytes against human breast tumor targets. Various techniques for making and isolating bivalent antibody fragments directly from recombinant cell culture have also been described. For example, bivalent heterodimers have been produced using leucine zippers. Kostelny et al., J. Immunol., 148 (5): 1547-1553 (1992).

The leucine zipper peptides from the Fos and Jun proteins were linked to the Fab'portions of two different antibodies by gene fusion. The antibody homodimers were reduced at the hinge region to form monomers and then re-oxidized to form the antibody heterodimers. The "diabody"technology described by Hollinger et al., Proc. Natl. Acad. Sci. USA, 90: 6444-6448 (1993) has provided an alternative mechanism for making bispecific/bivalent antibody fragments. The fragments comprise a heavy-chain variable domain (VH) connected to a light- chain variable domain (VL) by a linker which is too short to allow pairing between the two domains on the same chain. Accordingly, the VH and VL domains of one fragment are forced to pair with the complementary VL and VH domains of another fragment, thereby forming two antigen-binding sites. Another strategy for making bispecific/bivalent antibody fragments by the use of single-chain Fv (sFv) dimers has also been reported. See Gruber et al., J. Imnzunol., 152: 5368 (1994). Antibodies with more than two valencies are contemplated. For example, trispecific antibodies can be prepared. Tutt et al., J. Immunol. 147: 60 (1991).

Exemplary bispecific antibodies may bind to two different epitopes on a given molecule. Alternatively, an anti-protein arm may be combined with an arm which binds to a triggering molecule on a leukocyte such as a T-cell receptor molecule (e.g., CD2, CD3, CD28 or B7), or Fc receptors for IgG (FcyR), such as FcyRI (CD64), FcyRII (CD32) and FcyRIII (CD16) so as to focus cellular defense mechanisms to the cell expressing the particular protein.

Another bispecific antibody of interest binds the protein of interest and further binds Human Serum Albumine.

The "diabody" technology described by Hollinger et al. , Proc. Natl. Acad. Sci. USA, 90: 6444-6448 (1993) has provided an alternative mechanism for making bispecific antibody fragments. The fragments comprise a VH connected to a VL by a linker which is too short to allow pairing between the two domains on the same chain. Accordingly, the VH and VL domains of one fragment are forced to pair with the complementary VL and VH domains of another fragment, thereby forming two antigen-binding sites. Another strategy for making bispecific antibody fragments by the use of single-chain Fv (sFv) dimers has also been reported. See Gruber et al., J. Immunol., 152: 5368 (1994). Antibodies with more than two valencies are contemplated. For example, trispecific antibodies can be prepared. Tutt et al. J. Immunol. 147: 60 (1991).

A multivalent antibody may be internalized (and/or catabolized) faster than a bivalent antibody by a cell expressing an antigen to which the antibodies bind. The antibodies of the present invention can be multivalent antibodies (which are other than of the IgM class) with three or more antigen binding sites (e.g. tetravalent antibodies), which can be readily produced by recombinant expression of nucleic acid encoding the polypeptide chains of the antibody. The multivalent antibody can comprise a dimerization domain and three or more antigen binding sites. The preferred dimerization domain comprises (or consists of) an Fc region or a hinge region. In this scenario, the antibody will comprise an Fc region and three or more antigen binding sites amino-terminal to the Fc region. The preferred multivalent antibody herein comprises (or consists of) three to about eight, but preferably four, antigen binding sites. The multivalent antibody comprises at least one polypeptide chain (and preferably two polypeptide chains), wherein the polypeptide chain (s) comprise two or more variable domains. For instance, the polypeptide chain (s) may comprise VDI (X1ₙ-VD2- (X2)n-Fc, wherein VDI is a first variable domain, VD2 is a second variable domain, Fc is one polypeptide chain of an Fc region, X1 and X2 represent an amino acid or polypeptide, and n is 0 or 1. For instance, the polypeptide chain (s) may comprise: VH-CHI-flexible linker-VH-CHI-Fc region chain; or VH-CHI-VH-CHI-Fc region chain. The multivalent antibody herein preferably further comprises at least two (and preferably four) light chain variable domain polypeptides. The multivalent antibody herein may, for instance, comprise from about two to about eight light chain variable domain polypeptides. The light chain variable domain polypeptides contemplated here comprise a light chain variable domain and, optionally, further comprise a CL domain.

### 7) Heteroconjugate Antibodies

Heteroconjugate antibodies are also within the scope of the present invention.

Heteroconjugate antibodies are composed of two covalently joined antibodies. For example, one of the antibodies in the heteroconjugate can be coupled to avidin, the other to biotin. It is contemplated that the antibodies may be prepared in vitro using known methods in synthetic protein chemistry, including those involving crosslinking agents. For example, immunotoxins may be constructed using a disulfide exchange reaction or by forming a thioether bond. Examples of suitable reagents for this purpose include iminothiolate and methyl-4-mercaptobutyrimidate and those disclosed, for example, in U. S. Patent No. 4,676,980. Heteroconjugate antibodies may be made using any convenient cross-linking methods. Suitable cross-linking agents are well known in the art, and are disclosed in US Patent No. 4,676, 980, along with a number of cross-linking techniques.

For additional antibody production techniques, see Antibodies: A Laboratory Manual, eds. Harlow et al., Cold Spring Harbor Laboratory, 1988. The present invention is not necessarily limited to any particular source, method of production, or other special characteristics of an antibody.

The antibody of the present invention is preferably an "isolated" antibody. "Isolated" when used to describe antibodies disclosed herein, means an antibody that has been identified, separated and/or recovered from a component of its production environment. Preferably, the isolated antibody is free of association with all other components from its production environment. Contaminant components of its production environment, such as that resulting from recombinant transfected cells, are materials that would typically interfere with diagnostic or therapeutic uses for the polypeptide, and may include enzymes, hormones, and other proteinaceous or non-proteinaceous solutes. In preferred embodiments, the antibody will be purified (1) to a degree sufficient to obtain at least 15 residues of N-terminal or internal amino acid sequence by use of a spinning cup sequenator, or (2) to homogeneity by SDS-PAGE under non-reducing or reducing conditions using Coomassie blue or, preferably, silver stain. Ordinarily, however, an isolated antibody will be prepared by at least one purification step.

Amino acid sequence modifications of the Syndeca-4 antibodies described herein are contemplated. For example, it may be desirable to improve the binding affinity and/or other biological properties of the antibody. Amino acid sequence variants of the Syndecan-4 antibody are prepared by introducing appropriate nucleotide changes into the Syndecan-4 antibody nucleic acid, or by peptide synthesis.

Such modifications include, for example, deletions from, and/or insertions into, and/or substitutions of, residues within the amino acid sequences of the Syndecan antibody. Any combination of deletion, insertion, and substitution is made to arrive at the final construct, provided that the final construct possesses the desired characteristics. The amino acid changes also may alter post-translational processes of the Syndecan-4 antibody, such as changing the number or position of glycosylation sites.

A useful method for identification of certain residues or regions of the Syndecan-4 antibody that are preferred locations for mutagenesis is called "alanine scanning mutagenesis" as described by Cunningham and Wells in Science, 244: 1081-1085 (1989).

Here, a residue or group of target residues within the Syndeca-4 antibody are identified (e. g. , charged residues such as arg, asp, his, lys, and glu) and replaced by a neutral or negatively charged amino acid (most preferably alanine or polyalanine) to affect the interaction of the amino acids with epitope.

Those amino acid locations demonstrating functional sensitivity to the substitutions then are refined by introducing further or other variants at, or for, the sites of substitution. Thus, while the site for introducing an amino acid sequence variation is predetermined, the nature of the mutation per se need not be predetermined. For example, to analyze the performance of a mutation at a given site, ala scanning or random mutagenesis is conducted at a target codon or region and the expressed Syndecan-4 antibody variants are screened for the desired activity.

Amino acid sequence insertions include amino-and/or carboxyl-terminal fusions ranging in length from one, two, three, four, five, six, seven, eight, nine or ten residues to polypeptides containing a hundred or more residues, as well as intrasequence insertions of single or multiple amino acid residues. An insertional variant of the Syndecan-4 antibody molecule include the fusion to the N-or C-terminus of the antibody to an enzyme or a fusion to a polypeptide which increases the serum half-life of the antibody.

Another type of variant is an amino acid substitution variant. These variants have at least one, two, three, four, five, six, seven, eight, nine or ten amino acid residues in the Syndecan-4 antibody molecule replaced by a different residue. The sites of greatest interest for substitutional mutagenesis include the CDRs of the heavy and/or light chain, in particular the hypervariable regions, but FR alterations in the heavy and/or light chain are also contemplated.

For example, if a CDR sequence encompasses 6 amino acids, it is envisaged that one, two or three of these amino acids are substituted. Similarly, if a CDR sequence encompasses 15 amino acids it is envisaged that one, two, three, four, five or six of these amino acids are substituted.

Generally, if amino acids are substituted in one or more or all of the CDRs of the heavy and/or light chain, it is preferred that the then-obtained "substituted" sequence is at least 60%, more preferably 65%, even more preferably 70%, particularly preferable 75%, more particularly preferable 80% identical to the "original" CDR sequence. This means that it is dependent of the length of the CDR to which degree it is identical to the "substituted" sequence. For example, a CDR having 5 amino acids is preferably 80% identical to its substituted sequence in order to have at least one amino acid substituted. Accordingly, the CDRs of the Syndecan-4 antibody may have different degrees of identity to their substituted sequences, e.g., CDRL1 may have 80%, while CDRL3 may have 90%.

Preferred substitutions (or replacements) are conservative substitutions. However, any substitution (including non-conservative substitution or one or more from the "exemplary substitutions listed in Table 1, below) is envisaged as long as the Syndecan-4 antibody retains its capability to inhibit phosphorylation of ERK 1/2 by Syndecan-4 and/or its CDRs have an identity to the then substituted sequence (at least 60%, more preferably 65%, even more preferably 70%, particularly preferable 75%, more particularly preferable 80% identical to the "original" CDR sequence).

Conservative substitutions are shown in Table I under the heading of "preferred substitutions". If such substitutions result in a change in biological activity, then more substantial changes, denominated "exemplary substitutions" in Table 1, or as further described below in reference to amino acid classes, may be introduced and the products screened for a desired characteristic.

**TABLE I Amino Acid Substitutions**

| Original | Exemplary Substitutions | Preferred Substitutions |
|---|---|---|
| Ala (A) | val; leu; ile | Val |
| Arg (R) | lys: gln; asn | lys |
| Asn (N) | gln; his; asp, lys; arg | gln |
| Asp (D) | glu; asn | glu |
| Cys(C) | ser; ala | ser |
| Gln (Q) | asn: glu | asn |
| Glu (E) | asp; gln | asp |
| Gly (G) | ala | ala |
| His (H) | asn; gln; lys; arg | arg |
| Ile (I) | leu; val; met; all; phe; | leu |
| Leu(L) | norleucine; ile; val; met; ala; | ile |
| Lys (K) | arg; gin; asn | arg |
| Met(M) | leu; phe; ile | leu |
| Phe (F) | leu; val; ile; ala; tyr | tyr |
| Pro (P) | ala | ala |
| Ser (S) | thr | thr |
| Thr (T) | ser | ser |
| Tip (W) | tyr; phe | tyr |
| Tyr (Y) | trp; phe; thr; ser | Phe |
| Val (V) | ile; leu; met; phe; ala; | leu |

Substantial modifications in the biological properties of the antibody are accomplished by selecting substitutions that differ significantly in their effect on maintaining (a) the structure of the polypeptide backbone in the area of the substitution, for example, as a sheet or helical conformation, (b) the charge or hydrophobicity of the molecule at the target site, or (c) the bulk of the side chain. Naturally occurring residues are divided into groups based on common side-chain properties: (1) hydrophobic: norleucine, met, ala, val, leu, ile; (2) neutral hydrophilic: cys, ser, thr; (3) acidic: asp, glu; (4) basic: asn, gin, his, lys, arg; (5) residues that influence chain orientation: gly, pro; and (6) aromatic : trp, tyr, phe.

Non-conservative substitutions will entail exchanging a member of one of these classes for another class. Any cysteine residue not involved in maintaining the proper conformation of the Syndecan-4 antibody also may be substituted, generally with serine, to improve the oxidative stability of the molecule and prevent aberrant crosslinking. Conversely, cysteine bond (s) may be added to the antibody to improve its stability (particularly where the antibody is an antibody fragment such as an Fv fragment).

A particularly preferred type of substitutional variant involves substituting one or more hypervariable region residues of a parent antibody (e. g. a humanized or human antibody). Generally, the resulting variant (s) selected for further development will have improved biological properties relative to the parent antibody from which they are generated. A convenient way for generating such substitutional variants involves affinity maturation using phage display. Briefly, several hypervariable region sites (e. g. 6-7 sites) are mutated to generate all possible amino acid substitutions at each site. The antibody variants thus generated are displayed in a monovalent fashion from filamentous phage particles as fusions to the gene III product of M13 packaged within each particle. The phage-displayed variants are then screened for their biological activity (e. g. binding affinity) as herein disclosed. In order to identify candidate hypervariable region sites for modification, alanine scanning mutagenesis can be performed to identify hypervariable region residues contributing significantly to antigen binding. Alternatively, or additionally, it may be beneficial to analyze a crystal structure of the antigen-antibody complex to identify contact points between the antibody and, e.g., human Syndecan-4. Such contact residues and neighboring residues are candidates for substitution according to the techniques elaborated herein. Once such variants are generated, the panel of variants is subjected to screening as described herein and antibodies with superior properties in one or more relevant assays may be selected for further development.

Another type of amino acid variant of the antibody alters the original glycosylation pattern of the antibody. By altering is meant deleting one or more carbohydrate moieties found in the antibody, and/or adding one or more glycosylation sites that are not present in the antibody. Glycosylation of antibodies is typically either N-linked or 0-linked. N-linked refers to the attachment of the carbohydrate moiety to the side chain of an asparagine residue. The tripeptide sequences asparagine-X-serine and asparagine-X-threonine, where X is any amino acid except proline, are the recognition sequences for enzymatic attachment of the carbohydrate moiety to the asparagine side chain. Thus, the presence of either of these tripeptide sequences in a polypeptide creates a potential glycosylation site. 0-linked glycosylation refers to the attachment of one of the sugars N-aceylgalactosamine, galactose, or xylose to a hydroxyamino acid, most commonly serine or threonine, although 5-hydroxyproline or 5-hydroxylysine may also be used. Addition of glycosylation sites to the antibody is conveniently accomplished by altering the amino acid sequence such that it contains one or more of the above-described tripeptide sequences (for N-linked glycosylation sites). The alteration may also be made by the addition of, or substitution by, one or more serine or threonine residues to the sequence of the original antibody (for 0-linked glycosylation sites).

Other modifications of the antibody are contemplated herein. For example, the antibody may be linked to one of a variety of nonproteinaceous polymers, e. g., polyethylene glycol, polypropylene glycol, polyoxyalkylenes, or copolymers of polyethylene glycol and polypropylene glycol. The antibody also may be entrapped in microcapsules prepared, for example, by coacervation techniques or by interfacial polymerization (for example, hydroxymethylcellulose or gelatin-microcapsules and poly (methylmethacylate) microcapsules, respectively), in colloidal drug delivery systems (for example, liposomes, albumin microspheres, microemulsions, nano-particles and nanocapsules), or in macroemulsions. Such techniques are disclosed in Remington's Pharmaceutical Sciences, 16th edition, Oslo, A., Ed., (1980).

The Syndecan-4 antibodies disclosed herein may also be formulated as immunoliposomes. A "liposome" is a small vesicle composed of various types of lipids, phospholipids and/or surfactant which is useful for delivery of a drug to a mammal. The components of the liposome are commonly arranged in a bilayer formation, similar to the lipid arrangement of biological membranes. Liposomes containing the antibody are prepared by methods known in the art, such as described in Epstein et al., Proc. Natl. Acad. Sci. USA, 82: 3688 (1985); Hwang et al. , Proc. Natl Acad. Sci. USA, 77: 4030 (1980); U. S. Pat. Nos. 4, 485, 045 and 4,544, 545; and W097/38731 published October 23, 1997. Liposomes with enhanced circulation time are disclosed in U. S. Patent No. 5,013, 556. Particularly useful liposomes can be generated by the reverse phase evaporation method with a lipid composition comprising phosphatidylcholine, cholesterol and PEG-derivatized phosphatidylethanolamine (PEG-PE). Liposomes are extruded through filters of defined pore size to yield liposomes with the desired diameter. Fab' fragments of the antibody of the present invention can be conjugated to the liposomes as described in Martin et al. J. Biol. Chem. 257: 286-288 (1982) via a disulfide interchange reaction. A chemotherapeutic agent is optionally contained within the liposome. See Gabizon et al. J. National Cancer Inst. 81 (19) 1484 (1989).

When using recombinant techniques, the antibody can be produced intracellularly, in the periplasmic space, or directly secreted into the medium. If the antibody is produced intracellularly, as a first step, the particulate debris, either host cells or lysed fragments, are removed, for example, by centrifugation or ultrafiltration. Carter et al., Bio/Technology 10: 163-167 (1992) describe a procedure for isolating antibodies which are secreted to the periplasmic space of E coli.

The antibody composition prepared from the cells can be purified using, for example, hydroxylapatite chromatography, gel electrophoresis, dialysis, and affinity chromatography, with affinity chromatography being the preferred purification technique. The suitability of protein A as an affinity ligand depends on the species and isotype of any immunoglobulin Fc domain that is present in the antibody. Protein A can be used to purify antibodies (Lindmark et al., J. Immunol. Meth. 62: 1-13 (1983)). Protein G is recommended for all mouse isotypes and for human gamma3 (Guss et al., EMBO J. 5: 15671575 (1986)).

As mentioned herein, the recognition molecule, preferably an antibody of the present invention binds an epitope contained in an (Syndecan-4) amino acid sequence corresponding to the (mouse Syndecan-4) amino acid sequence between amino acids 93 and 121 of the amino acid sequence shown in SEQ ID NO:1 (Figure 1). The term "93 and 121" preferably encompasses that up to 5, 4, 3, 2 and/or 1 or 0 additional amino acids at the N-and/or C-terminal end of the amino acid sequence shown in SEQ ID NO:1 (Figure 1) are included. For example, the amino acid sequence may then span amino acids 88 and 121, 88 and 126 or 93 and 126.

Though the present inventors could have chosen several regions of the Syndecan-4 protein in order to generate an antibody, they chose an amino acid sequence corresponding to the (mouse Syndecan-4) amino acid sequence between amino acids 93 and 121 of the amino acid sequence shown in SEQ ID NO:1. Syndecan-4 contains three domains: an extracellular domain having an attachment site for heparane sulfate, a transmembrane domain and a cytoplasmic domain having a variable and conserved region. Antibodies could be raised against each of the three domains.

For example, an attractive region is the variable region of the cytoplasmic domain, since this region shows, so to say, the greatest diversity between Syndecan-1, 2, 3 and 4. Accordingly, it would be feasible to generate an antibody which is specific for Syndecan-4. Moroever, such an antibody would likely inhibit signal transduction effected by Syndecan-4 which would be desirable in order to prevent activation of, in particular, ADAMTS-5, since the variable region is the binding site for protein kinase C and phospho-inositol bisphosphate (PIP).

Another attractive region for the generation of an anti-Syndecan-4 antibody is the extracellular domain. The N-terminal portion of the extracellular domain is a highly attractive target for antibodies, since it is most distant from the cellular membrane and, thus, well accesible for the binding of an antibody. Apart from that, the N-terminal portion precedes the attachment site for heparane sulfate chains which could highly likely interfere with the binding of an antibody. In the prior art, antibodies against this region are known. However, none of them is known to have a blocking effect on Syndecan-4 activity.

A further attractive region for the generation of anti-Syndecan-4 antibodies comprises the attachment sites for heparan sulfate chains. Though these binding sites are conserved between Syndecan-1, 2, 3 and 4 proteins, and would thus not be suitable to generate a specific Syndecan-4 antibody, that region also comprises stretches between these attachment sites which are different between Syndecan proteins and are thus suitable targets.

Further C-terminal of the heparane sulfate attachment sites is a binding site for a ligand which is so far unknown. That binding site is not present in Syndecan proteins different from Syndecan-4 and would therefore qualify as ideal target for the generation of an antibody specific for Syndecan-4. Further C-terminal Syndecan proteins have cleavage sites for thrombin and plasmin (Schmidt, J. Biol. Chem. 2005;280:34441-34446) which are likewise antibody targets, since these cleavage sites are still far enough from the cellular membrane such that no steric hindrance should be given for an antibody.

It is apparent from the foregoing explanations that a Syndecan-4 protein offers many targets for the generation of a specific antibody. Thus, it is prima facie not evident which of these regions would be most suitable for the generation of a specific Syndecan-4 antibody which could then block Syndecan-4 activity on, in particular, ERK 1/2 and thus on ADAMTS-5. The present inventors chose a region located between the ligand binding site and the second protease cleavage site. The choice of this region was, in part, driven by the present inventors' assumption that it could be involved in the dimerization of Syndecan-4 and in the signal transduction effected by Syndecan-4 (see Figure 3 for the location of the aforedescribed regions).

The term "position" when used in accordance with the present invention means the position of an amino acid within an amino acid sequence depicted herein. The term "corresponding" as used herein also includes that a position is not only determined by the number of the preceding nucleotides/amino acids.

The position of a given amino acid in accordance with the present invention which may be substituted may very due to deletion or addition of amino acids elsewhere in the Syndecan-4 polypeptide.

Thus, under a "corresponding position" in accordance with the present invention it is to be understood that amino acids may differ in the indicated number but may still have similar neighbouring amino acids. Said amino acids which may be exchanged, deleted or added are also comprised by the term "corresponding position".

In order to determine whether an amino acid residue in a given Syndecan-4 amino acid sequence corresponds to a certain position in the amino acid sequence of SEQ ID NO: 1, the skilled person can use means and methods well-known in the art, e.g., alignments, either manually or by using computer programs such as BLAST2.0, which stands for Basic Local Alignment Search Tool or ClustalW or any other suitable program which is suitable to generate sequence alignments.
SEQ ID NO: 1 is the amino acid sequence encoding mouse Syndecan-4. SEQ ID NO: 2 is the amino acid sequence encoding human Syndecan-4.

As used herein, the term "Syndecan-4" (or Sdc-4) refers to the Syndecan-4 from any origin, preferably mammalian, more preferably human origin. Said term when used herein encompasses Syndecan-4 proteins as well as polynucleotides encoding Syndecan-4 proteins. The term "polynucleotide(s)" refers to nucleic acids such as DNA molecules and RNA molecules and analogs thereof (e.g., DNA or RNA generated using nucleotide analogs or using nucleic acid chemistry). As desired, the polynucleotides may be made synthetically, e.g., using art-recognized nucleic acid chemistry or enzymatically using, e.g., a polymerase, and, if desired, be modified. Typical modifications include methylation, biotinylation, and other art-known modifications. In addition, the nucleic acid molecule can be single-stranded or double-stranded and, where desired, linked to a detectable moiety.

Preferred Syndecan-4 proteins (i.e, their amino acid sequence) are shown in SEQ ID NO:1 (mouse), SEQ ID NO:2 (human), SEQ ID NO:3 (rat), SEQ ID NO:4 (pig), SEQ ID NO:5 (chicken), SEQ ID NO:6 (cattle), SEQ ID NO:7 (chimpanzee), SEQ ID NO:8 (olive baboon), SEQ ID NO:9 (Sumatran orangutan) or SEQ ID NO:10 (zebra fish). Any of these amino acid sequences could serve as reference sequence instead of SEQ ID NO:1 when determining whether an amino acid residue in a given Syndecan-4 amino acid sequence corresponds to a certain position in the amino acid sequence of SEQ ID NO:2, 3, 4, 5, 6, 7, 8, 9 or 10 as long as this sequence finds corresponding positions (in particular amino acid residues) in the amino acid sequence shown in SEQ ID NO:11 (NAQPGIRVPSEPKELEENEVIPKRAPSDV) (mouse sequence from 93-121).

Also encompassed by the term "Syndecan-4" are fragments thereof that comprise at least 50, more preferably at least 60, even more preferably at least 70 or at least 80 amino acid residues of the Syndecan-4 proteins shown in any one of SEQ ID Nos:1-10, wherein said fragments are, for example, capable of activating ADAMTS-5. Further encompassed by the term "Syndecan-4" are Syndecan-4 proteins or fragments thereof which share preferably 50 %, 60%, 70%, 80% or 90%, more preferably 95% or 97%, even more preferably 98% and most preferably 99% identity on amino acid level to any one of the amino acid sequences shown in SEQ ID Nos:1-10 and which are capable of activating ADAMTS-5. ADAMTS-5 activity can be measured, for example, as done by Gendron et al., J. Biol. Chem. 282 (2007), 18294-18306 or in Example 7 of WO 2004/011637.

Means and methods for determining the identity of sequences, for example, amino acid sequences is described elsewhere herein.

A "variant" of a Syndecan-4 protein is also encompassed by the term "Syndecan-4". In a variant one or more amino acid residues are substituted, preferably conservatively substituted compared to said polypeptide and wherein said variant is preferably able to phosphorylate ERK 1/2. Such variants include deletions, insertions, inversions, repeats, and substitutions selected according to general rules known in the art so as not having an effect on the activity of Syndecan-4, preferably on the phosphorylation of ERK 1/2. For example, guidance concerning how to make phenotypically silent amino acid substitutions is provided in Bowie, Science 247: (1990) 1306-1310.

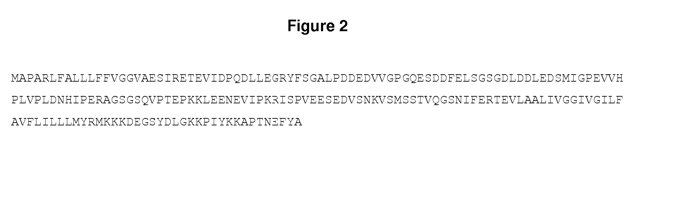

The present invention also envisages a polypeptide consisting of an amino acid sequence corresponding to
(i) the amino acid sequence between amino acids 93 and 121 of the amino acid sequence shown in SEQ ID NO:1 (Figure 1) (MAUS); or
(ii) the amino acid sequence between amino acids 92 and 122 of the amino acid sequence shown in SEQ ID NO:2 (Figure 2) (Mensch).

In a yet further aspect, the present invention relates to an epitope contained in the said polypeptide. Said epitope comprises at least 5, preferably at least 6 amino acids of said polypeptide and could thus be regarded as an immunogenic fragment of said polypeptide. In that case the epitope is a linear epitope. In case the epitope is a discontinuous epitope, it contains at least 2, 3 or 4 amino acids of a first portion of said polypeptide and 2, 3 or 4 amino acids of a second portion of said polypeptide.

Said polypeptide or epitope contained therein is preferably applied in the methods and uses of the present invention for the generation of an antibody against a Syndecan-4 protein as described herein elsewhere.

When used hererin, a pathological medical condition associated with turnover of the extracellular matrix and/or cartilage remodelling in which Syndecan-4, in particular Syndecan-4 of chondrocytes, is involved, encompasses abnormal, i.e., pathological processes in the turnover of the extracellular matrix and/or cartilage remodelling in which Syndecan-4 is involved. Said pathological medical condition may preferably be, inter alia, characterized by cartilage destruction/damage.

For example, under normal conditions, i.e., in a healthy subject, it can be reasonably assumed that Syndecan-4 expression is hardly (i.e., almost not) detectable, while its expression is up-regulated under pathological conditions, in particular inflammatory conditions. Then, Syndecan-4 is involved in receiving and transmitting extracellular signals (such as IL-1) via phosphorylation of ERK 1/2, thereby causing, for example, expression of genes involved in cartilage rebuilding, for example, MMP-3 and/or other matrixmeltalloproteases. MMP-3 can in turn activate ADAMTS-5 which is also involved in cartilage remodelling. Moreover, as is demonstrated herein and illustrated in the Examples, Syndecan-4 directly interacts with ADAMTS-5 and/or IL-1. However, both the direct interaction of Syndecan-4 with ADAMTS-5 and/or IL-1 and signal transduction via phosphorylation of ERK 1/2 may become abnormal and thus pathologic, thereby causing, for example, excessive cartilage turnover and/or breakdown.

The pathological condition associated with turnover of the extracellular matrix and/or cartilage remodelling in which Syndecan-4, in particular Syndecan-4 of chondrocytes, is involved that is to be treated and/or prevented by the recognition molecule of the present invention is preferably Syndecan-4 mediated arthritis, including in particular osteoarthritis and rheumatoid arthritis. Cartilage and remodelling processes thereof are described elsewhere herein.

"Syndecan-4 mediated" means that, in particular Syndecan-4 on the surface of specialized cells, preferably chondrocytes and/or fibroblasts, is directly (via binding to IL-1 and/or ADAMTS-5) or indirectly (via signal transduction through ERK 1/2) involved in the etiology of arthritis.

A major component of the cartilage extracellular matrix is aggrecan, a proteoglycan that imparts compressive resistance to the tissue. Aggrecan is cleaved at a specific 'aggrecanase' site in human osteoarthritic cartilage; this cleavage can be performed by several members of ADAMTS family of metalloproteases, with ADAMTS-5 being the primary 'aggrecanase' responsible for aggrecan degradation.

Accordingly, preventing abnormal ADAMTS-5 proteolytic activity is desirable and can be achieved by inhibiting Syndecan-4 with a recognition molecule as described herein.

Multiple mechanisms are involved in the degradation of articular cartilage in arthritides such as rheumatoid arthritis (RA) and osteoarthritis (OA). OA, a non-inflammatory arthritis, is the most common form of joint disease, and is second only to cardiovascular disease as a cause of early retirement and disability. Some individuals exhibit OA in a single or limited number of joints, such as may result from traumatic injury due to accident or surgery. Many other individuals suffer from OA in multiple joints due to wear and tear associated with aging or with athletic or occupational activity over an extended period of time.

RA is the most common form of inflammatory arthritis, affecting 3% of women and 1% of men. The majority of RA patients have symptoms in multiple joints, especially the small joints of the hand, the elbows, the wrists and the shoulders.

The destruction of hyaline articular cartilage is the hallmark of OA and disabling RA. Although various therapeutic approaches may provide relief of symptoms, no therapeutic regimen has been proven to retard progression of articular cartilage degradation. The progressive deterioration and loss of articular cartilage leads to an irreversible impairment of joint motion. These changes in cartilage are the final pathogenic events that are common to osteoarthritis (OA) and rheumatoid arthritis (RA).

Cartilage destructive processes may also be associated with or initiated by surgical procedures of the joint. Arthroscopy is a surgical procedure in which a camera, attached to a remote light source and video monitor, is inserted into an anatomic joint (e. g., knee, shoulder, etc.) through a small portal incision in the overlying skin and joint capsule. Through similar portal incisions, surgical instruments may be placed in the joint, their use guided by arthroscopic visualization.

As arthroscopists' skills have' improved, an increasing number of operative procedures, once performed by"open"surgical technique, now can be accomplished arthroscopically. Such procedures include, for example, partial meniscectomies and ligament reconstructions in the knee, shoulder acromioplasties and rotator cuff debridements and elbow synovectomies. As a result of widening surgical indications and the development of small diameter arthroscopes, wrist and ankle arthroscopies also have become routine. Throughout each arthroscopy, physiologic irrigation fluid (e. g. , normal saline or lactated Ringer's) is flushed continuously through the joint, distending the joint capsule and removing operative debris, thereby providing clearer intra-articular visualization.-U. S. Patent 4,504, 493 to Marshall discloses an isomolar solution of 20541 PCT - 3- glycerol in water for a non-conductive and optically clear irrigation solution for arthroscopy. Conventional physiologic irrigation fluids do not provide analgesic, anti-inflammatory or anti-cartilage degradation effects.

Rheumatoid arthritis (RA) is a chronic, systemic inflammatory disorder that may affect many tissues and organs, but principally attacks synovial joints. The process produces an inflammatory response of the synovium (synovitis) secondary to hyperplasia of synovial cells, excess synovial fluid, and the development of pannus in the synovium. The pathology of the disease process often leads to the destruction of articular cartilage and ankylosis of the joints. Rheumatoid arthritis can also produce diffuse inflammation in the lungs, pericardium, pleura, and sclera, and also nodular lesions, most common in subcutaneous tissue under the skin. Although the cause of rheumatoid arthritis is unknown, autoimmunity plays a pivotal role in both its chronicity and progression.

Onset is most frequent between the ages of 40 and 50, but people of any age can be affected. It can be a disabling and painful condition, which can lead to substantial loss of functioning and mobility. It is diagnosed chiefly on symptoms and signs, but also with blood tests (especially a test called rheumatoid factor) and X-rays. Diagnosis and long-term management are typically performed by a rheumatologist, an expert in the diseases of joints and connective tissues.

Unlike rheumatoid arthritis, osteoarthritis can affect both the larger and the smaller joints of the body, including the hands, feet, back, hip or knee. The disease is essentially one acquired from daily wear and tear of the joint. Osteoarthritis begins in the cartilage and eventually leads to the two opposing bones eroding into each other. Initially, the condition starts with minor pain while walking but soon the pain can be continuous and even occur at night. The pain can be debilitating and prevent one from doing any type of activity. Osteoarthritis typically affects the weight bearing joints like the back, spine and pelvis. Unlike rheumatoid arthritis, osteoarthritis is a disease of the elderly. Osteoarthritis (OA) also known as degenerative arthritis or degenerative joint disease, is a group of mechanical abnormalities involving degradation of joints, including articular cartilage and subchondral bone. Symptoms may include joint pain, tenderness, stiffness, locking, and sometimes an effusion. A variety of causes - hereditary, developmental, metabolic, and mechanical - may initiate processes leading to loss of cartilage. When bone surfaces become less well protected by cartilage, bone may be exposed and damaged. As a result of decreased movement secondary to pain, regional muscles may atrophy, and ligaments may become more lax.

In a further aspect, the present invention relates to a nucleic acid encoding the recognition molecule described herein.

Said nucleic acid molecule is preferably comprised in a vector which is preferably comprised in a host cell. Said host cell is capable of expressing the recognition molecule. For that purpose the nucleic acid molecule is operatively linked with control sequences.

As used herein, the term "host cell" is intended to refer to a cell into which a nucleic acid encoding the recognition molecule of the invention is introduced by way of transformation, transfection and the like. It should be understood that such terms refer not only to the particular subject cell but to the progeny or potential progeny of such a cell. Because certain modifications may occur in succeeding generations due to either mutation or environmental influences, such progeny may not, in fact, be identical to the parent cell, but are still included within the scope of the term as used herein.

As used herein, the term "expression" includes any step involved in the production of a recognition molecule including, but not limited to, transcription, post- transcriptional modification, translation, post-translational modification, and secretion.

The term "control sequences" refers to DNA sequences necessary for the expression of an operably linked coding sequence in a particular host organism. The control sequences that are suitable for prokaryotes, for example, include a promoter, optionally an operator sequence, and a ribosome binding site. Eukaryotic cells are known to utilize promoters, polyadenylation signals, and enhancers.

Nucleic acid is "operably linked" when it is placed into a functional relationship with another nucleic acid sequence. For example, DNA for a presequence or secretory leader is operably linked to DNA for a polypeptide if it is expressed as a preprotein that participates in the secretion of the polypeptide; a promoter or enhancer is operably linked to a coding sequence if it affects the transcription of the sequence; or a ribosome binding site is operably linked to a coding sequence if it is positioned so as to facilitate translation. Generally, "operably linked" means that the DNA sequences being linked are contiguous, and, in the case of a secretory leader, contiguous and in reading phase. However, enhancers do not have to be contiguous. Linking is accomplished by ligation at convenient restriction sites. If such sites do not exist, the synthetic oligonucleotide adaptors or linkers are used in accordance with conventional practice.

Suitable host cells include prokaryotic and eukaryotic host cells including yeasts, fungi, insect cells and mammalian cells.

Recognition molecules can be produced in bacteria, in particular when glycosylation and Fc effector function are not needed.

After expression, the recognition molecule, preferably an antibody is isolated from the E. coli cell paste in a soluble fraction and can be purified through, e.g., a protein A or G column depending on the isotype. Final purification can be carried out similar to the process for purifying antibody expressed e. g" in CHO cells.

In addition to prokaryotes, eukaryotic microbes such as filamentous fungi or yeast are suitable cloning or expression hosts for the recognition molecule of the present invention.

Saccharomyces cerevisiae, or common baker's yeast, is the most commonly used among lower eukaryotic host microorganisms. However, a number of other genera, species, and strains are commonly available and useful herein, such as Schizosaccharomyces pombe; Kluyveromyces hosts such as, e. g. , K. lactis, K. fragilis (ATCC 12424), K. bulgaricus (ATCC 16045), K. wickeramii (ATCC 24178), K. waltii (ATCC 56500), K. drosophilarum (ATCC 36906), K. thermotolerans, and K. marxianus; yarrowia (EP 402 226); Pichia pastoris (EP 183 070); Candida; Trichoderma reesia (EP 244 234); Neurospora crassa; Schwanniomyces such as Schwanniomyces occidentalis; and filamentous fungi such as, e.g., Neurospora, Penicillium, Tolypocladium, and Aspergillus hosts such as A. nidulans and A. niger.

Suitable host cells for the expression of glycosylated recognition molecules, preferably antibodies are derived from multicellular organisms. Examples of invertebrate cells include plant and insect cells. Numerous baculoviral strains and variants and corresponding permissive insect host cells from hosts such as Spodoptera frugiperda (caterpillar), Aedes aegypti (mosquito), Aedes albopictus (mosquito), Drosophila melanogaster (fruitfly), and Bombyx mori have been identified. A variety of viral strains for transfection are publicly available, e. g. , the L-1 variant of Autographa californica NPV and the Bm-5 strain of Bombyx mori NPV, and such viruses may be used as the virus herein according to the present invention, particularly for transfection of Spodoptera frugiperda cells.

Plant cell cultures of cotton, corn, potato, soybean, petunia, tomato, Arabidopsis and tobacco can also be utilized as hosts. Cloning and expression vectors useful in the production of proteins in plant cell culture are known to those of skill in the art. See e.g. Hiatt et al., Nature (1989) 342: 76-78, Owen et al. (1992) Bio/Technology 10: 790-794, Artsaenko et al. (1995) The Plant J 8: 745-750, and Fecker et al. (1996) Plant Mol Biol 32: 979-986.

However, interest has been greatest in vertebrate cells, and propagation of vertebrate cells in culture (tissue culture) has become a routine procedure. Examples of useful mammalian host cell lines are monkey kidney CV1 line transformed by SV40 (COS-7, ATCC CRL 1651) ; human embryonic kidney line (293 or 293 cells subcloned for growth in suspension culture, Graham et al. , J. Gen Virol. 36 : 59 (1977)); baby hamster kidney cells (BHK, ATCC CCL 10); Chinese hamster ovary cells/-DHFR (CHO, Urlaub et al. , Proc. Natl. Acad. Sci. USA 77: 4216 (1980)); mouse sertoli cells (TM4, Mather, Biol. Reprod. 23: 243-251 (1980)); monkey kidney cells (CVI ATCC CCL 70); African green monkey kidney cells (VERO-76, ATCC CRL1587) ; human cervical carcinoma cells (HELA, ATCC CCL 2); canine kidney cells (MDCK, ATCC CCL 34); buffalo rat liver cells (BRL 3A, ATCC CRL 1442); human lung cells (W138, ATCC CCL 75); human liver cells (Hep G2,1413 8065); mouse mammary tumor (MMT 060562, ATCC CCL5 1); TRI cells (Mather et al., Annals N. Y Acad. Sci. 383 : 44-68 (1982)) ; MRC 5 cells; FS4 cells; and a human hepatoma line (Hep G2).

When using recombinant techniques, the recognition molecule, preferably an antibody can be produced intracellularly, in the periplasmic space, or directly secreted into the medium. If the antibody is produced intracellularly, as a first step, the particulate debris, either host cells or lysed fragments, are removed, for example, by centrifugation or ultrafiltration. Carter et al., Bio/Technology 10: 163-167 (1992) describe a procedure for isolating antibodies which are secreted to the periplasmic space of E coli. Briefly, cell paste is thawed in the presence of sodium acetate (pH 3.5), EDTA, and phenylmethylsulfonylfluoride (PMSF) over about 30 min. Cell debris can be removed by centrifugation. Where the antibody is secreted into the medium, supernatants from such expression systems are generally first concentrated using a commercially available protein concentration filter, for example, an Amicon or Millipore Pellicon ultrafiltration unit. A protease inhibitor such as PMSF may be included in any of the foregoing steps to inhibit proteolysis and antibiotics may be included to prevent the growth of adventitious contaminants.

The recognition molecule, preferably an antibody prepared from the host cells can be purified using, for example, hydroxylapatite chromatography, gel electrophoresis, dialysis, and affinity chromatography, with affinity chromatography being the preferred purification technique.

The suitability of protein A as an affinity ligand depends on the species and isotype of any immunoglobulin Fc domain that is present in the antibody. Protein A can be used to purify antibodies that are based on human y1, y2, or y4 heavy chains (Lindmark et al., J. Immunol. Meth. 62: 1-13 (1983)). Protein G is recommended for all mouse isotypes and for human y3 (Guss et al., EMBO J. 5: 15671575 (1986)). The matrix to which the affinity ligand is attached is most often agarose, but other matrices are available. Mechanically stable matrices such as controlled pore glass or poly (styrenedivinyl) benzene allow for faster flow rates and shorter processing times than can be achieved with agarose. Where the antibody comprises a CH3 domain, the Bakerbond ABXMresin (J. T. Baker, Phillipsburg, NJ) is useful for purification. Other techniques for protein purification such as fractionation on an ion-exchange column, ethanol precipitation, Reverse Phase HPLC, chromatography on silica, chromatography on heparin SEPHAROSETM chromatography on an anion or cation exchange resin (such as a polyaspartic acid column), chromato-focusing, SDS-PAGE, and ammonium sulfate precipitation are also available depending on the antibody to be recovered.

In another aspect, the present invention provides a recognition molecule of the present invention for use as diagnostic composition. Accordingly, the recognition molecule can be used in diagnostic assays for their antigen, e. g., detecting its expression in specific cells, tissues, or serum.

Various diagnostic assay techniques known in the art may be used, such as competitive binding assays, direct or indirect sandwich assays and immunoprecipitation assays conducted in either heterogeneous or homogeneous phases (Zola, Monoclonal Antibodies: A Manual of Techniques, CRC Press, Inc. (1987) pp. 147-158). The recognition molecule used in the diagnostic assays can be labeled with a detectable moiety. For example, recognition molecules may be modified with detectable markers, including ligand groups (e.g., biotin), fluorophores and chromophores, radioisotopes, electron-dense reagents, or enzymes. Enzymes are detected by their activity. For example, horseradish peroxidase is detected by its ability to convert tetramethylbenzidine (TMB) to a blue pigment, quantifiable with a spectrophotometer. Other suitable binding partners include biotin and avidin, IgG and protein A, and other receptor-ligand pairs known in the art.

The detectable moiety should be capable of producing, either directly or indirectly, a detectable signal. For example, the detectable moiety may be a radioisotope, such as ³H, ¹⁴C, ³²P ³⁵S or ¹²⁵I a fluorescent or chemiluminescent compound, such as fluorescein isothiocyanate, rhodamine, or luciferin, or an enzyme, such as alkaline phosphatase, beta-galactosidase or horseradish peroxidase. Any method known in the art for conjugating the antibody to the detectable moiety may be employed.

The recognition molecule of the present invention when administered to a subject is preferably in the form of a composition. The composition is preferably suitable for pharmaceutical use and administration to subjects.

Accordingly, the recognition molecule of the present invention is envisaged for use in therapy. Accordingly, the present invention envisages a pharmaceutical composition (or medicament) comprising the recognition molecule described herein.

In yet another embodiment, the invention provides a method of treating a subject comprising administering a therapeutically effective amount of the recognition molecule of the present invention, wherein the subject has a pathological medical condition associated with turnover of the extracellular matrix and/or cartilage remodelling in which Syndecan-4 is involved.

The term "subject" is intended to include living organisms. Examples of subjects include mammals, e.g., humans, dogs, cows, horses, pigs, sheep, goats, cats, mice, rabbits, rats, and transgenic non-human animals. In preferred embodiments of the invention, the subject is a human.

The term "effective dose" or "effective dosage" is defined as an amount sufficient to achieve or at least partially achieve the desired effect. The term "therapeutically effective dose" is defined as an amount sufficient to cure or at least partially arrest the disease and its complications in a patient already suffering from the disease. Amounts effective for this use will depend upon the severity of the infection and the general state of the subject's own immune system. The term "patient" includes human and other mammalian subjects that receive either prophylactic or therapeutic treatment.

The appropriate dosage, or therapeutically effective amount, of the recognition molecule will depend on the condition to be treated, the severity of the condition, prior therapy, and the patient's clinical history and response to the therapeutic agent. The proper dose can be adjusted according to the judgment of the attending physician such that it can be administered to the patient one time or over a series of administrations. The pharmaceutical composition can be administered as a sole therapeutic or in combination with additional therapies as needed.

The pharmaceutical compositions of this invention are particularly useful for parenteral administration, i.e., subcutaneously, intramuscularly, intravenously, intra-articular and/or intra-synovial. Parenteral administration can be by bolus injection or continuous infusion.

In a preferred embodiment, the injection is a local or non-systemic injection, preferably into the synovia, synovia space, synovial fluid, or synovial joint, subchondral area, osteochondral defect, intra-articular space preferably of the knee, shoulder, hip, thumb, temporomandibular joint or facet joint, annulus fibrosus, nucleus pulposus, nucleus pulposus space, intradiscally or transdically. More preferably, the injection is an intra-articular injection preferably into the knee, shoulder, hip, thumb, temporomandibular joint or facet joint. Further preferably, the intra-articular injection is an intra-articular injection into the synovial fluid of the facet joint or the temporomandibular joint. A further preferred injection is an injection into the subsynovial room or area or an injection into the chondral or osteochondral defect. Also encompassed is an injection into the chondral or osteochondral defect before or after closure of the defect with a membrane. The membrane can be, but is not limited to, a periosteum or collagen. In another preferred embodiment the membrane is a membrane comprising of collagen type I, collagen type III, porcine or rat collagen type I or type III, hyaluronic acid or derivative thereof. An advantage of the closure of the defect before injection of the formulation is to reduce dilution of the formulation or to increase the local concentration of the active ingredient of the formulation. The membrane further acts as bioadhesive agent for the attachment of cells

If the pharmaceutical composition has been lyophilized, the lyophilized material is first reconstituted in an appropriate liquid prior to administration. The lyophilized material may be reconstituted in, e.g., bacteriostatic water for injection (BWFI), physiological saline, phosphate buffered saline (PBS), or the same formulation the protein had been in prior to lyophilization.

Pharmaceutical compositions for injection may be presented in unit dosage form, e.g., in ampoules or in multi-dose containers, with an added preservative. In addition, a number of recent drug delivery approaches have been developed and the pharmaceutical compositions of the present invention are suitable for administration using these new methods, e. g., Inject-ease, Genject, injector pens such as Genen, and needleless devices such as MediJector and BioJector. The present pharmaceutical composition can also be adapted for yet to be discovered administration methods. See also Langer, 1990, Science, 249: 1527-1533.

The pharmaceutical composition can also be formulated as a depot preparation. Such long acting formulations may be administered by implantation (for example subcutaneously, into the ligament or tendon, subsynovially or intramuscularly), by subsynovial injection or by intramuscular injection. Thus, for example, the formulations may be modified with suitable polymeric or hydrophobic materials (for example as a emulsion in an acceptable oil) or ion exchange resins, or as sparingly soluble derivatives, for example, as a sparingly soluble salt.

The pharmaceutical compositions may also be in a variety of conventional depot forms employed for administration to provide reactive compositions. These include, for example, solid, semi-solid and liquid dosage forms, such as liquid solutions or suspensions, slurries, gels, creams, balms, emulsions, lotions, powders, sprays, foams, pastes, ointments, salves, balms and drops.

The pharmaceutical compositions may, if desired, be presented in a vial, pack or dispenser device which may contain one or more unit dosage forms containing the active ingredient. In one embodiment the dispenser device can comprise a syringe having a single dose of the liquid formulation ready for injection. The syringe can be accompanied by instructions for administration.

The pharmaceutical composition may further comprise additional pharmaceutically acceptable components. Other pharmaceutically acceptable carriers, excipients, or stabilizers, such as those described in Remington's Pharmaceutical Sciences 16th edition, Osol, A. Ed. (1980) may also be included in a protein formulation described herein, provided that they do not adversely affect the desired characteristics of the formulation. As used herein, "pharmaceutically acceptable carrier" means any and all solvents, dispersion media, coatings, antibacterial and antifungal agents, isotonic and absorption delaying agents, compatible with pharmaceutical administration. The use of such media and agents for pharmaceutically active substances is well known in the art. Acceptable carriers, excipients, or stabilizers are nontoxic to recipients at the dosages and concentrations employed and include: additional buffering agents; preservatives; cosolvents; antioxidants, including ascorbic acid and methionine; chelating agents such as EDTA; metal complexes (e.g., Zn-protein complexes); biodegradable polymers, such as polyesters; salt-forming counterions, such as sodium, polyhydric sugar alcohols; amino acids, such as alanine, glycine, asparagine, 2-phenylalanine, , and threonine; sugars or sugar alcohols, such as lactitol, stachyose, mannose, sorbose, xylose, ribose, ribitol, myoinisitose, myoinisitol, galactose, galactitol, glycerol, cyclitols (e.g., inositol), polyethylene glycol; sulfur containing reducing agents, such as glutathione, thioctic acid, sodium thioglycolate, thioglycerol, [alpha]-monothioglycerol, and sodium thio sulfate; low molecular weight proteins, such as human serum albumin, bovine serum albumin, gelatin, or other immunoglobulins; and hydrophilic polymers, such as polyvinylpyrrolidone.

The formulations described herein are useful as pharmaceutical compositions in the treatment and/or prevention of the pathological medical condition as described herein in a patient in need thereof. The term "treatment" refers to both therapeutic treatment and prophylactic or preventative measures. Treatment includes the application or administration of the formulation to the body, an isolated tissue, or cell from a patient who has a disease/disorder, a symptom of a disease/disorder, or a predisposition toward a disease/disorder, with the purpose to cure, heal, alleviate, relieve, alter, remedy, ameliorate, improve, or affect the disease, the symptom of the disease, or the predisposition toward the disease.

Those "in need of treatment" include those already with the disorder, as well as those in which the disorder is to be prevented. The term "disorder" is any condition that would benefit from treatment with the protein formulation described herein. This includes chronic and acute disorders or diseases including those pathological conditions that predispose the mammal to the disorder in question. Non-limiting examples of disorders to be treated herein include degenerative diseases, bone and/or cartilage and/or articular cartilage defect, an immunological disease preferably chronic inflammation of a joint, bone or cartilage tissue such as arthritis (including but not limited to osteoarthritis, rheumatoid arthritis) and a spinal disorder such as degenerative disc disease. In a preferred embodiment the spinal disorder is idiopathic low back pain, disc herniation, internal disc disruption or fissured discs, radiculopathy, spinal stenosis, herniated nucleus pulposus-induced sciatica, sciatica, idiopathic scoliosis or myelopathy.

It is also contemplated that the recognition molecule of the present invention is applied together with a medicament suitable for the treatment of arthritis, in particular osteoarthritis or rheumatoid arthritis. "Together with" means that the recognition molecule is administered prior to the second medicament, at the same time or after the second medicament. A medicament suitable for the treatment of rheumatoid arthritis is, for example, an antibody against TNF, IL-1 and/or GM-CSF. Similarly, an antibody against the IL-1 receptor or GM-CSF receptor may be suitable. Another medicament that us suitable is a soluble TNF receptor, i.e., Etanercept.

In a further embodiment of the invention, there are provided articles of manufacture and kits containing recognition molecules which can be used, for instance, for the therapeutic or non-therapeutic applications described above. The article of manufacture comprises a container with a label. Suitable containers include, for example, bottles, vials, and test tubes. The containers may be formed from a variety of materials such as glass or plastic. The container holds a composition which includes an active agent that is effective for therapeutic or non-therapeutic applications, such as described above. The active agent in the composition is the recognition molecule. The label on the container indicates that the composition is used for a specific therapy or non-therapeutic application, and may also indicate directions for either in vivo or in vitro use, such as those described above. The kit of the invention will typically comprise the container described above and one or more other containers comprising materials desirable from a commercial and user standpoint, including buffers, diluents, filters, needles, syringes, and package inserts with instructions for use.

The invention is also characterized by the following items:
1. A recognition molecule against Syndecan-4, wherein said recognition molecule binds an epitope contained in an amino acid sequence corresponding to the amino acid sequence between amino acids 93 and 121 of the amino acid sequence shown in SEQ ID NO:1 (Figure 1) for use in the treatment and/or prevention of a pathological medical condition associated with turnover of the extracellular matrix and/or cartilage remodelling in which Syndecan-4 is involved.
2. The recognition molecule of item 1, wherein said amino acid sequence shown in SEQ ID NO:1 depicts the mouse Syndecan-4 protein.
3. The recognition molecule of item 1, wherein an amino acid sequence corresponding to the amino acid sequence between amino acids 93 and 121 of the amino acid sequence shown in SEQ ID NO:1 is the amino acid sequence between amino acids 92 and 122 shown in SEQ ID NO:2.
4. The recognition molecule of item 3, wherein said amino acid sequence shown in SEQ ID NO:2 depicts the human Syndecan-4 protein.
5. The recognition molecule of any one of the preceding items, wherein said antibody inhibits phosphorylation of ERK 1/2 by Syndecan-4.
6. The recognition molecule of any one of the preceding items, wherein said antibody is a polyclonal or monoclonal antibody.
7. The recognition molecule of any one of the preceding item, wherein said antibody is a chimeric, humanized, or human antibody.
8. The recognition molecule of any one of the preceding items, wherein said disorder is characterized by cartilage destruction/damage.
9. The recognition molecule of any one of the preceding items, wherein said disorder is osteoarthritis.
10. The recognition molecule of any one of the preceding items, wherein said disorder is rheumatoid arthritis.
11. The recognition molecule of any one of the preceding items for use as diagnostic composition.
12. A nucleic acid encoding the recognition molecule of any one of item 1-7.
13. A vector comprising the nucleic acid of item 12.
14. A host cell comprising the vector of item 13.
15. A method for the generation of an antibody against Syndecan-4 which inhibits phosphorylation of ERK 1/2 by Syndecan-4, comprising
   (a) immunizing a non-human mammal with a polypeptide comprising amino acids 93 to 121 of the amino acid sequence shown in SEQ ID NO:1 or with a polypeptide comprising amino acids 92 to 122 of the amino acid sequence shown in SEQ ID NO:2;
   (b) isolating an antibody obtained in step(a); and
   (c) determining whether the antibody obtained in step (b) inhibits phosphorylation of ERK 1/2 by Syndecan-4.
16. The method of item 15, wherein the Sydecan-4 is the human Syndecan-4 protein.
17. The method of item 15 or 16, wherein the antibody is a monoclonal or polyclonal antibody.
18. A monoclonal or polyclonal antibody obtainable by the method of any one of items 15-17.
19. Use of a polypeptide comprising amino acids 93 to 121 of the amino acid sequence shown in SEQ ID NO:1 or a polypeptide comprising amino acids 92 to 122 of the amino acid sequence shown in SEQ ID NO:2 for immunizing a non-human animal.
20. A polypeptide consisting of an amino acid sequence corresponding to
   (i) the amino acid sequence between amino acids 93 and 121 of the amino acid sequence shown in SEQ ID NO:1 (Figure 1); or
   (ii) the amino acid sequence between amino acids 92 and 122 of the amino acid sequence shown in SEQ ID NO:2 (Figure 2).
21. An epitope contained in the polypeptide of item 21, said epitope contains at least 5 amino acids.

The Figures show:
**Figure 1****: Mouse Syndecan-4 amino acid sequence**
**Figure 2****: Human Syndecan-4 amino acid sequence**
**Figure 3****: Location of the amino acid sequence within the mouse Syndecan-4 protein against which the antibody was raised (a) and Syndecan-4 Alignment (b)**
   - signal peptide: dotted line
   - extracellular domain: dark grey shaded (1^{st} underlined sequence GSDDFELSGSG is the heparan sulphate attachment site, 2^{nd} underlined sequence NAQP is the cell attachment site)
   - transmembrane domain: italics
   - cytoplasmic domain: boxed (underlined sequences RMKKKDEGSYDL and APTNEFYA are conserved regions, GKKPIYKK is the variable region
   - antigen sequence NAQPGIRVPSEPKELEENEVIPKrAPSDV: double-underlined
   - r, v: plasmin- (r, v) and thrombin- (r) cleavage sites
**Figure 4****: Role of syndecan-4 in cartilage degradation**
   The diagramm shows the proposed pathway of syndecan-4 signalling. IL-1 binds to the side chains of syndecan-4 and thereby leads to dimerization of syndecan-4. The syndecan-4 dimer signals via ERK and induces the expression of MMP-3. MMP-3 activates ADAMTS-5, which binds to the side chains of syndecan-4 and gets fixed to the cell surface. The bound ADAMTS-5 cleaves aggrecan of ECM and mediates the cartilage degradation.

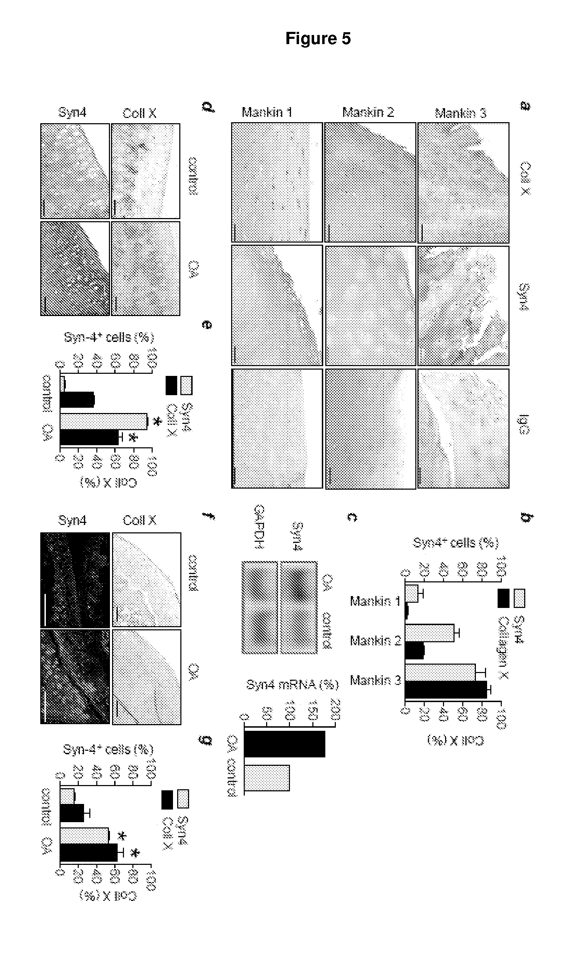

**Figure 5****: Expression of syndecan-4 in human, rat and mouse is upregulated in osteoarthritic chondrocytes and correlates with the progression of the disease**
   a) Staining for syndecan-4 (SDC4, black, middle column) and collagen X (blue, left column) in superficial chondrocytes of osteoarthritic subjects. IgG served as control of the primary antibody (right column). Severity of OA is indicated by Mankin scores. Counterstaining: Safranin-Orange, scale bar, 200 µm.
   **b)** Number of SDC4 stained cells (grey bars) and amount of collagen X (black bars) in analysed slices (*n*=15).
   **c)** Northern-blot analysis of syndecan-4 mRNA expression in chondrocytes isolated from healthy (Control) and osteoarthritic cartilage (OA) with quantification.
   **d)** Expression of syndecan-4 (black) and collagen X (blue) in knee sections of exercise induced osteoarthritic rats (OA) and in non-exercise controls (Control). Counterstaining: Safranin-Orange, scale bar, 200 µm.
   **e)** Number of cells stained for syndecan-4 (grey bars) and intensity of collagen X stain (black bars) in analyzed slices (*n*=4 each group, *P<0.05)).
   **f)** Expression of syndecan-4 (green) and collagen X (blue) in knee sections of wild-type (WT) mice after induction of joint instability (OA) and in control knees (Control). Scale bar, 200 µm.
   **g)** Number of cells stained for syndecan-4 (grey bars) and the intensity of collagen X staining (black bars) is significantly in analyzed slices (*n=*3 each group, **P*<0.05).

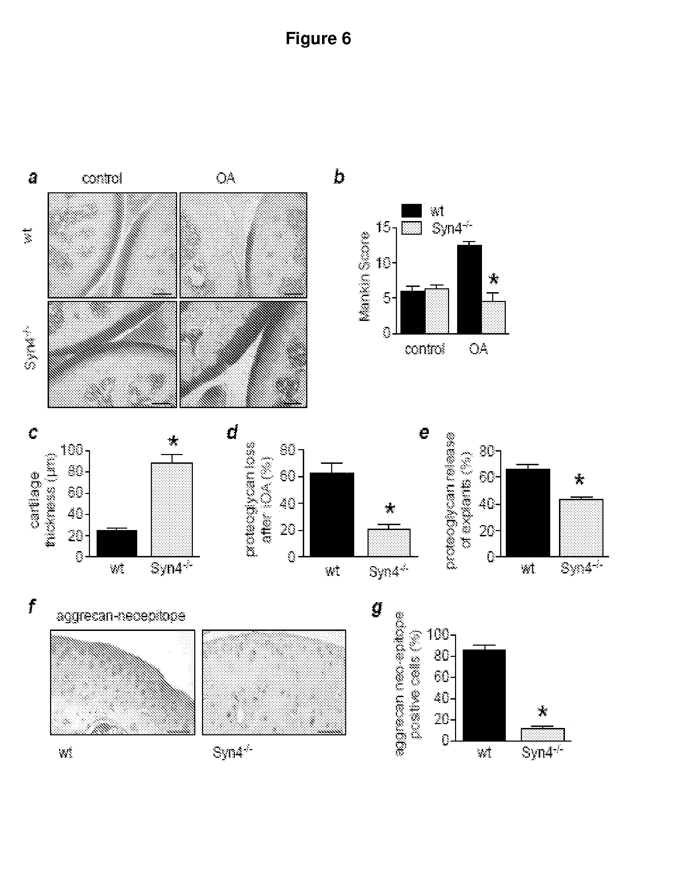

**Figure 6****: Deletion of syndecan-4 protects OA cartilage from proteoglycan loss that is accompanied by reduced aggrecan neo-epitope staining**
   **a)** Safranin-Orange staining of knee sections in control joints (Control) and joints with induced osteoarthritis (OA) from wild-type (wt) and *Sdc4*^{*-*/*-*} mice. Scale bar, 200 µm.
   **b)** Mankin score of control (Control) and induced osteoarthritic (OA) articular cartilage in wild-type (WT, black bars) and *Sdc4*^{*-*/*-*} mice (*Sdc4*^{*-*/*-*}, grey bars) (*n=*4 each group, **P*<0.05 versus WT).
   **c)** Morphometric analysis of cartilage thickness in osteoarthritic WT and *Sdc4*^{-/-} cartilage (*n=*4 each group, **P*<0.05 versus WT).
   **d)** Percentage of proteoglycan loss in the knee cartilage of osteoarthritic WT and *Sdc4*^{*-*/*-*} mice (*n=*4 each group, **P*<0.05 versus WT).
   **e)** IL-1-induced proteoglycan release in cultured cartilage explants of WT and *Sdc4*^{*-*/*-*} mice (*n=*4 each group, **P*<0.05 versus WT).
   **f)** Immunostaining of aggrecan neo-epitope (³⁷⁴ARGSV) after induction of osteoarthritis in WT and *Sdc4*^{*-*/*-*} mice. Scale bar, 100 µm.
   **g)** The number of aggrecan neo-epitope stained chondrocytes after induction of osteoarthritis in WT and *Sdc4*^{*-*/*-*} mice (*n=*3 each group, **P*<0.05 versus WT).

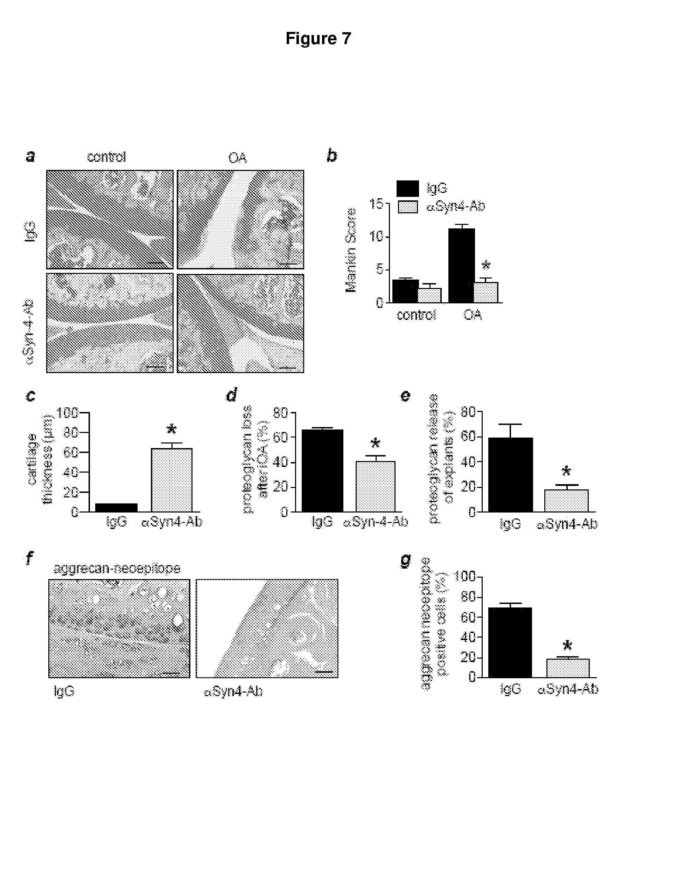

**Figure 7****: Injection of syndecan-4-specific antibodies into osteoarthritic knees of wild-type mice protects from proteoglycan loss that is accompanied by reduced aggrecan neo-epitope staining**
   **a)** Safranin-Orange staining of sections from control (Control) knees and osteoarthitic (OA) knees of wild-type mice treated with either rabbit IgG (IgG) or syndecan-4-specific antibodies (anti-Sdc4-Ab). Scale bar, 200 µm.
   **b)** Mankin score of control and osteoarthitic knees of wild-type mice treated with either IgG or anti-Sdc4-Ab **(*n=*8,** **P*<0.05 versus IgG).
   **c)** Morphometric analysis of knee cartilage thickness in osteoarthritic wild-type mice treated with either IgG or anti-Sdc4-Ab (*n=*10, **P*<0.05 versus IgG).
   **d)** Percentage of proteoglycan loss in the knees of osteoarthritic wild-type mice treated with either IgG or anti-Sdc4-Ab (*n=*10, **P*<0.05 versus IgG).
   **e)** IL-1 induced proteoglycan release in cultured cartilage explants of wild-type mice treated with either IgG or anti-Sdc4-Ab (*n=*10, **P*<0.05 versus IgG).
   **f)** Immunostaining of aggrecan neo-epitope (³⁷⁴ARGSV) in the knee cartilage of wild-type mice treated with either IgG or anti-Sdc4-Ab. Scale bar, 100 µm.
   **g)** Number of aggrecan neo-epitope stained chondrocytes in the knees of osteoarthritic wild-type mice treated with either IgG or anti-Sdc4-Ab (*n=*10, **P*<0.05 versus IgG).

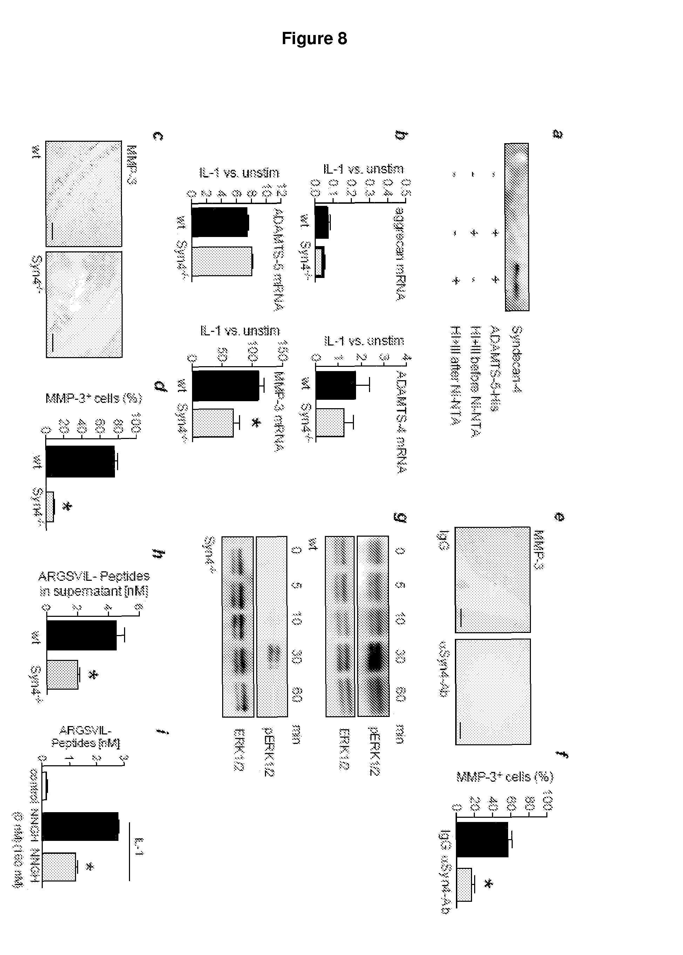

**Figure 8****: The activation of ADAMTS-5 depends on its direct interaction with syndecan-4 and on syndecan-4 regulated MMP3 expression and activity**
   **a)** Syndecan-4 (SDC4) immunoblot of heparitinase (HI+III) treated protein complexes of syndecan-4 and histidine-tagged ADAMTS-5 (ADAMTS-5-His) after purification though a Nickel-NTA column (Ni-NTA).
   **b)** Real time PCR of *aggrecan, Adamts4* and *Adamts5* and *Mmp3* in IL-1 induced cartilage cultures of WT and *Sdc4*^{*-*/*-*} mice (*n=*4, **P*<0.05 versus WT)
   **c) e)** Immunostaining of Mmp3 (red) in articular cartilage after induction of osteoarthitis in *Sdc4*^{*-*/*-*} and anti-Sdc4-Ab treated mice as compared to wild-type (WT) mice and IgG treated mice, respectively. Scale bar, 100 µm.
   **d) f)** Number of Mmp3 stained chondrocytes after induction of osteoarthritis in *Sdc4*^{*-*/*-*} mice and anti-Sdc4-Ab treated mice as compared to wild-type (WT) mice and IgG treated mice, respectively (*n=*4 each group, **P*<0.05 versus WT and IgG, respectively).
   **g)** Western blots with phospho-specific ERK1/2 antibody (p-ERK) after different time points of IL-1 treatment in wild-type (WT) and *Sdc4*^{-/-} cartilage. Antibodies against total ERK1/2 (ERK) were used as loading control.
   **h)** Aggrecanase activity in wild-type (WT) and *Sdc4*^{-/-} cartilage explants after IL-1 treatment (*n=*4).
   i) Effect of Mmp3 inhibitor (NNGH) on aggrecanase activity in untreated WT cartilage (Control) and in WT cartilage treated with IL-1 (*n=*4, **P*<0.05 versus IL-1 treated cartilage without NNGH).

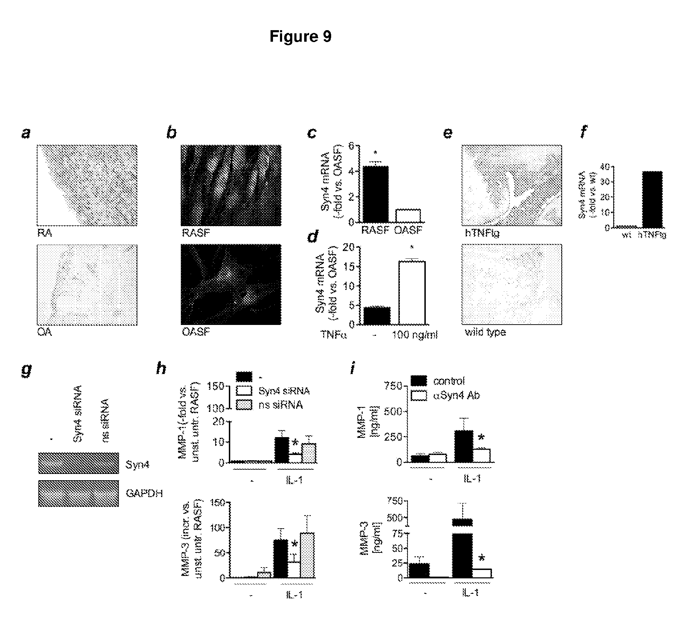

**Figure 9****: Syndecan-4 is increased in chronic inflammation by TNFalpha and regulates IL-1 induced MMP production**
   **a)** Enhanced syndecan-4 staining in tissue sections from rheumatoid patients (RA) compared to osteoarthritic patients (OA). Original magnification X200.
   **b)** At the cellular level, high syndecan-4 expression is localized at the surface of RASF vs. OASF (green).
   c) Quantitative real-time PCR of syndecan-4 mRNA level in RASF and OASF revealed upregulation of syndecan-4 in RA patients (n=10, p<0.05).
   **d)** Synovial fibroblasts of RA patients (n=5) were stimulated with TNFalpha. Syndecan-4 mRNA levels were analyzed by quantitative real-time PCR and normalized to HPRT. Values are shown in comparison to unstimulated control cells from OA patients (*p<0.05).
   **e)** Immunohistochemical staining of synovial tissue sections of hTNFtg and wild type tissue mice with monoclonal syndecan-4 antibody (red) showed a high syndecan-4 staining in the pannus. The nuclei were counterstained with methyl green. (Original magnification X200.)
   **f)** Quantitative real-time PCR showed a 36.5-fold upregulation in synovial fibroblasts of hTNFtg mice as compared to synovial fibroblasts of wild type mice normalized to HPRT.
   **g)** Syndecan-4 was knocked down in RA synovial fibroblasts by siRNA and supernatants of transfected and stimulated RASF were used for MMP-3 ELISA.
   **h)** Knock down of syndecan-4 significantly reduced the IL-1 induced expression of MMP-1 and -3 while nonsense siRNA had no effects (n=5, p<0.05 vs. untransfected cells).
   i) Inhibition of syndecan-4 by a polyclonal antibody directed against the membrane proximal part of the extracellular domain of syndecan-4 resulted in a downregulation of MMP-1 and MMP-3 production following IL-1 treatment (n=4, p<0.05 vs. controls).

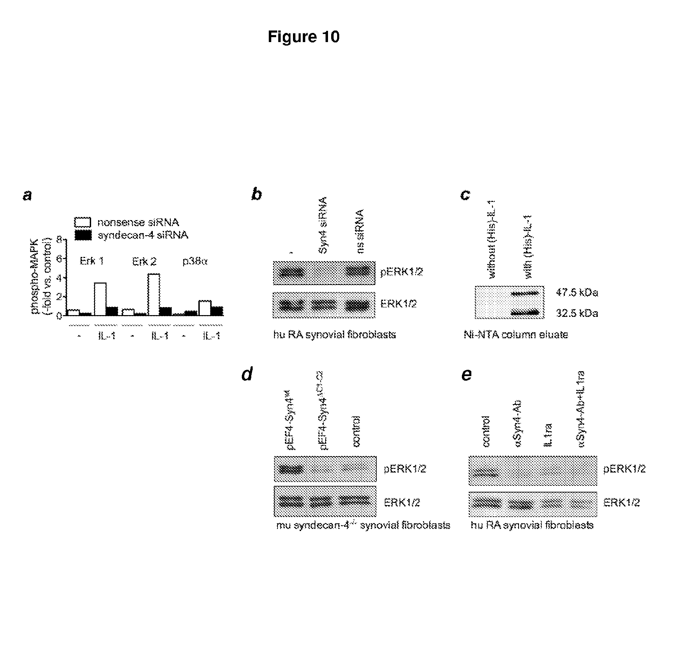

**Figure 10****: Syndecan-4 modulates IL-1 ERK1/2 phosphorylation by acting as an alternative IL-1 binding factor**
   **a)** Representative image of Proteome ProfilerTM Human Phospho-MAPK array blot. RASF were transfected with syndecan-4 siRNA and stimulated with IL-lalpha over 20min. When compared to nonsense siRNA, knock down of syndecan-4 significantly reduced the IL-1 mediated ERK1 and ERK2 phosphorylation.
   **b)** For western blot analysis, human RA synovial fibroblasts were transfected with syndecan-4 siRNA and stimulated with IL-1. Knock down of syndecan-4 reduced phospho-ERK1/2 after stimulation with IL-1 while no changes were seen in total ERK levels.
   **c)** Binding of IL-1 to syndecan-4 was demonstrated by the release of syndecan-4 from IL-1/syndecan-4 complexes isolated form Nickel-NTA column material after incubation of wild type murine synovial fibroblasts with or without recombinant histidinetagged IL-1.
   **d)** murine syndecan-4-/- fibroblasts were reconstituted with a syndecan-4 construct lacking cytoplasmic region C1-C2 (pEF4-Syn4ΔC1-C2) or with full-length syndecan-4 (pEF4-Syn4wt) and stimulated with IL-1. ERK1/2 phosphorylation was detected only in syndecan-4 deficient synovial fibroblasts expressing the full-length syndecan-4 sequence (pEF4-Syn4wt), whereas syndecan-4-/- cells (control) or syndecan-4-/- cells expressing syndecan-4 without the cytoplasmic region C1-C2 (pEF4-Syn4ΔC1-C2) showed no increase in ERK1/2 phosphorylation.
   **e)** human synovial fibroblasts were analysed for ERK phosphorylation in the presence of the human recombinant IL-1 receptor antagonist (IL-1ra), blocking antibodies against syndecan-4 or both. ERK phosphorylation was seen also, though to a lesser extent, when the IL-1 receptor signaling was blocked through IL-1ra. Blocking antibodies against syndecan-4 (αSyn4-Ab) had a clear inhibitory effect on IL-1 mediated ERK phosphorylation and the combination of both syndecan-4 and IL-1ra (IL1ra+ αSyn4-Ab) antibodies nearly completely prevented the phosphorylation of ERK.

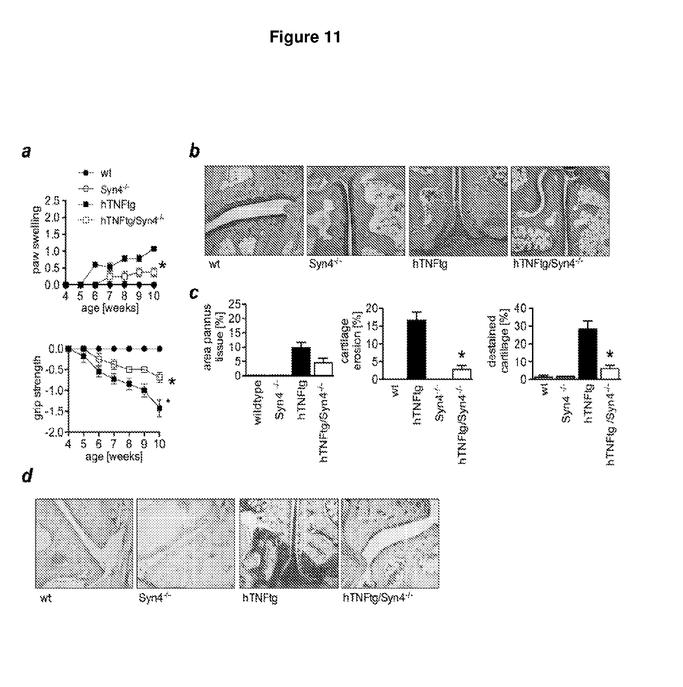

**Figure 11****: Syndecan-4 deficiency ameliorates the arthritis in the hTNFtg mouse model of RA and prevents hTNFtg mice from MMP- induced cartilage damage**
   **a)** Wild type, syndecan-4-/-, hTNFtg and hTNFtg/syndecan-4-/- mice were scored once a week for paw swelling and grip strength. Paw swelling was delayed and significantly reduced in its severity in hTNFtg/syndecan-4-/- mice accompanied by a higher grip strength when compared with hTNFtg animals (n=8, *p<0.05 at week 10).
   b) Representative Toluidine blue staining of hind paw sections of 8 weeks old wild type, syndecan-4-/-, hTNFtg and hTNFtg/syndecan-4-/- mice are shown. (original magnification x100).
   c) Mean areas of synovial pannus tissue, cartilage erosion (in percent of cartilage) and destaining of the cartilage (in percent of total cartilage) in the tarsal joints was measured. hTNFtg/syndecan-4-/- mice revealed within the articular cartilage a decreased pannus formation that resulted in the preservation of articular cartilage in comparison to hTNFtg mice (n=8, *p<0.05 at week 10).
   **d)** MMP-3 staining (red) of hind paw sections from analyzed mice. Loss of syndecan-4 in hTNFtg/syndecan-4-/- mice caused a strong downregulation of MMP-3 expression in the pannus tissue in comparison to hTNFtg mice. MMP-3 staining in wild type and syndecan-4-/- animals is restricted to a small number of chondrocytes in the articular cartilage.

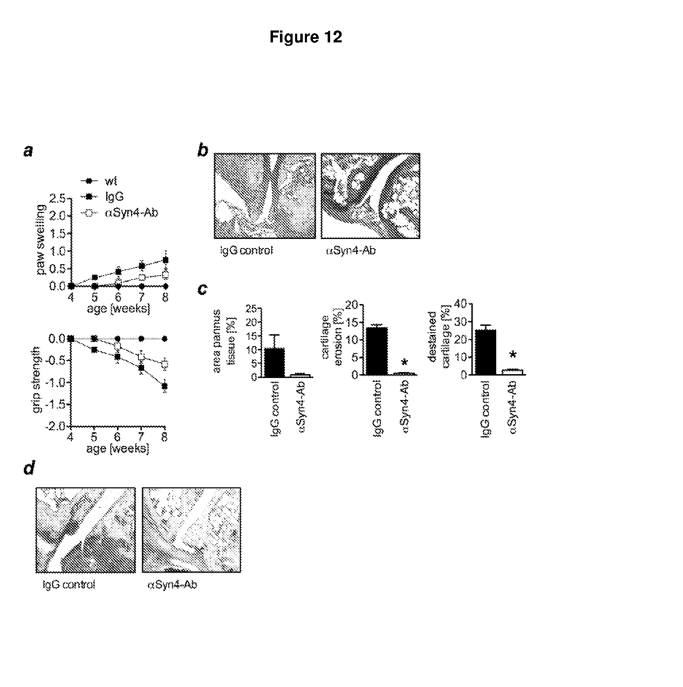

**Figure 12****: Antibodies against syndecan-4 ameliorates the hTNFtg arthritis and prevent hTNFtg mice from MMP- induced cartilage damage**
   **a)** hTNFtg were treated with anti-syndecan-4 Antibodies (αSyn4-Ab) or non-specific IgG (IgG control) from week 4 to week 8 and scored once a week for paw swelling and grip strength. Paw swelling was delayed and reduced in its severity in hTNFtg/syndecan-4-/- mice accompanied by a higher grip strength when compared with hTNFtg animals (n=6).
   **b)** Representative Toluidine blue staining of hind paw sections of 8 weeks old hTNFtg mice treated with anti-syndecan-4 Antibodies (αSyn4-Ab) or non-specific IgG (IgG control) are shown (original magnification x100).
   c) Mean areas of synovial pannus tissue, cartilage erosion (in percent of cartilage) and destaining of the cartilage (in percent of total cartilage) in the tarsal joints was measured. Treatment of hTNFtg mice with anti-syndecan-4 Antibodies (αSyn4-Ab) showed decreased pannus formation resulting in the preservation of cartilage in comparison to IgG treated hTNFtg mice (n=12, *p<0.05 at week 8).
   **d)** MMP-3 staining (red) of hind paw sections. Anti-syndecan- 4 antibody (αSyn4-Ab) treatment of hTNFtg mice caused a strong downregulation of MMP-3 expression in the pannus tissue in comparison to IgG treated hTNFtg mice.

The following examples are offered for illustrative purposes only, and are not intended to limit the scope of the present invention in any way.

### I. Experimental results (Syndecan-4 in osteoarthritis)

### Example 1: Syndecan-4 is expressed in cartilage of osteoarthritic subject

It was analyzed if Syndecan-4 protein is expressed in adult knee cartilage of osteoarthritis subjects and non-arthritic controls. In the superficial zone of human osteoarthritic cartilage, a strong cellular staining for Syndecan-4 in up to 80% of chondrocytes was detected that was accompanied by enhanced expression of collagen type X and proteoglycan loss (Fig. 5a). These typical changes of osteoarthritis and the number of Syndecan-4 expressing chondrocytes correlated with the histological severity of the disease as assessed by Mankin score (Fig. 5b) suggesting a hypertrophic fate of the chondrocytes. In contrast, sections with low Mankin scores were largely negative for Syndecan-4 and collagen type X. Staining controls using rabbit IgG instead of the primary antibodies were also negative. An increased expression of Syndecan-4 mRNA in chondrocytes isolated from osteoarthritic cartilage samples by Northern-blot was also observed (Fig. 5c). In a rat model of disease, where early osteoarthritic changes were induced by exposing the animals to extensive running, Syndecan-4 expression was significantly elevated in collagen type X expressing chondrocytes and - most likely due to Syndecan-4 ectodomain shedding - also in the surrounding matrix (Fig. 5d, e). In contrast, cartilage from non-exercised control rats did not stain for Syndecan-4 (Fig. 5d, e).

### Example 2: Induction of osteoarthrosis in mice elevates Syndecan-4 expression

Osteoarthritis was induced in mice through surgically achieved unilateral joint instability as this mouse model of osteoarthritis has been used successfully to investigate the role of a number of genes including ADAMTS-4, ADAMTS-5 and MMP-3. A strong and early induction of Syndecan-4 (54% positive cells) and collagen X (61% of total) protein expression in articular chondrocytes of osteoarthritis cartilage was found (Fig. 5f, g), whereas chondrocytes of the control knee cartilage show minimal Syndecan-4 (16% positive cells) and collagen X staining (22% of total, Fig. 5f, g). These data indicated that the elevated expression of Syndecan-4 in collagen type X expressing chondrocytes is a characteristic event during the development of human osteoarthritis and in animal models of the disease and prompted us to analyze the functional relevance of a Syndecan-4 deletion for the development of osteoarthritis-like changes in mice.

### Example 3: Induction of osteoarthrosis in Syndecan-4 knockout mice elevates Syndecan-4 expression

Osteoarthritis was induced in Syndecan-4 knockout (Sdc4-/-) mice, which are viable and have no gross abnormalities besides a delay in mesenchymal cell based skin wound repair. By Safranin-Orange staining typical signs of proteoglycan loss in the operated knees of wild-type controls was visible, whereas a strong staining was found in the knee cartilage of the Sdc4-/- mice. Using the Mankin score, which reflects abnormalities in cartilage structure, cell distribution, Safranin-Orange stain and tidemark integrity, an increase in the operated knees of wild-type mice to a mean of 12.5 points was found (Fig. 6b) with a significant reduction of cartilage thickness (Fig. 6c) and a substantial loss of proteoglycans (Fig. 6d). Syndecan-4 deficiency completely protected the mice from the development of osteoarthritis-like changes, and this was reflected by a normal Mankin score (Fig. 6b), preserved cartilage thickness (Fig. 6c). When quantifying the Safranin-Orange stained cartilage area in the operated knee joints, Sdc4-/- mice showed less than 30% of the proteoglycan loss of wild-type animals (Fig. 6d). Confirming these findings in vitro, IL-1 treated cartilage explants from Sdc4-/- mice showed a significantly reduced release of proteoglycans as compared to wild type controls (Fig. 6e). During osteoarthritis ADAMTS-4 and -5 activity is increased in articular chondrocytes10 which changes the physiological balance between matrix synthesis and degradation and results in an enhanced aggrecan proteolysis.

### Example 4: Loss of Syndecan-4 protects mice from osteoarthrits-like changes

Since the loss of Syndecan-4 protected mice from early cartilage degradation, it was analyzed whether the appearance of ADAMTS mediated aggrecan cleavage products. While wild-type mice showed a prominent aggrecan neo-epitope staining throughout the arthritic cartilage, we noticed a reduced staining for aggrecan neo-epitopes in Sdc4-/- osteoarthritis cartilage (Fig. 6f). Morphometric analysis of the cartilage sections showed that 80% of wild-type but only 20% of Sdc4-/- chondrocytes were surrounded by an aggrecan neo-epitope positive matrix (Fig. 6g). These data clearly demonstrated that the loss of Syndecan-4 in a similar way as ADAMTS-5 deficiency protects mice from the development of osteoarthritis-like changes.

### Example 5: Antibodies against Syndecan-4 protect mice from osteoarthritis

To exclude that developmental effects in Sdc4-/- mice are responsible for the protection against cartilage degradation, polyclonal antibodies raised against the C-terminal part of the extracellular domain of Syndecan-4 (see SEQ ID NO:11) were used to block Syndecan-4 function during cartilage degradation in wild-type cartilage or in wild-type mice with surgically induced osteoarthritis. In vitro, the IL-1 induced release of proteoglycans in wild-type cartilage was efficiently blocked by Syndecan-4-specific antibodies (anti-Sdc4-Ab) treatment to 18%, whereas medium or IgG treated cartilage lost nearly 60% of their proteoglycan content. Based on these data, the anti-Sdc4-Ab were injected into the knee joint of wild-type mice with surgically induced osteoarthritis and found a protection of anti-Sdc4-Ab treated joints from typical osteoarthritic changes with a mean Mankin-score of 3.1 that characterizes healthy cartilage (Fig. 7a, b). Moreover, anti-Sdc4-Ab treatment prevented the thinning of cartilage (65µm compared to 8 µm in the IgG controls, Fig. 7c) and caused a significant reduction in the release of proteoglycans (41% versus 66% in the IgG treatment controls, Fig. 7d). In line with our above data in the Sdc4-/- mice, treatment of wild-type mice with the anti-Sdc4-Ab also prevented the generation of cleavage products of aggrecan (Fig. 7f, g).

For the production of polyclonal antibodies rabbits were immunized three times with a peptide having the amino acid sequence shown in SEQ ID NO:11.

### Example 6: Syndecan-4 interacts directly with ADAMTS-5

In light of the above described data, it was investigated as to how Syndecan-4 is involved in cartilage degradation and why blocking of Syndecan-4 protects from osteoarthritic damage. First, it was investigated if there is a direct interaction between ADAMTS5 and Syndecan-4. To this end, recombinant histidine-tagged ADAMTS5 was added to IL-1-stimulated human chondrocytes and detected Syndecan-4 as a single 32.5 kDa band in isolated and heparitinase treated protein complexes (Fig. 8a). These data demonstrated that Syndecan-4 can bind ADAMTS-5 via its heparin sulfate chains and at the same time raised the question whether binding of ADAMTS-5 to Syndecan-4 is associated with an increased production of this enzyme or rather facilitates its activation by other mechanisms.

### Example 7: Syndecan-4 modulates activation of ADAMTS-5 by controlling the release of MMP-3

Next it was analyzed if Syndecan-4 deficiency is regulating IL-1 induced synthesis of aggrecan or of aggrecan degrading proteases and their inhibitors in cartilage organ cultures. As expected, an IL-1-dependent decrease in aggrecan synthesis was observed and an increase in the expression of cartilage proteases in wild-type cartilage explants, the latter of which was most prominent for Adamts5 and Mmp3. While Syndecan-4 deficiency did not significantly alter the IL-1-induced expression of mRNA for aggrecan or the aggrecanases ADAMTS-4 and ADAMTS-5, it lead to a significantly reduced induction of Mmp3 (67.6 fold in Sdc4-/- cartilage versus 107.7 fold in wild-type cartilage, Fig. 8b). IL-1-induced expression of other disease relevant MMPs and of tissue inhibitors of metalloproteinases was not altered significantly in the absence of Syndecan-4 (not shown). These data suggested that Syndecan-4 modulates the activation of ADAMTS-5 by controlling the release of MMP-3 rather than by regulating the expression of Adamts5 directly. These results correspond with data showing that aged Mmp3-/- mice are protected against cartilage damage occurring through spontaneous osteoarthritis.

Therefore, we next analyzed if Syndecan-4 regulates the expression of Mmp3 in osteoarthritis chondrocytes in vivo. As expected for osteoarthritis in wild-type mice, we noticed a strong Mmp3 straining in about 80% of the chondrocytes. In contrast, only few chondrocytes were positive for Mmp3 in Sdc4-/- mice, and the staining intensity was markedly reduced (Fig. 8c, d). A similar reduction in the staining intensity and distribution for Mmp3 was seen in osteoarthritic wild-type mice treated with anti-Syn4-Ab as compared to the IgG treated controls (Fig. 8e, f).

### Example 8: MMP-3 is involved in the activation of cell-surface bound ADAMTS-5

IL-1-induced expression of MMP-3 is mediated through mitogen activated protein kinases (MAPKs). Therefore, it was analyzed, if the loss of Syndecan-4 affects IL-1-induced MAPK phosphorylation. It was documented an IL-1 dependent phosphorylation of p38 MAPK, JNK and ERK1/2 both in wild-type and in Sdc4-/- cartilage explants. However, while IL-1 induced phosphorylation of p38 MAPK or JNK was not different between wild-type and Sdc4-/- cartilage (not shown), the loss of Syndecan-4 clearly inhibited the IL-1-mediated phosphorylation of ERK1/2 at all time points (Fig. 8g), suggesting that Syndecan-4 controls the expression of Mmp3 through regulation of ERK1/2 phosphorylation. These findings indicated that Mmp3 is involved in the activation of cell-surface bound Adamts5 and made us wonder whether syndcan-4 deficiency and Mmp3 inhibition in a similar way inhibit aggrecanase activity.
In line with what was expected from the in vivo data (Fig. 6f, 7f), aggrecanase activity was significantly reduced in the supernatant of IL-1 treated Sdc4-/- cartilage explants (2nM ARGSVL peptides in Sdc4-/- versus 4.1 nM in wild-type cartilage, Fig. 4h). Since Syndecan-4 regulated Mmp3 but not aggrecanase expression, we finally analyzed, if Mmp3 contributes to the activation of aggrecanases. The activity of Mmp3 was blocked with the specific MMP-3 inhibitor NNGH and found a significantly reduced aggrecanase activity in the supernatant of IL-1 stimulated wild-type cartilage cultures (1.2 nM ARGSVIL peptides versus 2.8 nM in controls, Fig. 8i). Of note, NNGH did not directly affect aggrecanase activity (data not shown), indicating that Mmp3 indeed contributes to the activation of ADAMTS-5.

### II. Experimental results (Syndecan-4 in rheumatoid arthritis)

### Example 1: Syndecan-4 is expressed in rheumatiuc cartilage

To understand the role of Syndecan-4 in rheumatoid cartilage destruction, it was first investigated the expression and tissue distribution of Syndecan-4 in synovial membranes of RA patients. As shown in Fig. 9a, Syndecan-4 was highly expressed in RA synovial membranes with very prominent staining in the most superficial lining layer that mediates the attachment to and degradation of articular cartilage. In contrast, only very few Syndecan-4 expressing cells were found in synovial tissues from OA patients. Immunocytochemical analysis revealed a significant upregulation of Syndecan-4 expression on primary synovial fibroblast of RA patients compared to OA patients (Fig. 9b). The upregulation was also evident at the mRNA level as determined by Northernblot, where RASF expressed significantly higher levels of Syndecan-4 than OASF (Fig. 9c). These data suggested that the inflammatory environment in the RA synovium leads to a strong and sustained upregulation of Syndecan-4 in synovial fibroblasts. Indeed, stimulation of synovial fibroblasts with tumor necrosis factor alpha (TNFalpha), a main inflammatory cytokine in RA, resulted in a further significant increase in the expression of Syndecan-4 mRNA. As measured by quantitative PCR, TNFalpha induced the expression of Syndecan-4 both in RASF and OASF, but both basal levels of Syndecan-4 and the level of upregulation were significantly higher in RASF (Fig 9d).
To further study if chronic exposure to TNFalpha results in a sustained upregulation of Syndecan-4 in synovial fibroblasts, human TNF transgenic (hTNFtg) which develop an RA- like chronic destructive arthritis were analyzed for the expression of Syndecan-4. As seen in immunohistochemistry, there was a strong expression of Syndecan-4 in the synovial membranes of hTNFtg (Fig. 9e) mice, whereas only negligible staining for Syndecan-4 was found in synovial tissues of wild type animals. In vitro, synovial fibroblasts isolated from hTNFtg mice showed more than 30-fold higher expression of Syndecan-4 than wild type controls (Fig. 9f).

### Example 2: Syndecan-4 contributes directly to the invasive behaviour of RA synovial fibroblasts

These findings prompted the present inventors to hypothesize that Syndecan-4 is involved in the activation of RA synovial fibroblasts and raised the question if Syndecan-4 contributes directly to the invasive behaviour of RA synovial fibroblasts. Based on the established role of interleukin-1 (IL-1) in regulating the expression of matrix degrading enzymes such as matrix metalloproteinases (MMPs) and in light of most recent data that had reinforced the concept of IL-1 as a critical mediator of cartilage breakdown in chronic destructive arthritis17, the role of Syndecan-4 in the IL-1 induced expression of disease-relevant MMPs was investigated. Of interest, knock down of Syndecan-4 in RA synovial fibroblasts by siRNA (Fig. 9g) significantly impaired the ability of these cells to produce MMPs in response to IL-1 stimulation. In untransfected RA synovial fibroblasts, stimulation with IL-1alpha for 24h resulted in an expected upregulation of MMP-1 and MMP-3 (Fig. 9h and i). However, when Syndecan-4 was knocked down by siRNA, IL-1 induced MMP-1 and MMP-3 production was decreased by more than 50% (Fig 9h) as determined by ELISA. Since knock down by RNAi only affects the neosynthesis of Syndecan-4, residual Syndecan-4 molecules on the cell surface may still mediate Syndecan-4 effects. Therefore, we next used established blocking antibodies directed against the membrane proximal part of the extracellular domain of Syndecan-4 to confirm our data. As shown in Fig. 9i, treatment of RA synovial fibroblasts with these antibodies largely abrogated the IL-1 induced upregulation of MMP-1 (Fig. 9i, upper panel) and MMP-3 (Fig. 9i, lower panel) indicating that indeed Syndecan-4 is required for IL-1 mediated induction of MMPs in RA synovial fibroblasts.

### Example 3: Syndecan-4 transduces IL-1 binding via phosphorylation of ERK 1/2 and a MAPK cascade

As mitogen activated protein kinases (MAPKs) have been identified as major regulators of IL-1 mediated MMP- expression, the role of Syndecan-4 in the activation of MAPK-pathways in response to IL-1 stimulation of RA synovial fibroblasts was analyzed. Using a human phospho-MAPK array we found that the knock down of Syndecan-4 not only reduced the basal levels of ERK1/2 phosphorylation in these cells as found in scleroderma fibroblasts, but even more pronouncedly affected IL-1 mediated induction of ERK phosphorylation. In fact, siRNA mediated knock down of Syndecan-4 nearly abolished the IL-1 induced activation of ERK1/2 (Fig. 10a). While, knock down of Syndecan-4 lead to a minor reduction in the activation of p38alpha, other MAPKs were not affected. These results were further confirmed by Western blot analysis, where knock down of Syndecan-4 in IL-1 treated RA synovial fibroblasts revealed a clear reduction of ERK1/2 phosphorylation that was almost equal to the basal not induced levels of ERK1/2 phosphorylation (Fig. 10e).

### Example 4: Syndecan-4 interacts directly with IL-1

The above data raised the question whether there is a direct interaction between IL-1 and Syndecan-4 and if Syndecan-4 itself is transmitting signals that result in ERK1/2 phosphorylation. To analyse a potential binding of IL-1 to Syndecan-4, recombinant histidine-tagged IL-1 was incubated with synovial fibroblasts from wild type mice. IL-1/Syndecan-4 complexes were purified on a Nickel-NTA column and Syndecan-4 protein was visualized by western blotting using the polyclonal Syndecan-4 antibody. Without the addition of histidine-tagged IL-1, no Syndecan-4 protein could be eluted from the Nickel NTA column, while Syndecan-4 could be detected both as a monomeric 32.5 kDa protein and as a dimer following the addition of histidine-tagged IL-1 (Fig. 10c). These data for the first time showed a direct interaction and binding of IL-1 to Syndecan-4 prompting the present inventors to investigate if Syndecan-4 is able to signal by itself after IL-1 binding.

The cytoplasmic domain of Syndecan-4 has been shown to be critically involved in binding of and activation of protein kinase C to allow signal transduction. Therefore the IL-1 dependent activation of ERK1/2 was analyzed in Syndecan-4 deficient murine synovial fibroblasts that were reconstituted either with a full-length Syndecan-4 construct (pEF4-Syn4wt) or with a mutant form of Syndecan-4 lacking the cytoplasmic domain (pEF4-Syn4ΔC1-C2). Of note, IL-1 was able to induce phosphorylation of ERK1/2 only in Syndecan-4-/- cells that were reconstituted with the full-length Syndecan-4, whereas cells expressing Syndecan-4 without the cytoplasmic domain were unable to respond to IL-1 with a phosphorylation of ERK1/2 and did not differ in their IL-1 response from Syndecan-4-/- cells (Fig 10d). To verify that Syndecan-4 is able to transmit IL-1 signals independently of the IL-1 receptor, human synovial fibroblasts were then analysed for ERK phosphorylation in the presence of the human recombinant IL-1 receptor antagonist (IL-1ra), blocking antibodies against Syndecan-4 or both. As shown in Fig. 10e, ERK phosphorylation was seen also, though to a lesser extent, when the IL-1 receptor signaling was blocked through IL-1ra. Consistent with our other data, blocking antibodies against Syndecan-4 had a clear inhibitory effect on IL-1 mediated ERK phosphorylation and the combination of both IL-1ra and Syndecan-4 antibodies completely prevented IL-1 induced phosphorylation of ERK (Fig. 10e). Taken together, these data demonstrate that Syndecan-4 interacts with IL-1 and regulates the production of MMP-1 and MMP-3 through the activation of ERK1/2. It is important to note that through binding to its cognate receptor, IL-1 triggers the activation of a number of signaling pathways. In addition to ERK, these include the NfkappaB pathway as well as the other MAPKs such as JNK24. This certainly explains why inhibition of Syndecan-4 by either RNAi or anti-Syndecan-4 antibodies did not prevent completely, the IL-1 induced upregulation of MMPs. However, these data also indicate that Syndecan- 4 provides an important second signal for IL-1 mediated ERK phosphorylation.

### Example 5: Syndecan-4 is involved in the breakdown of articular cartilage in RA

These data lead the present inventors to ask whether the loss of Syndecan-4 protects from cartilage damage during inflammatory destructive arthritis. To this end, Syndecan-4-/- mice were crossed with hTNFtg mice that develop a destructive RA- like disease. Interestingly, clinical symptoms of arthritis were clearly reduced in hTNFtg/Syndecan-4-/- mice in comparison to hTNFtg mice. As shown in Fig. 11 a, paw swelling was delayed and significantly reduced in its severity in hTNFtg/Syndecan-4-/- mice when compared with hTNFtg animals (p<0.05 at week 10). Both wild type and Syndecan-4-/- mice showed no clinical signs of arthritis. Amelioration of clinical arthritis in hTNFtg/Syndecan-4-/- mice was accompanied by a higher grip strength in these animals. As shown also in Fig. 11 a, hTNFtg/Syndecan-4-/- showed significantly higher values over time than hTNFtg mice suggesting that structural damage is less pronounced in the absence of Syndecan-4. To study the extent of cartilage damage, histological sections of hTNFtg/Syndecan-4-/- and hTNFtg mice as well as Syndecan-4-/- and wild type animals (representative pictures in Fig. 11 b) were evaluated for pannus invasion and cartilage destruction by histomorphometric analysis. As demonstrated in Fig. 11 b and c, the loss of Syndecan-4 decreased the invasion of pannus tissue into the articular cartilage and resulted in significantly reduced cartilage erosion and a reduced loss of cartilage components as measured by the percentage of destained cartilage that resulted in the preservation of articular cartilage. This was seen from significantly reduced cartilage erosion and a reduced loss of cartilage components as measured by the percentage of destained cartilage (Fig. 11 b, c). In line with our in vitro data, reduced cartilage destruction was accompanied by a strong decrease in synovial MMP-3 production (Fig. 11 d) These data raised the question if the timely application of our blocking anti-Syndecan-4 antibodies can prevent cartilage destruction in hTNFtg mice that express Syndecan-4 in the course of disease. To this end, the anti-Syndecan-4 antibodies were injected into 4 weeks old hTNFtg mice with our anti-Syndecan-4 antibodies. The antibodies were applied twice weekly for a periode of 4 weeks into the hind paws, and not only ameliorated the clinical signs of arthritis (Fig. 12a) but protected the treated joints nearly completely from cartilage damage (Fig. 12b, c). Again, this this statement is based not only on decreased pannus formation but even more pronouncedly on significantly decreased cartilage erosion and on decreased proteoglycan loss (Fig. 12c). Matching both our in vitro data and those in the Syndecan-4-/- mice, blocking Syndecan-4 with antibodies resulted in a reduced cartilage destruction that was accompanied by a strong decrease in synovial MMP-3 production in vivo (Fig. 12d).
Collectively, these data demonstrate that Syndecan-4 is involved critically in the breakdown of articular cartilage in RA. Exposure of both human RA synovial fibroblasts and murine synovial fibroblasts to TNFalpha results in a sustained upregulation of Syndecan-4 in vitro and in vivo. It was further shown that Syndecan-4 is upregulated early in the course of destructive arthritis and is an essential regulator of IL-1 signaling pathways leading to elevated levels of MMPs in fibroblast-like cells of the diseased synovium and that the activation of ERK1/2 by IL-1 depends on Syndecan-4. The loss of Syndecan-4 or the application of blocking antibodies against Syndecan-4 significantly reduces cartilage destruction in the hTNFtg mouse model of RA suggesting Syndecan-4 may be a highly interesting target for interfering with fibroblast activation and joint destruction in RA.

### Materials and Methods

### Cell culture and stimulation

Synovial fibroblasts were obtained from patients with rheumatoid arthritis and osteoarthritis during joint replacement surgery. Synovial fibroblasts from hTNFtg mice, syndecan-4-/- mice, hTNFtg/syndecan-4-/- mice and wild type mice were isolated from deskinned dipase II (Roche, Minneapolis, MN) digested hind paws. All cells were cultured in DMEM with 10% FCS at 37°C and 5% CO₂ until passages 4-6 as described26. For ELISA and western blot analysis, cells were seeded on Fibronectin coated dishes. Cells were stimulated for the indicated time with 100ng/ml human TNFalpha (Novitec) and 10ng/ml human IL-1alpha (R&D Systems).

### Animals

hTNFtg mice and syndecan-4-/- mice were back-crossed on C57BI/6 mice for at least 8 generations. For genotyping primer were used as described. Wild type, hTNFtg, syndecan-4-/- and hTNFtg/syndecan-4-/- mice were scored once a week for paw swelling and grip strength. For the antibody-experiments, our anti-syndecan-4 antibody or nonspecific IgG were injected twice weekly into the hind paws of the animals from week 4 to week 8.

### siRNA and transfection

High purity human syndecan-4 siRNA and nonsense siRNA oligonucleotides with 3'dTdT overhangs were used as described and purchased from Invitrogen (Carlsbad, CA). Downregulation of syndecan-4 in RASF was performed by siRNA transfection with Lipofectamin 2000 (Invitrogen) according to the manufactures instructions.

### Histology

Tissue samples from RA and OA patients as well as hind paws of wild type, syndecan-4-/-, hTNFtg and hTNftg/syndecan-4-/- mice were fixed in 4% paraformaldehyde overnight, decalcified in 10% EDTA/TBS, embedded into paraffin, sectioned into 5µm slices and stained with Tolouidine blue.

### Histomorphometric analysis

Inflammation and cartilage destruction was determined in Tolouidine blue stained tissue sections from 8 weeks old wild type, syndecan-4-/-, hTNFtg and hTNFtg/syndecan-4-/- mice. Measurements were performed using Spot 4.6 (Diagnostic Instruments Inc.).

### Immunohistochemistry

Tissue sections were pre-treated with 1x Trypsin/EDTA (PAA Laboratories, Pasching, Austria) for 20min at 37°C, blocked with 10% horse serum and stained with a monoclonal syndecan-4 antibody (Santa Cruz, clone 5G9) for human tissue sections and for mouse tissue section with a syndecan-4 antibody (BD Pharmingen, clone KY/8.2) over night at 4°C. Immunohistochemistry was performed with an alkaline phosphatase technique using Vectastain ABC-A, Vector Red Substance and secondary biotinylated antibodies (Vector Laboratories, Burlingame, CA). Nuclei were counterstained with methyl green (Vector Laboratories, Burlingame, CA). Immunohistochemical stainings for MMP-3 were done accordingly using a polyclonal anti-MMP-3 antibody (abcam, ab53015).

### Immunocytochemistry

RASF and OASF were seeded on glass cover slips, fixed with 4% paraformaldehyde including 0.1% Tween 20, blocked with 1% BSA and stained with the monoclonal syndecan-4 antibody 5G9 and an Alexa488 goat anti-mouse antibody (Molecular Probes) for detection of syndecan-4 expression9. Propidium iodide (Sigma) was used to stain the nuclei.

### Conventional and Reverse Transcriptase PCR

Synovial fibroblasts were plated on six-well plates (2x105 cell per well), transfected and cultured as described25. Cells were harvested 24h after stimulation with 100ng/ml TNFIalpha or 10ng/ml IL-1alpha. For quantitative real-time RT-PCR a CyberGreen Kit (Eppendorf), a RotoGene cycler (Corbett Life Science) and the following primer sets were used for quantification of syndecan-4 expression after normalizing mRNA levels to the house keeping genes GAPDH and/or HPRT: human syndecan-4 forward 5'-cgg gca gga atc tga tga ctt tga-3' (SEQ ID NO:12) and reverse: 5'-gct tca cgc gta gaa ctc att ggt-3' (SEQ ID NO:13); human GAPDH (forward 5'-tcc tgc acc acc aac tgc tt - 3' (SEQ ID NO:14) and reverse 5'-tcc acc acc ctg ttg ctg ta-3') (SEQ ID NO:15) and human HPRT (forward 5'-ctt tgc ttt cct tgg tca ggc agt-3' (SEQ ID NO:16) and reverse 5'-aac aac aat ccg ccc aaa ggg aac-3') (SEQ ID NO:17).

### Western Blot analysis of ERK1/2 phosphorylation

Total protein extracts were prepared after stimulation with IL-1alpha for 20 min from untransfected, syndecan-4 siRNA and nonsense siRNA transfected RASF or murine syndecan-4-/- synovial fibroblasts transfected either with the full-length syndecan-4 sequence or a cytoplasmic deletion construct cloned into the eukaryotic expression vector pEF4 (Invitrogen) containing the promotor of the elongation factor 4. Synovial fibroblasts were seeded on fibronectin, lysed in 1mM PMSF, 50mM Octylglycosid and 1mM MgCl2 containing buffer with the protease inhibitor mixture SetIII (Calbiochem, San Diego, CA) and separated on 10% SDS/PAGE. Proteins were detected by antibodies against phosphorylated and total ERK1/2 (Cell Signalling Technology, Beverly, MA).

### Phospho-MAPK Array

RASF were cultured on fibronectin, transfected with syndecan-4 and nonsense siRNA and treated with IL-1alpha for 20min. Sample preparation and the Proteome ProfilerTM Human Phospho-MAPK Array (R&D Systems, Minneapolis, MN, USA) were performed according to the manufacturer's instructions.

### MMP ELISAs

2x10⁵ RASF were seeded on fibronectin coated six-well plates, transfected with syndecan-4 and nonsense siRNA. Cells were stimulated with IL-1alpha and a blocking syndecan-4 antibody against the extracellular domain of syndecan over 24hrs and 6x10⁴ synovial fibroblsts of analyzed mouse models were seeded on 24-well plates. Supernatants were collected, centrifuged to remove cell debris and stored at -20°C. ELISAs were performed according to the manufacturer's instructions (R&D Systems).

### IL-1 binding to syndecan-4

5x10⁶ wild type murine synovial fibroblasts were incubated with 5µg recombinant histidine-tagged IL-1 or medium only for 1 hr, resuspended in lysis buffer containing 1% Triton X-100 and 0.1% SDS, bound to 500 µl Nickel-NTA column material (Qiagen) overnight at 4°C, eluted with 60 µl buffer containing 250 mM imidazole, digested with 0.004 U/ml heparitinase I (Seikagaku) overnight 28, subjected to SDS-Page on a 4-20% gradient gel, transfered to a PVDF membrane (Millipore), incubated with our polyclonal anti-syndecan-4 antibody, followed by a HRP conjugated secondary antibody (Sigma) and visualized by the Chemi-Smart-5100 imaging system (Vilber Lourmat).

### Statistical analysis

Data are shown as means ±SEM. Because of the likely non-Gaussian distribution of the data, a non-parametric Kruskal-Wallis test was performed, followed, if p<0.05, by a Mann-Whitney U test to identify significant differences between groups at p<0.05 (GraphPad Prism, GraphPad Inc., San Diego, Calif.).

## Claims

1. A recognition molecule against Syndecan-4, wherein said recognition molecule binds an epitope contained in an amino acid sequence corresponding to the amino acid sequence between amino acids 93 and 121 of the amino acid sequence shown in SEQ ID NO:1 (Figure 1) for use in the treatment and/or prevention of a pathological medical condition associated with turnover of the extracellular matrix and/or cartilage remodelling in which Syndecan-4 is involved.

2. The recognition molecule of claim 1, wherein an amino acid sequence corresponding to the amino acid sequence between amino acids 93 and 121 of the amino acid sequence shown in SEQ ID NO:1 is the amino acid sequence between amino acids 92 and 122 shown in SEQ ID NO:2.

3. The recognition molecule of any one of the preceding claims, wherein said antibody inhibits phosphorylation of ERK 1/2 by Syndecan-4.

4. The recognition molecule of any one of the preceding claims, wherein said antibody is a polyclonal or monoclonal antibody.

5. The recognition molecule of any one of the preceding claims, wherein said disorder is **characterized by** cartilage destruction/damage.

6. The recognition molecule of any one of the preceding claims, wherein said disorder is osteoarthritis.

7. The recognition molecule of any one of the preceding claims, wherein said disorder is rheumatoid arthritis.

8. A nucleic acid encoding the recognition molecule of any one of item 1-7.

9. A method for the generation of an antibody against Syndecan-4 which inhibits phosphorylation of ERK 1/2 by Syndecan-4, comprising
(a) immunizing a non-human mammal with a polypeptide comprising amino acids 93 to 121 of the amino acid sequence shown in SEQ ID NO:1 or with a polypeptide comprising amino acids 92 to 122 of the amino acid sequence shown in SEQ ID NO:2;
(b) isolating an antibody obtained in step(a); and
(c) determining whether the antibody obtained in step (b) inhibits phosphorylation of ERK 1/2 by Syndecan-4.

10. The method of claim 9, wherein the Sydecan-4 is the human Syndecan-4 protein.

11. The method of claim 9 or 10, wherein the antibody is a monoclonal or polyclonal antibody.

12. A monoclonal or polyclonal antibody obtainable by the method of any one of claims 9-11.

13. Use of a polypeptide comprising amino acids 93 to 121 of the amino acid sequence shown in SEQ ID NO:1 or a polypeptide comprising amino acids 92 to 122 of the amino acid sequence shown in SEQ ID NO:2 for immunizing a non-human animal.

14. A polypeptide consisting of an amino acid sequence corresponding to
(i) the amino acid sequence between amino acids 93 and 121 of the amino acid sequence shown in SEQ ID NO:1 (Figure 1); or
(ii) the amino acid sequence between amino acids 92 and 122 of the amino acid sequence shown in SEQ ID NO:2 (Figure 2).

15. An epitope contained in the polypeptide of claim 14, said epitope contains at least 5 amino acids.


SEQUENCE LISTING

<110>  Universitaetsklinikum Muenster

<120>  Anti-Syndecan-4 antibodies

<130>  UKM13614EP

<160>  17

<170>  PatentIn version 3.5

<210>  1
<211>  198
<212>  PRT
<213>  Mus musculus

<400>  1

Met Ala Pro Ala Cys Leu Leu Ala Pro Leu Leu Leu Leu Leu Leu Gly
1               5                   10                  15

Gly Phe Pro Leu Val Pro Gly Glu Ser Ile Arg Glu Thr Glu Val Ile
            20                  25                  30

Asp Pro Gln Asp Leu Leu Glu Gly Arg Tyr Phe Ser Gly Ala Leu Pro
        35                  40                  45

Asp Asp Glu Asp Ala Gly Gly Ser Asp Asp Phe Glu Leu Ser Gly Ser
    50                  55                  60

Gly Asp Leu Asp Asp Thr Glu Glu Pro Arg Pro Phe Pro Glu Val Ile
65                  70                  75                  80

Glu Pro Leu Val Pro Leu Asp Asn His Ile Pro Glu Asn Ala Gln Pro
                85                  90                  95

Gly Ile Arg Val Pro Ser Glu Pro Lys Glu Leu Glu Glu Asn Glu Val
            100                 105                 110

Ile Pro Lys Arg Ala Pro Ser Asp Val Gly Asp Asp Met Ser Asn Lys
        115                 120                 125

Val Ser Met Ser Ser Thr Ala Gln Gly Ser Asn Ile Phe Glu Arg Thr
    130                 135                 140

Glu Val Leu Ala Ala Leu Ile Val Gly Gly Val Val Gly Ile Leu Phe
145                 150                 155                 160

Ala Val Phe Leu Ile Leu Leu Leu Val Tyr Arg Met Lys Lys Lys Asp
                165                 170                 175

Glu Gly Ser Tyr Asp Leu Gly Lys Lys Pro Ile Tyr Lys Lys Ala Pro
            180                 185                 190

Thr Asn Glu Phe Tyr Ala
        195

<210>  2
<211>  198
<212>  PRT
<213>  Homo sapiens

<400>  2

Met Ala Pro Ala Arg Leu Phe Ala Leu Leu Leu Phe Phe Val Gly Gly
1               5                   10                  15

Val Ala Glu Ser Ile Arg Glu Thr Glu Val Ile Asp Pro Gln Asp Leu
            20                  25                  30

Leu Glu Gly Arg Tyr Phe Ser Gly Ala Leu Pro Asp Asp Glu Asp Val
        35                  40                  45

Val Gly Pro Gly Gln Glu Ser Asp Asp Phe Glu Leu Ser Gly Ser Gly
    50                  55                  60

Asp Leu Asp Asp Leu Glu Asp Ser Met Ile Gly Pro Glu Val Val His
65                  70                  75                  80

Pro Leu Val Pro Leu Asp Asn His Ile Pro Glu Arg Ala Gly Ser Gly
                85                  90                  95

Ser Gln Val Pro Thr Glu Pro Lys Lys Leu Glu Glu Asn Glu Val Ile
            100                 105                 110

Pro Lys Arg Ile Ser Pro Val Glu Glu Ser Glu Asp Val Ser Asn Lys
        115                 120                 125

Val Ser Met Ser Ser Thr Val Gln Gly Ser Asn Ile Phe Glu Arg Thr
    130                 135                 140

Glu Val Leu Ala Ala Leu Ile Val Gly Gly Ile Val Gly Ile Leu Phe
145                 150                 155                 160

Ala Val Phe Leu Ile Leu Leu Leu Met Tyr Arg Met Lys Lys Lys Asp
                165                 170                 175

Glu Gly Ser Tyr Asp Leu Gly Lys Lys Pro Ile Tyr Lys Lys Ala Pro
            180                 185                 190

Thr Asn Glu Phe Tyr Ala
        195

<210>  3
<211>  202
<212>  PRT
<213>  Rattur norwegicus

<400>  3

Met Ala Pro Val Cys Leu Phe Ala Pro Leu Leu Leu Leu Leu Leu Gly
1               5                   10                  15

Gly Phe Pro Val Ala Pro Gly Glu Ser Ile Arg Glu Thr Glu Val Ile
            20                  25                  30

Asp Pro Gln Asp Leu Leu Glu Gly Arg Tyr Phe Ser Gly Ala Leu Pro
        35                  40                  45

Asp Asp Glu Asp Ala Gly Gly Leu Glu Gln Asp Ser Asp Phe Glu Leu
    50                  55                  60

Ser Gly Ser Gly Asp Leu Asp Asp Thr Glu Glu Pro Arg Thr Phe Pro
65                  70                  75                  80

Glu Val Ile Ser Pro Leu Val Pro Leu Asp Asn His Ile Pro Glu Asn
                85                  90                  95

Ala Gln Pro Gly Ile Arg Val Pro Ser Glu Pro Lys Glu Leu Glu Glu
            100                 105                 110

Asn Glu Val Ile Pro Lys Arg Val Pro Ser Asp Val Gly Asp Asp Asp
        115                 120                 125

Val Ser Asn Lys Val Ser Met Ser Ser Thr Ser Gln Gly Ser Asn Ile
    130                 135                 140

Phe Glu Arg Thr Glu Val Leu Ala Ala Leu Ile Val Gly Gly Val Val
145                 150                 155                 160

Gly Ile Leu Phe Ala Val Phe Leu Ile Leu Leu Leu Val Tyr Arg Met
                165                 170                 175

Lys Lys Lys Asp Glu Gly Ser Tyr Asp Leu Gly Lys Lys Pro Ile Tyr
            180                 185                 190

Lys Lys Ala Pro Thr Asn Glu Phe Tyr Ala
        195                 200

<210>  4
<211>  202
<212>  PRT
<213>  Sus sp.

<400>  4

Met Ala Ser Pro Arg Leu Leu Ala Leu Leu Leu Leu Leu Val Gly Ala
1               5                   10                  15

Phe Asn Ala Ala Ala Ala Glu Ser Ile Arg Glu Thr Glu Val Ile Asn
            20                  25                  30

Pro Gln Asp Leu Leu Glu Gly Arg Tyr Phe Ser Gly Asp Leu Pro Asp
        35                  40                  45

Asp Glu Asp Val Gly Gly Pro Gly Gln Glu Pro Asp Asp Phe Glu Trp
    50                  55                  60

Ser Gly Ser Gly Asp Leu Glu Gly Pro Glu Asp Lys His Met Leu Pro
65                  70                  75                  80

Glu Val Thr His Pro Leu Val Pro Leu Asp Asn His Ile Pro Glu Arg
                85                  90                  95

Thr Gly Pro Gly Gly Arg Val Pro Thr Glu Pro Lys Glu Leu Glu Glu
            100                 105                 110

Asn Glu Val Ile Pro Lys Arg Met Ser Pro Phe Asp Gly Asp Glu Asp
        115                 120                 125

Val Ser Asn Lys Val Ser Met Ser Ser Thr Ala Gln Gly Ser Asn Ile
    130                 135                 140

Phe Glu Arg Thr Glu Val Leu Ser Ala Leu Ile Val Gly Gly Ile Val
145                 150                 155                 160

Gly Ile Leu Phe Ala Val Phe Leu Val Leu Leu Leu Val Tyr Arg Met
                165                 170                 175

Lys Lys Lys Asp Glu Gly Ser Tyr Asp Leu Gly Lys Lys Pro Ile Tyr
            180                 185                 190

Lys Lys Ala Pro Thr Asn Glu Phe Tyr Ala
        195                 200

<210>  5
<211>  197
<212>  PRT
<213>  Gallus sp.

<400>  5

Met Pro Leu Pro Arg Ala Ala Phe Leu Leu Gly Leu Leu Leu Ala Ala
1               5                   10                  15

Ala Ala Ala Glu Ser Val Arg Glu Thr Glu Thr Met Asp Ala Arg Trp
            20                  25                  30

Leu Asp Asn Val Gly Ser Gly Asp Leu Pro Asp Asp Glu Asp Ile Gly
        35                  40                  45

Glu Phe Thr Pro His Leu Thr Ser Asp Glu Phe Asp Ile Asp Asp Thr
    50                  55                  60

Ser Gly Ser Gly Asp Tyr Ser Asp Tyr Asp Asp Ala Ile Tyr Leu Thr
65                  70                  75                  80

Thr Val Asp Thr Pro Ala Ile Ser Asp Asn Tyr Ile Pro Gly Asp Thr
                85                  90                  95

Glu Arg Lys Met Glu Gly Glu Lys Lys Asn Thr Met Leu Asp Asn Glu
            100                 105                 110

Ile Ile Pro Asp Lys Ala Ser Pro Val Glu Ala Asn Leu Ser Asn Lys
        115                 120                 125

Ile Ser Met Ala Ser Thr Ala Asn Ser Ser Ile Phe Glu Arg Thr Glu
    130                 135                 140

Val Leu Thr Ala Leu Ile Ala Gly Gly Ala Val Gly Leu Leu Phe Ala
145                 150                 155                 160

Val Phe Leu Ile Leu Leu Leu Val Tyr Arg Met Lys Lys Lys Asp Glu
                165                 170                 175

Gly Ser Tyr Asp Leu Gly Lys Lys Pro Ile Tyr Lys Lys Ala Pro Thr
            180                 185                 190

Asn Glu Phe Tyr Ala
        195

<210>  6
<211>  199
<212>  PRT
<213>  Bos bovis

<400>  6

Met Ala Pro Ala Arg Leu Leu Ala Leu Leu Leu Leu Leu Val Gly Val
1               5                   10                  15

Pro Ala Ala Ala Ala Glu Ser Ile Arg Glu Thr Glu Val Ile Asp Pro
            20                  25                  30

Gln Asp Leu Leu Glu Ser Gln Tyr Phe Ser Gly Asp Leu Pro Asp Asp
        35                  40                  45

Glu Asp Val Gly Gly Pro Gly Gln Glu Ser Asp Asp Phe Glu Leu Ser
    50                  55                  60

Gly Ser Gly Asp Leu Asp Asp Ser Glu Asp Ile Arg Ile Phe Pro Glu
65                  70                  75                  80

Val Met His Pro Leu Val Pro Ile Asp Asn His Ile Pro Glu Lys Thr
                85                  90                  95

Gly Ser Gly Val Pro Thr Glu Pro Lys Glu Leu Glu Glu Asn Glu Val
            100                 105                 110

Ile Pro Lys Arg Ser Ser Pro Phe Asp Gly Asp Glu Asp Val Ser Asn
        115                 120                 125

Lys Val Ser Met Ser Ser Thr Ala Gln Gly Ser Asn Ile Phe Glu Arg
    130                 135                 140

Thr Glu Val Leu Ala Ala Leu Val Val Gly Gly Val Val Gly Ile Leu
145                 150                 155                 160

Phe Ala Val Phe Leu Val Leu Leu Leu Val Tyr Arg Met Lys Lys Lys
                165                 170                 175

Asp Glu Gly Ser Tyr Asp Leu Gly Lys Lys Pro Ile Tyr Lys Lys Ala
            180                 185                 190

Pro Thr Asn Glu Phe Tyr Ala
        195

<210>  7
<211>  198
<212>  PRT
<213>  Pan sp.

<400>  7

Met Ala Pro Ala Arg Leu Phe Ala Leu Leu Leu Leu Phe Val Gly Gly
1               5                   10                  15

Leu Ala Glu Ser Ile Arg Glu Thr Glu Val Ile Asp Pro Gln Asp Leu
            20                  25                  30

Leu Glu Gly Arg Tyr Phe Ser Gly Ala Leu Pro Asp Asp Glu Asp Val
        35                  40                  45

Val Gly Pro Gly Gln Glu Ser Asp Asp Phe Glu Leu Ser Gly Ser Gly
    50                  55                  60

Asp Leu Asp Asp Leu Glu Asp Ser Met Ile Gly Pro Glu Val Ile His
65                  70                  75                  80

Pro Leu Val Pro Leu Asp Asn His Ile Pro Glu Arg Ala Gly Ser Gly
                85                  90                  95

Ser Gln Val Pro Thr Glu Pro Lys Lys Leu Glu Glu Asn Glu Val Ile
            100                 105                 110

Pro Lys Arg Ile Ser Pro Ile Glu Glu Ser Glu Asp Val Ser Asn Lys
        115                 120                 125

Val Ser Met Ser Ser Thr Val Gln Gly Ser Asn Ile Phe Glu Arg Thr
    130                 135                 140

Glu Val Leu Ala Ala Leu Ile Val Gly Gly Ile Val Gly Ile Leu Phe
145                 150                 155                 160

Ala Val Phe Leu Ile Leu Leu Leu Met Tyr Arg Met Lys Lys Lys Asp
                165                 170                 175

Glu Gly Ser Tyr Asp Leu Gly Lys Lys Pro Ile Tyr Lys Lys Ala Pro
            180                 185                 190

Thr Asn Glu Phe Tyr Ala
        195

<210>  8
<211>  198
<212>  PRT
<213>  Papio sp.

<400>  8

Met Ala Pro Ala Arg Leu Phe Ala Leu Leu Leu Leu Phe Val Gly Gly
1               5                   10                  15

Val Ala Glu Ser Ile Arg Glu Thr Glu Val Ile Asp Pro Gln Asp Leu
            20                  25                  30

Leu Glu Gly Arg Tyr Phe Ser Gly Ala Leu Pro Asp Asp Glu Asp Val
        35                  40                  45

Val Gly Pro Gly Gln Glu Ser Asp Asp Phe Glu Leu Ser Gly Ser Gly
    50                  55                  60

Asp Leu Asp Asp Leu Glu Asp Ser Thr Ile Ser Pro Glu Val Ile His
65                  70                  75                  80

Pro Leu Val Pro Leu Asp Asn His Ile Pro Glu Lys Ala Gly Ser Gly
                85                  90                  95

Ser Gln Val Pro Thr Glu Ala Lys Lys Leu Glu Glu Asn Glu Val Ile
            100                 105                 110

Pro Lys Arg Ile Ser Pro Ile Glu Thr Ser Glu Asp Val Ser Asn Lys
        115                 120                 125

Val Ser Met Ser Ser Thr Met Gln Gly Ser Asn Ile Phe Glu Arg Thr
    130                 135                 140

Glu Val Leu Ala Ala Leu Ile Val Gly Gly Val Val Gly Ile Leu Phe
145                 150                 155                 160

Ala Val Phe Leu Ile Leu Leu Leu Met Tyr Arg Met Lys Lys Lys Asp
                165                 170                 175

Glu Gly Ser Tyr Asp Leu Gly Lys Lys Pro Ile Tyr Lys Lys Ala Pro
            180                 185                 190

Thr Asn Glu Phe Tyr Ala
        195

<210>  9
<211>  198
<212>  PRT
<213>  Pongo sp.

<400>  9

Met Ala Pro Ala Arg Leu Phe Ala Leu Leu Leu Leu Phe Val Gly Gly
1               5                   10                  15

Val Ala Glu Ser Ile Arg Glu Thr Glu Val Ile Asp Pro Gln Asp Leu
            20                  25                  30

Leu Glu Gly Arg Tyr Phe Ser Gly Ala Leu Pro Asp Asp Glu Asp Val
        35                  40                  45

Val Gly Pro Gly Gln Glu Ser Asp Asp Phe Glu Leu Ser Gly Ser Gly
    50                  55                  60

Asp Leu Asp Asp Leu Glu Asp Ser Ile Ile Gly Pro Glu Val Ile His
65                  70                  75                  80

Pro Leu Val Pro Leu Asp Asn His Ile Pro Glu Arg Ala Gly Ser Gly
                85                  90                  95

Ser Gln Val Pro Thr Glu Pro Lys Lys Leu Glu Glu Asn Glu Val Ile
            100                 105                 110

Pro Lys Arg Ile Ser Pro Ile Glu Glu Ser Glu Asp Val Ser Asn Lys
        115                 120                 125

Val Ser Met Ser Ser Thr Val Gln Gly Ser Asn Ile Phe Glu Arg Thr
    130                 135                 140

Glu Val Leu Ala Ala Leu Ile Val Gly Gly Ile Val Gly Ile Leu Phe
145                 150                 155                 160

Ala Val Phe Leu Ile Leu Leu Leu Met Tyr Arg Met Lys Lys Lys Asp
                165                 170                 175

Glu Gly Ser Tyr Asp Leu Gly Lys Lys Pro Ile Tyr Lys Lys Ala Pro
            180                 185                 190

Thr Asn Glu Phe Tyr Ala
        195

<210>  10
<211>  200
<212>  PRT
<213>  Danio rerio

<400>  10

Met Leu Val Leu Ser Ala Ala Ala Ala Val Phe Ala Glu Ser Val Arg
1               5                   10                  15

Glu Thr Glu Thr Trp Val Pro Leu Lys Asn Lys Ala Ala Ile Glu Asp
            20                  25                  30

Leu Glu Ser Ser Gly Asn Ser Gln Asn Glu Asp Phe Glu Phe Thr Asp
        35                  40                  45

Leu Asn Gln Thr Asp Asp Glu Asp Gly Phe Asp Glu Asp Asp Glu Asp
    50                  55                  60

Asp Tyr Asp Asp Met Ser Gly Ser Gly Phe Ala Asp Val Ile Asp Asp
65                  70                  75                  80

Ile Glu Asp Glu Phe Val Asn Asn Gln Ile Pro Asp Asp Lys Gly Pro
                85                  90                  95

Phe Ala Pro Arg Pro Thr Val Asp Glu Asn Ile Leu Val Gln Asn Asn
            100                 105                 110

Glu Val Phe Leu Arg Gly Ala Pro Asn Gln Asp Leu Asp Asn His Ser
        115                 120                 125

Asn Val Leu Met Thr His Ala Gly Asp Asp Ser Leu Phe Asn Asn Thr
    130                 135                 140

Glu Val Leu Ala Ala Val Ile Ala Gly Gly Ala Val Gly Leu Val Phe
145                 150                 155                 160

Ala Ile Leu Leu Ile Ile Leu Leu Val His Phe Val Leu Arg Ile Lys
                165                 170                 175

Lys Lys Asp Glu Gly Ser Tyr Asp Leu Gly Lys Thr Pro Ile Tyr Lys
            180                 185                 190

Lys Ala Pro Thr Thr Glu Ile Tyr
        195                 200

<210>  11
<211>  29
<212>  PRT
<213>  Mus musculus

<400>  11

Asn Ala Gln Pro Gly Ile Arg Val Pro Ser Glu Pro Lys Glu Leu Glu
1               5                   10                  15

Glu Asn Glu Val Ile Pro Lys Arg Ala Pro Ser Asp Val
            20                  25

<210>  12
<211>  24
<212>  DNA
<213>  artificial

<220>
<223>  primer binding to human Syndecan-4 sequence

<400>  12
cgggcaggaa tctgatgact ttga                                              24

<210>  13
<211>  24
<212>  DNA
<213>  artificial

<220>
<223>  primer binding to human Syndecan-4 sequence

<400>  13
gcttcacgcg tagaactcat tggt                                              24


<210>  14
<211>  20
<212>  DNA
<213>  artificial

<220>
<223>  primer binding to human GAPDH sequence

<400>  14
tcctgcacca ccaactgctt                                                   20


<210>  15
<211>  20
<212>  DNA
<213>  artificial

<220>
<223>  primer binding to human GAPDH sequence

<400>  15
tccaccaccc tgttgctgta                                                   20


<210>  16
<211>  24
<212>  DNA
<213>  artificial

<220>
<223>  primer binding to human HPRT sequence

<400>  16
ctttgctttc cttggtcagg cagt                                              24


<210>  17
<211>  24
<212>  DNA
<213>  artificial

<220>
<223>  primer binding to human HPRT sequence

<400> 17
aacaacaatc cgcccaaagg gaac 24